# EUROPEAN PATENT APPLICATION

(11) **EP 4 005 371 A1**
(43) Date of publication of application: **01.06.2022**
(21) Application number: 20846474.3
(22) Date of filing: 30.07.2020
(51) Int. Cl.: A01H 1/06, C07H 15/256, A23L 27/00, A01H 6/14

(54) **PLANT BODY CONTAINING NOVEL STEVIOL GLYCOSIDE**

(30) Priority: 31.07.2019 JP 2019141705
(71) Applicant: Suntory Holdings Limited, Osaka 530-8203 (JP)
(72) Inventor: URAI, Soichiro, Kawasaki-shi, Kanagawa 211-0067 (JP); IWAKI, Kazunari, Kawasaki-shi, Kanagawa 211-0067 (JP); MIYAGAWA, Katsuro, Soraku-gun, Kyoto 619-0284 (JP); HIRAI, Tadayoshi, Soraku-gun, Kyoto 619-0284 (JP); NAGAO, Koji, Kawasaki-shi, Kanagawa 211-0067 (JP); YOKOO, Yoshiaki, Kawasaki-shi, Kanagawa 211-0067 (JP); WATANABE, Takehiro, Soraku-gun, Kyoto 619-0284 (JP); FUJIKAWA, Kohki, Soraku-gun, Kyoto 619-0284 (JP)
(74) Representative: Mewburn Ellis LLP
(86) International application number: PCT/JP2020/029272
(87) International publication number: WO 2021/020517

(57) **Abstract**

The present invention provides a plant body containing a compound represented by Formula (1), or a salt or a hydrate thereof: 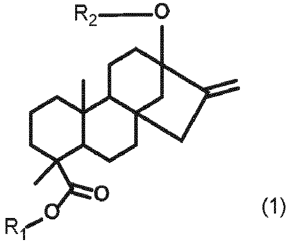 [in the formula: (i) R₁ represents Xyl(1-2)Glc1- and R₂ represents Glc(1-2)[Glc(1-3)]Glc1-or (ii) R₁ represents Glc(1-2)[Glc(1-3)]Glc1- and R₂ represents Xyl(1-2)[Glc(1-3)]Glc1-(where Glc stands for glucose and Xyl stands for xylose)].

## Description

### Technical Field

The present invention relates to a plant comprising a novel steviol glycoside, and an extract thereof, etc.

### Background Art

A leaf of *Stevia rebaudiana* contains a secondary metabolite called steviol which is one kind of diterpenoids, where the steviol glycosides provide sweetness that is nearly 300 times the sweetness of sugar and is therefore utilized as a calorieless sweetener in the food industry. The demand for calorieless sweeteners is growing day by day as obesity has become a serious social problem worldwide and also for the sake of health promotion and reduction in the medical expenditure. Currently, aspartame and acesulfame potassium, i.e., artificially synthesized amino acid derivatives, are utilized as artificial sweeteners, but natural calorieless sweeteners like the steviol glycosides are expected to be safer and more likely to gain public acceptance.

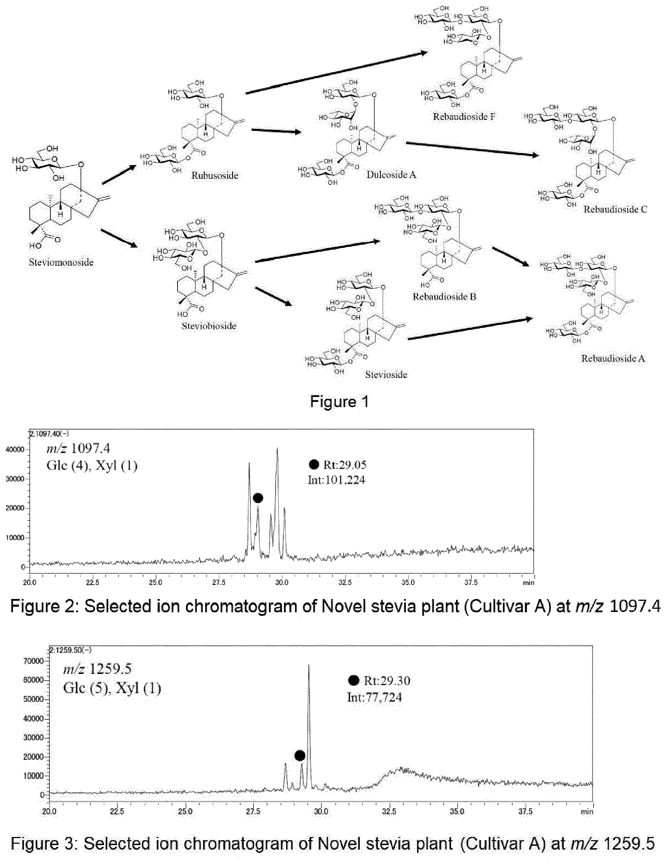

The major steviol glycosides from stevia are ultimately glycosylated to a glycoside called rebaudioside A (Reb.A) that has four sugar moieties (Figure 1). Stevioside, namely, a tri-glycosylated steviol glycoside which is a precursor of Reb.A, is the most abundant glycoside. These two glycosides are the main substances responsible for the sweetness of stevia. Stevioside accounts for the largest content in a stevia leaf and is known to provide sweetness that is about 250-300 times the sweetness of sugar. Reb.A is a tetra-glycosylated steviol glycoside that has strong sweetness (350-450 times sugar) with good taste quality. They have been drawing attention as calorieless sweeteners. Besides them, existence of glycosides that are considered to be reaction intermediates and analogs having different types of sugar moieties are known. For example, while all of the four glycoside sugar moieties of Reb.A are glucose, rebaudioside C (Reb.C) is known to have rhamnose instead of glucose attached to C-2 of glucose at C-13, and rebaudioside F (Reb.F) is known to have xylose attached at the same position.

To date, attempts have been made to obtain a stevia plant having a higher Reb.A content than wild-type stevia plants by variety improvement or the like since taste quality of Reb.A, in which all of the four glycoside sugar moieties are glucose, is good (for example, Patent literature 1). In addition, an attempt has also been made to obtain a novel steviol glycoside by decomposing a known steviol glycoside such as rebaudioside M (Reb.M (also referred to as Reb.X)) that has good taste quality with an acid (for example, Patent literature 2).

### Citation List

### Patent Literature

[Patent Literature 1] Japanese Patent No. 3436317
[Patent Literature 2] International Patent Application Publication WO2014/146135

### Summary of Invention

### Problem to be Solved by the Invention

Since the sweetness level and the taste quality of steviol glycosides may vary greatly depending on the difference in the number and the type of the sugar moiety that attaches to the diterpene structure serving as a backbone, there has been a need for a novel steviol glycoside.

### Means for Solving the Problems

The present invention provides a novel steviol glycoside comprising xylose, and a sweetener composition, a food or beverage and the like comprising the same as shown below.
[1] A stevia plant comprising a compound represented by Formula (1): 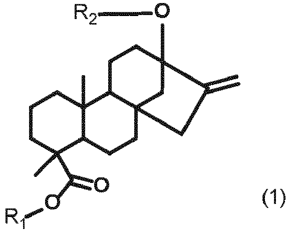 wherein, (i) R₁ represents Xyl(1-2)Glc1- while R₂ represents Glc(1-2)[Glc(1-3)]Glc1-; or (ii) R₁ represents Glc(1-2)[Glc(1-3)]Glc1- while R₂ represents Xyl(1-2)[Glc(1-3)]Glc1-, where Glc represents glucose and Xyl represents xylose).
[2] The plant according to [1], wherein the compound is represented by Formula (2) or (3) below: 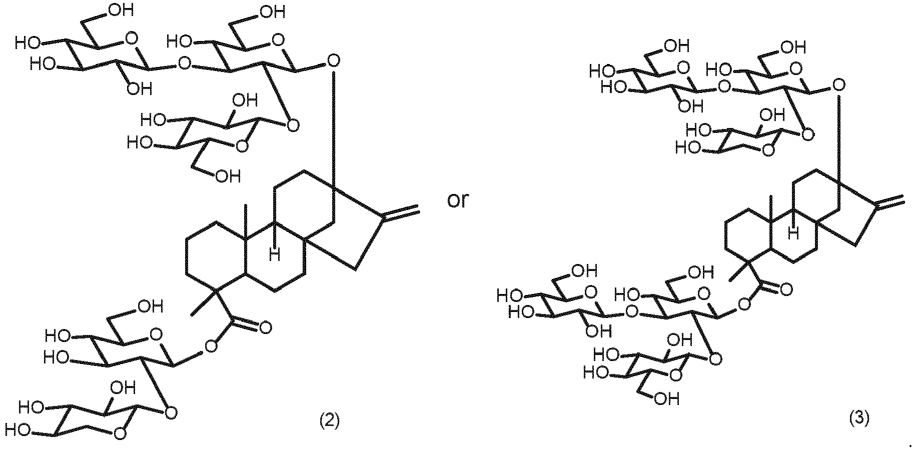
[3] The plant according to [1] or [2], wherein the compound is represented by Formula (4) or (5) below: 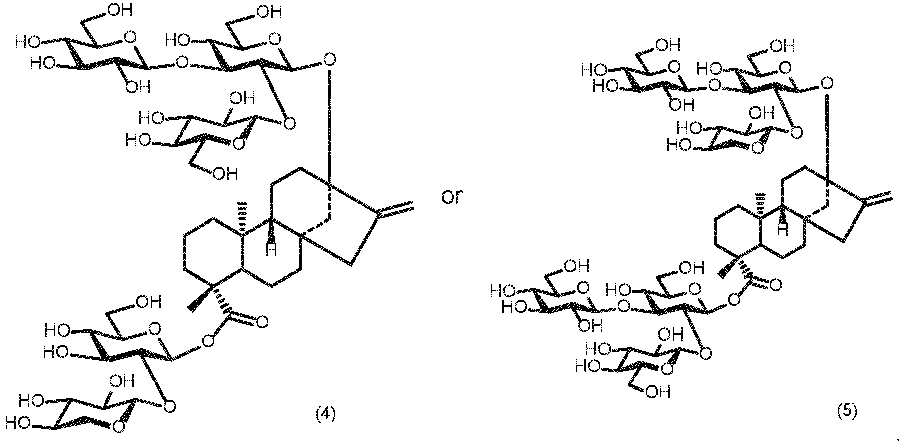
[4] The plant according to any one of [1] to [3], wherein the content of the compound comprised in a leaf is 0.050 wt% or more relative to the amount of total steviol glycosides comprised in the leaf.
[5] The plant according to any one of [1] to [4], having at least one genetic feature selected from the following (1) to (8):
   (1) being homozygous or heterozygous for the allele wherein the base at the position corresponding to position 298 of SEQ ID NO: 1 is T;
   (2) being homozygous or heterozygous for the allele wherein the base at the position corresponding to position 328 of SEQ ID NO: 1 is C;
   (3) being homozygous or heterozygous for the allele wherein the base at the position corresponding to position 360 of SEQ ID NO: 1 is T;
   (4) being homozygous or heterozygous for the allele wherein the base at the position corresponding to position 386 of SEQ ID NO: 1 is T;
   (5) being homozygous or heterozygous for the allele wherein the base at the position corresponding to position 393 of SEQ ID NO: 1 is T;
   (6) being homozygous or heterozygous for the allele wherein the base at the position corresponding to position 411 of SEQ ID NO: 1 is T;
   (7) being homozygous or heterozygous for the allele wherein the base at the position corresponding to position 427 of SEQ ID NO: 1 is C; and
   (8) being homozygous or heterozygous for the allele wherein the base at the position corresponding to position 453 of SEQ ID NO: 1 is T.
[6] The plant according to any one of [1] to [5], wherein a gene encoding a protein having the amino acid sequence of SEQ ID NO: 97 is expressed at a higher level than a wild type line.
[7] The plant according to any one of [1] to [6], wherein the plant is a non-genetically modified plant.
[8] The plant according to any one of [1] to [7], wherein the plant comprises a stevia plant subjected to mutagenesis treatment and a progeny plant thereof.
[9] A method of producing a stevia plant comprising a compound as defined in any one of [1] to [3], comprising a step of crossing the plant according to any one of [1] to [8] with a second stevia plant.
[10] An extract of the stevia plant according to any one of [1] to [8], comprising the compound as defined in any one of [1] to [3].
[11] The extract according to [10], wherein the content of the compound as defined in any one of [1] to [3] is 0.050 wt% or more relative to a content of total steviol glycoside.
[12] A method of producing the extract according to [10] or [11], comprising a step of obtaining the extract from the plant according to any one of [1] to [8].
[13] A method of producing a purified product of the compound as defined in any one of [1] to [3], comprising: a step of obtaining an extract from the plant according to any one of [1] to [8]; and a step of purifying the compound from the obtained extract.
[14] A food or beverage, a sweetener composition, a flavoring agent or a pharmaceutical product comprising the plant according to any one of [1] to [8] or the extract according to [10] or [11].
[15] The food or beverage, sweetener composition, flavoring agent or pharmaceutical product according to [14], wherein the content of the compound as defined in any one of [1] to [3] is 1 mass ppm to 600 mass ppm.
[16] The food or beverage according to [14] or [15], wherein the food or beverage is a beverage.
[17] A method of producing a food or beverage, a sweetener composition, a flavoring agent or a pharmaceutical product, comprising:
   a step of providing the extract according to [10] or [11] or a purified product thereof; and
   a step of adding the extract or the purified product to a raw material for the food or beverage, sweetener composition, flavoring agent or pharmaceutical product.
[18] A method of screening for a plant comprising the compound as defined in any one of [1] to [3], comprising:
   (i) a step of detecting from the genome of a test stevia plant the presence and/or the absence of at least one of genetic features (1) to (8) as defined in claim 5; and/or
   (ii) a step of detecting expression of a gene encoding a protein having the amino acid sequence of SEQ ID NO: 97 in the test stevia plant.
[19] The method according to [18], further comprising a step of evaluating the content of the compound as defined in any one of [1] to [3] in the test stevia plant.
[20] A screening kit for the stevia plant comprising the compound as defined in any one of [1] to [3], comprising a reagent for detecting the presence and/or absence of at least one of genetic features (1) to (8) as defined in [5] and/or a reagent for detecting expression of a gene encoding a protein having the amino acid sequence of SEQ ID NO: 97.
[21] A method of producing a stevia plant comprising the compound as defined in any one of [1] to [3], comprising:
   (1) a step of introducing a variation from C to T to a position corresponding to position 298 of SEQ ID NO: 1;
   (2) a step of introducing a variation from A to C to a position corresponding to position 328 of SEQ ID NO: 1;
   (3) a step of introducing a variation from C to T to a position corresponding to position 360 of SEQ ID NO: 1;
   (4) a step of introducing a variation from C to T to a position corresponding to position 386 of SEQ ID NO: 1;
   (5) a step of introducing a variation from C to T to a position corresponding to position 393 of SEQ ID NO: 1;
   (6) a step of introducing a variation from C to T to a position corresponding to position 411 of SEQ ID NO: 1
   (7) a step of introducing a variation from A to C to a position corresponding to position 427 of SEQ ID NO: 1; and/or
   (8) a step of introducing a variation from C to T to a position corresponding to position 453 of SEQ ID NO: 1.

### Advantageous Effects of Invention

The present invention can provide a novel steviol glycoside comprising xylose, a method for producing the same, and a sweetener composition, a food or beverage, a plant, an extract thereof and a flavor controlling agent comprising the novel steviol glycoside. A steviol glycoside in a preferable aspect of the present invention has excellent taste quality and a high sweetness level. A sweetener composition in another aspect of the present invention has excellent taste quality.

### Brief Description of Drawings

Figure 1 is a diagram showing structures and names of steviol glycosides.
Figure 2 is a diagram showing a selected ion chromatogram of Cultivar A at m/z of 1097.4.
Figure 3 is a diagram showing a selected ion chromatogram of Cultivar A at m/z of 1259.5.
Figure 4 is a diagram showing MS/MS and MS³-fragmented mass spectra of Novel steviol glycoside IE.
Figure 5 is a diagram showing MS/MS and MS³-fragmented mass spectra of Novel steviol glycoside 2E.

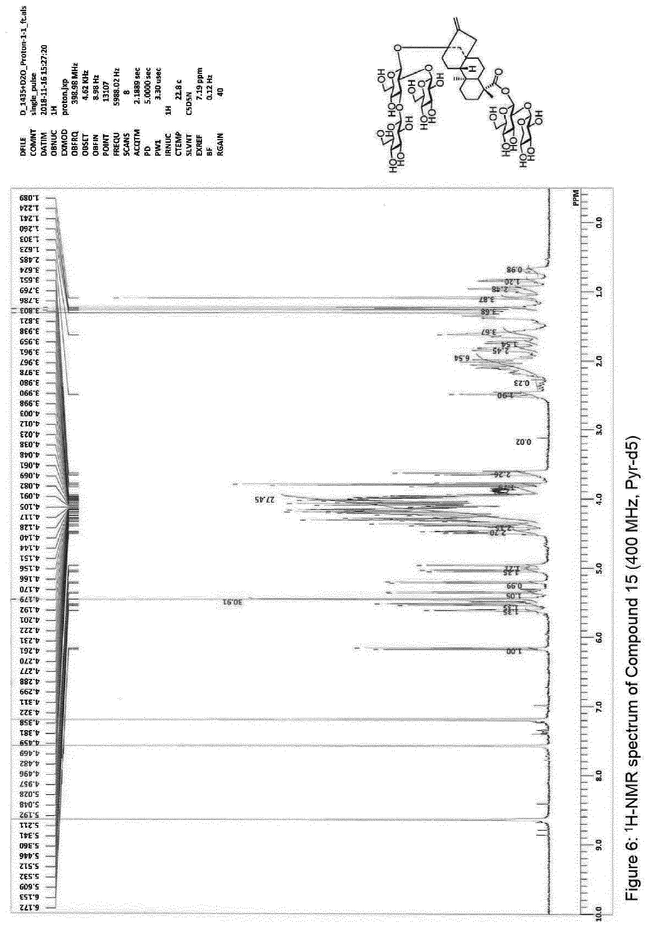

Figure 6 is a diagram showing a ¹H-NMR spectrum of Compound 15 (400 MHz, Pyr-d5).

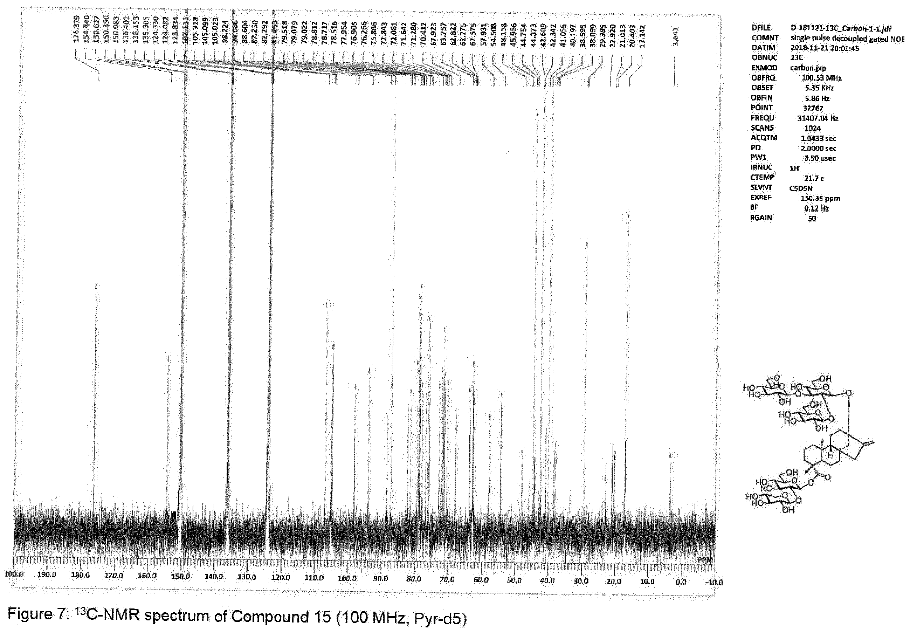

Figure 7 is a diagram showing a ¹³C-NMR spectrum of Compound 15 (100 MHz, Pyr-d5).

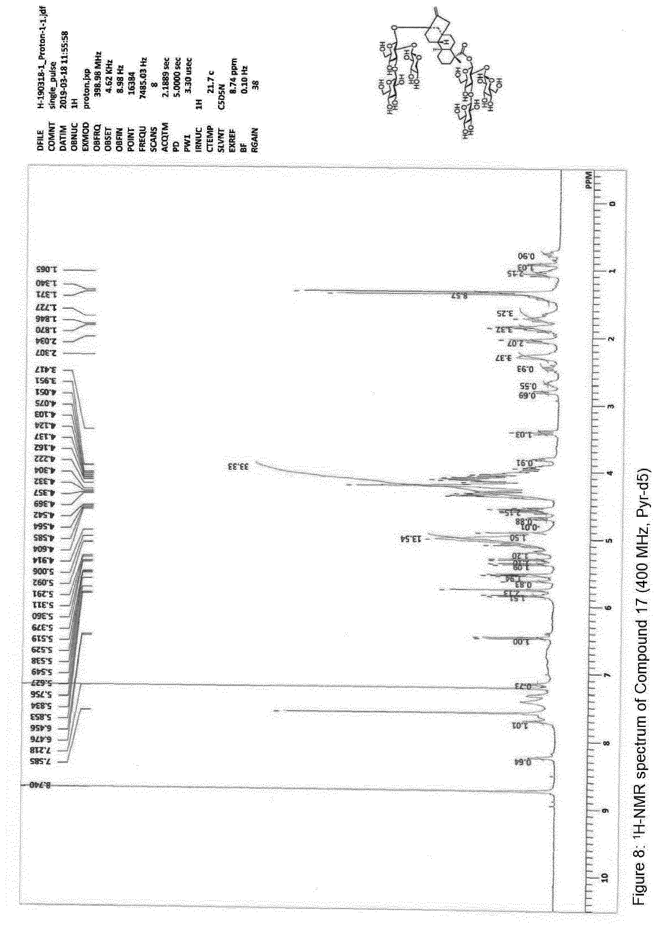

Figure 8 is a diagram showing a ¹H-NMR spectrum of Compound 17 (400 MHz, Pyr-d5).

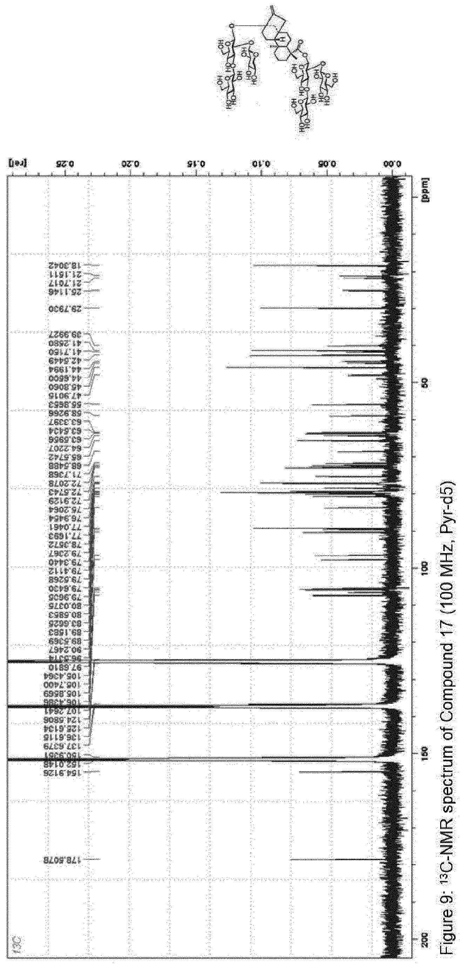

Figure 9 is a diagram showing a ¹³C-NMR spectrum of Compound 17 (100 MHz, Pyr-d5).
Figure 10 is a diagram showing extracted ion chromatograms of Novel steviol glycoside IE (stevia leaf extract) and a chemically synthesized product (β-form of Compound 15).
Figure 11 is a diagram showing MS/MS and MS³-fragmented mass spectra of Novel steviol glycoside IE (stevia leaf extract) and the chemically synthesized product (β-form of Compound 15).
Figure 12 is a diagram showing extracted ion chromatograms of Novel steviol glycoside 2E (stevia leaf extract) and a chemically synthesized product (β-form of Compound 17).
Figure 13 is a diagram showing MS/MS and MS³-fragmented mass spectra of Novel steviol glycoside 2E (stevia leaf extract) and the chemically synthesized product (β-form of Compound 17).
Figure 14 is a diagram showing results of sensory evaluations for comparison of Novel steviol glycoside A with sugar, rebaudioside A, rebaudioside D and rebaudioside M.
Figure 15 is a diagram showing results of sensory evaluations for comparison of Novel steviol glycoside B with sugar, rebaudioside A, rebaudioside D and rebaudioside M.
Figure 16 is a diagram showing results of an expression analysis in relation to identification of the genetic features in the example.

### Description of Embodiments

Hereinafter, the present invention will be described in detail. The following embodiment is provided for illustrating the present invention with no intention of limiting the present invention solely to this embodiment. The present invention may be carried out in various modes without departing from the scope thereof. All of the documents, publications, patent publications and other patent documents cited herein are incorporated herein by reference. The present specification incorporates the contents of the specification and the drawings of Japanese Patent Application No. 2019-141705, filed on July 31, 2019, from which the present application claims priority.

The terms "rebaudioside", "Reb" and "Reb." as used herein have the same meaning and all of them refer to "rebaudioside". Similarly, the term "dulcoside" as used herein refers to "dulcoside".

### 1. Novel steviol glycoside

For the first time, the present inventors identified the structure of a novel steviol glycoside that contains xylose. The novel steviol glycoside of the present invention (herein, also referred to as the "glycoside of the present invention") is a compound represented by Formula (1), or a salt or a hydrate thereof: 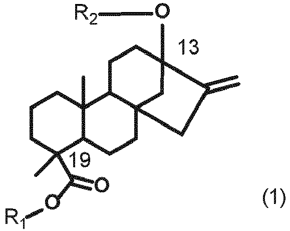 wherein, (i) R₁ represents Xyl(1-2)Glc1- while R₂ represents Glc(1-2)[Glc(1-3)]Glc1-; or (ii) R₁ represents Glc(1-2)[Glc(1-3)]Glc1- while R₂ represents Xyl(1-2)[Glc(1-3)]Glc1-, where Glc represents glucose and Xyl represents xylose. Herein, a glucose moiety and a xylose moiety in a sugar chain may also be referred to as glucopyranosyl and xylopyranosyl, respectively.

As represented above, the glycoside of the present invention comprises a steviol glycoside having a sugar chain containing three glucose moieties at C-13 of steviol and one glucose moiety and one xylose moiety at C-19 of steviol (herein, also referred to as "Glycoside A of the present invention"), and a steviol glycoside having a sugar chain containing two glucose moieties and one xylose moiety at C-13 of steviol and a sugar chain containing three glucose moieties at C-19 of steviol (herein, also referred to as "Glycoside B of the present invention").

Furthermore, as described above, Glc represents glucose and Xyl represents xylose. As used herein, "Glc" may be α- or β-glucose while Xyl may be α- or β-xylose. Alternatively, as used herein, Glc may be α- and β-glucose while Xyl may be α- and β-xylose. Moreover, "Glcl-" indicates that the carbon atom at C-1 of glucose is attached to steviol via a glycosidic bond, and "Glc(1-3)-Glc1-" indicates that the carbon atom at C-3 of glucose represented by "Glcl-" is attached to a carbon atom at C-1 of another glucose via a glycosidic bond. Furthermore, "Xyl(1-2)-Glc1-" indicates that the carbon atom at C-2 of glucose represented by "Glcl-" is attached to the carbon atom at C-1 of xylose via a glycosidic bond. Furthermore, "Xyl(1-2)[Glc(1-3)]Glc1-" indicates that the carbon atom at C-2 of glucose represented by "Glcl-" is attached to the carbon atom at C-1 of xylose via a glycosidic bond, and the carbon atom at C-3 of glucose represented by "Glcl-" is attached to the carbon atom at C-1 of glucose via a glycosidic bond.

Examples of Glycoside A include glycosides having the structures represented by Formulae (2), (2)', (4) and (4)'.

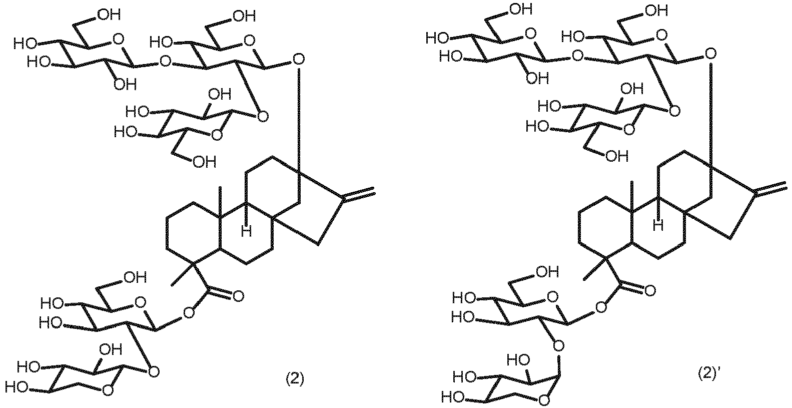

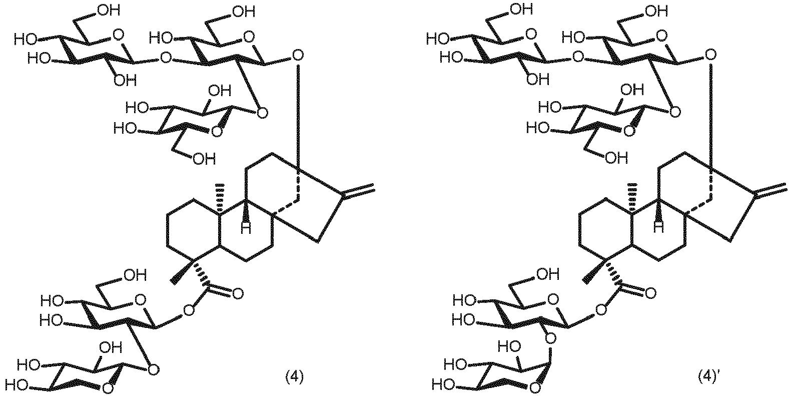

In Glycoside A represented by Formula (2), glucose is attached to the carboxylic group at C-19 of steviol via a β-glycosidic bond and xylose is attached to said glucose via a β1-2 bond, whereas in Glycoside A represented by Formula (2)', glucose is attached to the carboxylic group at C-19 of steviol via a β-glycosidic bond, and xylose is attached to said glucose via an α1-2 bond. Formulae (4) and (4)' represent structures having further specified conformations of Glycosides A represented by Formulae (2) and (2)', respectively.

Examples of Glycoside B include glycosides having the structures represented by Formulae (3), (3)', (5) and (5)'.

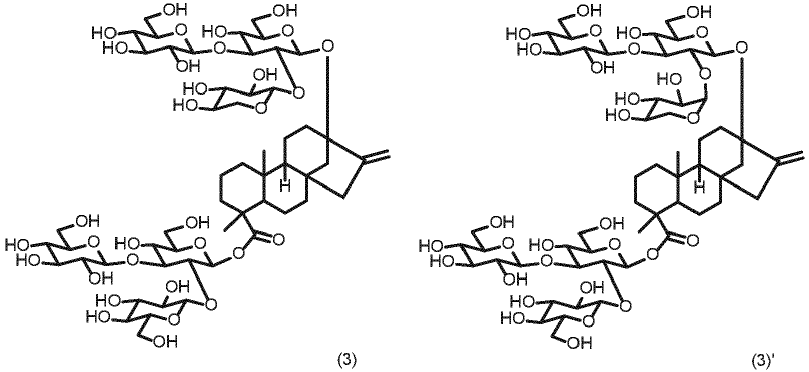

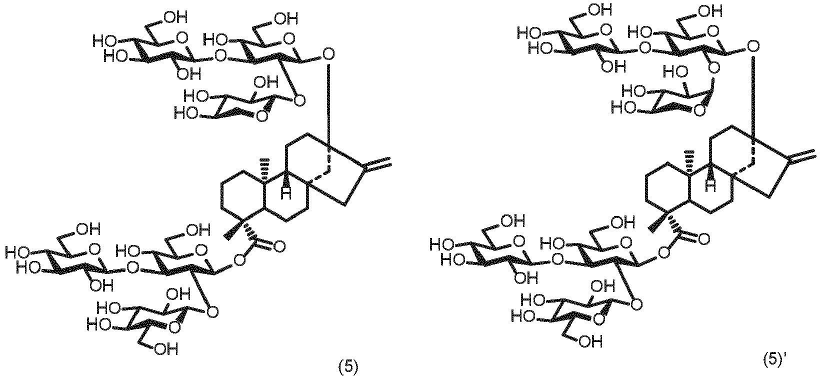

In Glycoside B represented by Formula (3), glucose is attached to the hydroxy group at C-13 of steviol via a β-glycosidic bond, another glucose is attached to said glucose via a β1-3 bond and xylose is attached to said glucose via a β1-2 bond. In Glycoside B represented by Formula (3)', glucose is attached to the hydroxy group at C-13 of steviol via a β-glycosidic bond, another glucose is attached to said glucose via a β1-3 bond and xylose is attached to said glucose via an α1-2 bond. Formulae (5) and (5)' represent structures having further specified conformations of Glycosides B represented by Formulae (3) and (3)', respectively.

The glycoside of the present invention also comprises isomers such as the α- and β-forms as described above. Therefore, the glycoside of the present invention may comprise only the α-form, only the β-form or a mixture of the α- and β-forms. The proportion of the β-form in the glycoside of the present invention is preferably 80% or more, more preferably 90% or more, still more preferably 95% or more and particularly preferably 99% or more. The α- and β-forms can be isolated/purified by a known method such as high-performance liquid chromatography (HPLC), ultra (high) performance liquid chromatography (UPLC), or the like.

The glycoside of the present invention may not only be the compound represented by Formula (1) but may also be a derivative, a salt or a hydrate thereof. The term "derivative" as used herein refers to a compound resulting from a structural change of a minor moiety of the compound, for example, a compound in which some of the hydroxy groups are substituted with other substituents. Therefore, derivatives of the compound represented by Formula (1) include compounds in which some of the hydroxy groups contained in the compound have been substituted with a substituent selected from hydrogen, a halogen, an alkyl group, an alkenyl group, an alkynyl group, an aryl group, an amino group, a cyano group or the like. As used herein, a "salt of the compound represented by Formula (1)" refers to a physiologically acceptable salt, for example, a sodium salt, of the compound represented by Formula (1). Furthermore, a "hydrate of the compound represented by Formula (1)" as used herein refers to a compound resulting from attachment of a water molecule to the compound represented by Formula (1).

The glycoside of the present invention is sweeter than sugar (sucrose), and can affect the sweetness of a food or beverage in a small amount. Thus, the glycoside of the present invention can be used as a novel sweetener.

A glycoside in a preferable aspect of the present invention is selected from Glycoside A or Glycoside B. Glycoside A is sweeter than sugar, has less lingering sweet and bitter aftertastes, and has weaker bitterness than other components including sugar. Glycoside B is also sweeter than sugar, and has weaker bitterness than other steviol glycosides. Accordingly, the steviol glycoside of the present invention can favorably be used as a sweetener in various applications as will be described later.

### 2. Sweetener composition comprising novel steviol glycoside

In one aspect of the present invention, a sweetener composition comprising the compound represented by Formula (1), or a derivative, a salt or a hydrate thereof (hereinafter, also referred to as the "sweetener composition of the present invention") is provided. The sweetener composition of the present invention is not particularly limited as long as it contains the compound represented by Formula (1), or a derivative, a salt or a hydrate thereof, and it may be a composition comprising an extract comprising the compound represented by Formula (1), or a derivative, a salt or a hydrate thereof.

The amount of the glycoside of the present invention contained in the sweetener composition of the present invention is not particularly limited, and may be, for example, 1-99 wt%, 5-95 wt%, 10-90 wt%, 15-85 wt%, 20-80 wt%, 25-75 wt%, 30-70 wt%, 35-65 wt%, 40-60 wt%, 45-55 wt%, 1-5 wt%, 1-10 wt%, 1-15 wt%, 1-20 wt%, 1-25 wt%, 1-30 wt%, 1-35 wt%, 1-40 wt%, 1-45 wt% or 1-50 wt% relative to the total amount of the sweetener composition.

The sweetener composition of the present invention may further contain other steviol glycosides. For example, the sweetener composition of the present invention may contain, in addition to the glycoside of the present invention, one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside.

In a case where other steviol glycoside is contained, the composition ratio of the glycoside of the present invention and other steviol glycoside may be 1:99 to 99:1, 5:95 to 95:5, 10:90 to 90:10, 15:85 to 85:15, 20:80 to 80:20, 25:75 to 75:25, 30:70 to 70:30, 35:65 to 65:35, 40:60 to 60:40, 45:65 to 65:45 or 50:50 in a mass ratio. Moreover, either a single or multiple types of glycosides of the present invention may be used.

The sweetener composition of the present invention may further contain a sweetener other than the steviol glycosides. Examples of such a sweetener include natural sweeteners such as fructose, sugar, fructose-glucose syrup, glucose, maltose, high-fructose syrup, sugar alcohol, oligosaccharide, honey, pressed sugarcane juice (brown sugar syrup), starch syrup, Lo Han Kuo (*Siraitia grosvenorii)* powder, a Lo Han Kuo (*Siraitia grosvenorii)* extract, licorice powder, a licorice extract, *Thaumatococcus daniellii* seed powder, a *Thaumatococcus daniellii* seed extract, and artificial sweeteners such as acesulfame potassium, sucralose, neotame, aspartame and saccharin. Among them, a natural sweetener is preferably used from the aspect of imparting clean taste, easy drinkability, natural flavor and moderately rich taste, where fructose, glucose, maltose, sucrose and sugar are particularly preferably used. Either a single or multiple types of these sweetness components may be used.

A method for producing the sweetener composition of the present invention is not particularly limited as long as a sweetener composition having the above-described composition can be obtained. In one aspect of the present invention, a method for producing a sweetener composition of the present invention, the method comprising the steps of: obtaining a glycoside of the present invention; and mixing the glycoside with other steviol glycoside or a sweetener other than a steviol glycoside, is provided. The step of obtaining a glycoside of the present invention may be carried out by isolation/purification from a plant, a chemical synthesis or a biosynthesis, where the glycoside of the present invention resulting from this step may be obtained as a mixture with other steviol glycoside (for example, Reb.A or Reb.D).

### 3. Food or beverage, flavoring agent and pharmaceutical product comprising novel steviol glycoside

In one aspect of the present invention, a food or beverage, a flavoring agent and a pharmaceutical product comprising the compound represented by Formula (1) or a derivative, a salt or a hydrate thereof or the sweetener composition of the present invention (herein, also referred to as a "food or beverage of the present invention", a "flavoring agent of the present invention" and a "pharmaceutical product of the present invention", respectively) are provided. The food or beverage, the flavoring agent and the pharmaceutical product of the present invention are not particularly limited as long as they contain the compound represented by Formula (1), or a derivative, a salt or a hydrate thereof, and they may be a food or beverage, a flavoring agent and a pharmaceutical product comprising an extract or a sweetener composition comprising the compound represented by Formula (1), or a derivative, a salt or a hydrate thereof. Herein, a food or beverage refers to beverages and foods. Therefore, in some embodiments, the present invention provides a novel beverage or food, and a method for producing said beverage or food.

While the amount of the glycoside of the present invention contained in the food or beverage, the flavoring agent and the pharmaceutical product of the present invention differs depending on the specific food or beverage, it is preferably around 1-600 mass ppm, for example, 20-550 mass ppm, 25-550 mass ppm, 30-550 mass ppm, 35-550 mass ppm, 40-550 mass ppm, 45-550 mass ppm, 50-550 mass ppm, 55-550 mass ppm, 20-540 mass ppm, 25-540 mass ppm, 30-540 mass ppm, 35-540 mass ppm, 40-540 mass ppm, 45-540 mass ppm, 50-540 mass ppm, 55-540 mass ppm, 20-530 mass ppm, 25-530 mass ppm, 30-530 mass ppm, 35-530 mass ppm, 40-530 mass ppm, 45-530 mass ppm, 50-530 mass ppm, 55-530 mass ppm, 20-520 mass ppm, 25-520 mass ppm, 30-520 mass ppm, 35-520 mass ppm, 40-520 mass ppm, 45-520 mass ppm, 50-520 mass ppm, 55-520 mass ppm, 20-510 mass ppm, 25-510 mass ppm, 30-510 mass ppm, 35-510 mass ppm, 40-510 mass ppm, 45-510 mass ppm, 50-510 mass ppm, 55-510 mass ppm, 20-505 mass ppm, 25-505 mass ppm, 30-505 mass ppm, 35-505 mass ppm, 40-505 mass ppm, 45-505 mass ppm, 50-505 mass ppm, 55-505 mass ppm, 20-500 mass ppm, 25-500 mass ppm, 30-500 mass ppm, 35-500 mass ppm, 40-500 mass ppm, 45-500 mass ppm, 50-500 mass ppm, 55-500 mass ppm, 20-495 mass ppm, 25-495 mass ppm, 30-495 mass ppm, 35-495 mass ppm, 40-495 mass ppm, 45-495 mass ppm, 50-495 mass ppm, 55-495 mass ppm, 20-490 mass ppm, 25-490 mass ppm, 30-490 mass ppm, 35-490 mass ppm, 40-490 mass ppm, 45-490 mass ppm, 50-490 mass ppm, 55-490 mass ppm, 100-400 mass ppm, 150-400 mass ppm, 200-400 mass ppm, 250-400 mass ppm, 300-400 mass ppm, 100-150 mass ppm, 100-200 mass ppm, 100-250 mass ppm or 100-300 mass ppm, in a case of a beverage. The content within this range is advantageous for imparting moderate sweetness. The content within this range is advantageous for suppressing the lingering aftertaste. Unless otherwise specified, "ppm" as used herein refers to "mass ppm".

The food or beverage, the flavoring agent and the pharmaceutical product of the present invention may further contain other steviol glycosides. For example, the sweetener composition of the present invention may contain, in addition to the glycoside of the present invention, one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside.

In a case where other steviol glycoside is contained, the composition ratio of the glycoside of the present invention and other steviol glycoside may be 1:99 to 99:1, 5:99 to 95:5, 10:90 to 90:10, 15:85 to 85:15, 20:80 to 80:20, 25:75 to 75:25, 30:70 to 70:30, 35:65 to 65:35, 40:60 to 60:40, 45:65 to 65:45 or 50:50 in a mass ratio.

The food or beverage, the flavoring agent and the pharmaceutical product of the present invention may further contain a sweetener other than a steviol glycoside. Examples of such a sweetener include natural sweeteners such as fructose, sugar, fructose-glucose syrup, glucose, maltose, sucrose, high-fructose syrup, sugar alcohol, oligosaccharide, honey, pressed sugarcane juice (brown sugar syrup), starch syrup, Lo Han Kuo *(Siraitia grosvenorii)* powder, a Lo Han Kuo (*Siraitia grosvenorii)* extract, licorice powder, a licorice extract, *Thaumatococcus daniellii* seed powder and a *Thaumatococcus daniellii* seed extract, and artificial sweeteners such as acesulfame potassium, sucralose, neotame, aspartame and saccharin. Among them, a natural sweetener is preferably used from the aspect of imparting clean taste, easy drinkability, natural flavor and moderately rich taste, where fructose, glucose, maltose, sucrose and sugar are particularly preferably used. Either a single or multiple types of these sweetness components may be used.

Examples of the food of the present invention include, but not particularly limited to, a confection, a bread, cereal flour, noodles, rice, a processed agricultural/forestry food, a processed livestock product, a processed fishery product, a milk/dairy product, an oil-and-fat/processed oil-and-fat product, seasoning or other food materials.

Examples of the beverage of the present invention include, but not particularly limited to, a carbonated beverage, a non-carbonated beverage, an alcoholic beverage, a non-alcoholic beverage, a beer-taste beverage such as beer or non-alcohol beer, a coffee beverage, a tea beverage, a cocoa beverage, a nutritious beverage and a functional beverage.

The beverage of the present invention may be heat sterilized and packaged to be prepared as a packaged beverage. Examples of such a package include, but not particularly limited to, a PET bottle, an aluminum can, a steel can, a paper package, a chilled cup and a bottle. If heat sterilization is to be performed, the type of heat sterilization is not particularly limited, and heat sterilization may be performed, for example, by employing a common technique such as UHT sterilization, retort sterilization or the like. While the temperature during the heat sterilization process is not particularly limited, it is, for example, 65-130°C, preferably 85-120°C for 10-40 minutes. Sterilization, however, can be carried out at an appropriate temperature for a several seconds, for example, 5-30 seconds, without any problem as long as a sterilizing value comparative to that under the above-described conditions can be earned.

The method for producing the food or beverage, the flavoring agent and the pharmaceutical product of the present invention is not particularly limited as long as a food or beverage, a flavoring agent and a pharmaceutical product having the above-described components can be obtained. In one aspect of the present invention, a method for producing a food or beverage, a flavoring agent and a pharmaceutical product of the present invention, the method comprising the steps of: obtaining the extract, the glycoside or the sweetener composition of the present invention; and adding the extract, the glycoside or the sweetener composition to a food or beverage, a flavoring agent, a pharmaceutical product or a raw material thereof, is provided. The step of obtaining the glycoside or the sweetener composition of the present invention and the step of obtaining the extract of the present invention are described in "2. Sweetener composition comprising novel steviol glycoside" and "4. Stevia plant comprising novel steviol glycoside and extract thereof", respectively. The step of adding the extract, the glycoside or the sweetener composition of the present invention to a food or beverage, a flavoring agent, a pharmaceutical product or a raw material thereof can be carried out during any step of producing the food or beverage, the flavoring agent and the pharmaceutical product. For example, it may be carried out upon mixing the raw materials of the food or beverage, the flavoring agent or the pharmaceutical product, or upon the final adjustments of the taste quality of the food or beverage, the flavoring agent or the pharmaceutical product.

### 4. Stevia plant comprising novel steviol glycoside and extract thereof

In one aspect of the present invention, a stevia plant comprising the compound represented by Formula (1), or a derivative, a salt or a hydrate thereof and an extract thereof (herein, also referred to as "the plant of the present invention and the extract thereof") are provided. Furthermore, in another aspect of the present invention, a food or beverage, a flavoring agent and a pharmaceutical product, preferably a beverage, comprising the plant of the present invention or the extract thereof, are provided. While the amount of the glycoside of the present invention contained in the plant of the present invention is not particularly limited, it may be 0.001-1.000 wt%, 0.005-0.950 wt%, 0.008-0.900 wt%, 0.010-0.850 wt% or 0.015-0.800 wt% in a dried leaf.

In one aspect of the present invention, the plant of the present invention has at least one of the genetic features (1) to (8) below (hereinafter, may be collectively referred to as the "genetic feature of the present invention"):
(1) being homozygous or heterozygous for the allele wherein the base at the position corresponding to position 298 of SEQ ID NO: 1, position 11 of SEQ ID NO:3, position 21of SEQ ID NO:5 or position 26 of SEQ ID NO:7 is T;
(2) being homozygous or heterozygous for the allele wherein the base at the position corresponding to position 328 of SEQ ID NO: 1, position 11 of SEQ ID NO:9, position 21 of SEQ ID NO:11 or position 26 of SEQ ID NO:13 is C;
(3) being homozygous or heterozygous for the allele wherein the base at the position corresponding to position 360 of SEQ ID NO: 1, position 11 of SEQ ID NO:15, position 21 of SEQ ID NO:17 or position 26 of SEQ ID NO:19 is T;
(4) being homozygous or heterozygous for the allele wherein the base at the position corresponding to position 386 of SEQ ID NO: 1, position 11 of SEQ ID NO:21, position 21 of SEQ ID NO:23 or position 26 of SEQ ID NO:25 is T;
(5) being homozygous or heterozygous for the allele wherein the base at the position corresponding to position 393 of SEQ ID NO: 1, position 11 of SEQ ID NO:27, position 18 of SEQ ID NO:29 or position 23 of SEQ ID NO:31 is T;
(6) being homozygous or heterozygous for the allele wherein the base at the position corresponding to position 411 of SEQ ID NO: 1, position 11 of SEQ ID NO:33, position 18 of SEQ ID NO:35 or position 23 of SEQ ID NO:37 is T;
(7) being homozygous or heterozygous for the allele wherein the base at the position corresponding to position 427 of SEQ ID NO:1, position 11 of SEQ ID NO:39, position 18 of SEQ ID NO:41 or position 23 of SEQ ID NO:43 is C; and
(8) being homozygous or heterozygous for the allele wherein the base at the position corresponding to position 453 of SEQ ID NO: 1, position 11 of SEQ ID NO:45, position 18 of SEQ ID NO:47 or position 23 of SEQ ID NO:49 is T.

In other aspect of the present invention, the plant of the present invention has at least two, at least three, at least four, at least five, at least six, at least seven or all of the eight genetic features (1) to (8).

The phrase "position (or a part) corresponding to" refers to the position or the part (for example, position 298, position 328, position 360, position 386, position 393, position 411, position 427 and position 453, etc.) of the sequence existing in the genome if said sequence existing in the genome is identical to the reference sequence (for example, SEQ ID NO: 1, etc.). If none of the sequences existing in the genome is identical to the reference sequence, the phrase refers to a position or a part of a sequence existing in the genome which correlates with the position or the part of the reference sequence. Whether or not a sequence identical to or correlating with the reference sequence exists in the genome can be determined, for example, as follows: the genomic DNA of the intended stevia plant is amplified with primers that can amplify the reference sequence through PCR; the amplified product is sequenced; and an alignment analysis is performed between the resulting sequence and the reference sequence. Examples of sequences corresponding to the reference sequence include, but not limited to, nucleotide sequences having sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more or 99.8% or more to the reference sequence. A position or a part of a sequence existing in the genome which correlates with the position or the part of the reference sequence can be determined by considering the nucleotide sequences preceding and following the position or the part of the reference sequence and the like. For example, the reference sequence can be aligned with a sequence in the genome corresponding to the reference sequence so as to determine the position or the part of the sequence existing in the genome which correlates with the position or the part of the reference sequence.

For example, in the case of "the position corresponding to position 298 of SEQ ID NO:1" as in the genetic feature (1) of the present invention, if the genome of the stevia plant has a part consisting of a nucleotide sequence identical to SEQ ID NO: 1, "the position corresponding to position 298 of SEQ ID NO: 1" refers to position 298 from the 5' end of said part of the genome, which consists of a nucleotide sequence identical to SEQ ID NO:1. Meanwhile, if the genome of the stevia plant has a part consisting of a nucleotide sequence that is not identical but that corresponds to SEQ ID NO: 1, "the position corresponding to position 298 of SEQ ID NO:1" does not necessarily refers to the 298th position from the 5' end of the part corresponding to SEQ ID NO: 1 since the genome does not have a part consisting of a nucleotide sequence that is identical to SEQ ID NO: 1, but "the position corresponding to position 298 of SEQ ID NO:1" in said genome of the stevia plant can be specified by considering the nucleotide sequences preceding or following position 298 of SEQ ID NO: 1. For example, "the position corresponding to position 298 of SEQ ID NO: 1" in the genome of the stevia plant can be specified by an alignment analysis between a nucleotide sequence in the genome of the stevia plant, which corresponds to SEQ ID NO: 1 and the nucleotide sequence of SEQ ID NO: 1.

While the aforementioned genetic features can be detected by PCR method, TaqMan PCR method, sequencing method, microarray method, Invader assay, TILLING assay, RAD (random amplified polymorphic DNA) method, restriction fragment length polymorphism (RFLP) method, PCR-SSCP method, AFLP (amplified fragment length polymorphism) method, SSLP (simple sequence length polymorphism) method, CAPS (cleaved amplified polymorphic sequence) method, dCAPS (derived cleaved amplified polymorphic sequence) method, allele-specific oligonucleotide (ASO) method, ARMS method, denaturing gradient gel electrophoresis (DGGE) method, CCM (chemical cleavage of mismatch) method, DOL method, MALDI-TOF/MS method, TDI method, padlock probe assay, molecular beacon assay, DASH (dynamic allele specific hybridization) method, UCAN method, ECA method, PINPOINT method, PROBE (primer oligo base extension) method, VSET (very short extension) method, Survivor assay, Sniper assay, Luminex assay, GOOD method, LCx method, SNaPshot method, Mass ARRAY assay, pyrosequencing method, SNP-IT method, melting curve analysis or the like, the detection method is not limited thereto. A more specific method of detecting the genetic features of the present invention is described in "8. Screening method and kit for plant comprising novel steviol glycoside", Examples, and the like.

"The portion consisting of a nucleotide sequence corresponding to SEQ ID NO: 1" means, for instance, a portion consisting of a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the nucleotide sequence of SEQ ID NO: 1.

In some aspects, "the portion consisting of a nucleotide sequence corresponding to SEQ ID NO: 1" includes a portion of the genome of a stevia plant which can be amplified by PCR using a forward primer hybridizing to a complementary sequence of a portion of 15 to 25 base long from the 5' end of SEQ ID NO: 1 and a reverse primer hybridizing to a portion of 15 to 25 base long from the 3' end of SEQ ID NO: 1.

In a specific aspect, the "part consisting of a nucleotide sequence corresponding to SEQ ID NO: 1" may include, for example, a part of the genome of the stevia plant which can be amplified by PCR using a forward primer containing the nucleotide sequence represented by SEQ ID NO:51 and a reverse primer containing the nucleotide sequence represented by SEQ ID NO:52.

In a specific aspect, an "allele wherein the base at the position corresponding to position 298 of SEQ ID NO:1 is T" includes the nucleotide sequence represented by SEQ ID NO:4, 6 or 8.

In a specific aspect, an "allele wherein the base at the position corresponding to position 328 of SEQ ID NO:1 is C" includes the nucleotide sequence represented by SEQ ID NO:10, 12 or 14.

In a specific aspect, an "allele wherein the base at the position corresponding to position 360 of SEQ ID NO:1 is T" includes the nucleotide sequence represented by SEQ ID NO:16, 18 or 20.

In a specific aspect, an "allele wherein the base at the position corresponding to position 386 of SEQ ID NO:1 is T" includes the nucleotide sequence represented by SEQ ID NO:22, 24 or 26.

In a specific aspect, an "allele wherein the base at the position corresponding to position 393 of SEQ ID NO:1 is T" includes the nucleotide sequence represented by SEQ ID NO:28, 30 or 32.

In a specific aspect, an "allele wherein the base at the position corresponding to position 411 of SEQ ID NO:1 is T" includes the nucleotide sequence represented by SEQ ID NO:34, 36 or 38.

In a specific aspect, an "allele wherein the base at the position corresponding to position 427 of SEQ ID NO:1 is C" includes the nucleotide sequence represented by SEQ ID NO:40, 42 or 44.

In a specific aspect, an "allele wherein the base at the position corresponding to position 453 of SEQ ID NO:1 is T" includes the nucleotide sequence represented by SEQ ID NO:46, 48 or 50.

Here, a position selected from the group consisting of (1) a position corresponding to position 298 of SEQ ID NO: 1, (2) a position corresponding to position 328 of SEQ ID NO: 1, (3) a position corresponding to position 360 of SEQ ID NO: 1, (4) a position corresponding to position 386 of SEQ ID NO: 1, (5) a position corresponding to position 393 of SEQ ID NO: 1, (6) a position corresponding to position 411 of SEQ ID NO: 1, (7) a position corresponding to position 427 of SEQ ID NO: 1, and (8) a position corresponding to position 453 of SEQ ID NO: 1, may be generically referred to as a "polymorphic site of the present invention" or a "variation site of the present invention".

Also, a variation selected from the group consisting of (1) a variation from C to T at a position corresponding to position 298 of SEQ ID NO: 1, (2) a variation from A to C at a position corresponding to position 328 of SEQ ID NO: 1, (3) a variation from C to T at a position corresponding to position 360 of SEQ ID NO: 1, (4) a variation from C to T at a position corresponding to position 386 of SEQ ID NO: 1, (5) a variation from C to T at a position corresponding to position 393 of SEQ ID NO: 1, (6) a variation from C to T at a position corresponding to position 411 of SEQ ID NO: 1, (7) a variation from A to C at a position corresponding to position 427 of SEQ ID NO: 1, and (8) a variation from C to T at a position corresponding to position 453 of SEQ ID NO: 1, may be generically referred to as a "polymorphism of the present invention" or a "variation of the present invention".

In another aspect, the plant of the present invention expresses a gene encoding a protein having the amino acid sequence of SEQ ID NO: 97 (herein, sometimes referred to as "the gene of the present invention"). In a specific aspect, the gene has the nucleotide sequence of SEQ ID NO: 98. A method of detecting the gene is described in "8. Screening method and kit for plant comprising novel steviol glycoside", Examples, and the like.

In a preferable aspect, the plant of the present invention expresses the gene of the present invention at a higher level than a plant not having the variation of the present invention (for example, wild type line). In a specific aspect, the plant of the present invention expresses the gene of the present invention at a level 10 time or more, 25 time or more, 50 time or more, 100 time or more, 150 time or more, 200 time or more, 250 time or more, 300 time or more, 350 time or more, 400 time or more, 450 time or more, 500 time or more, 550 time or more, 600 time or more, 650 time or more, 700 time or more, 750 time or more, 800 time or more, 850 time or more, 900 time or more, 950 time or more, or 1000 time or more as large as a plant not having the variation of the present invention.

In another preferable aspect, the plant of the present invention expresses the gene of the present invention at a higher level than ubiquitin gene. In a specific aspect, the plant of the present invention expresses the gene of the present invention at a level larger by 5.0% or more, 7.5% or more, 10.0% or more, 12.5% or more, 15.0% or more, 17.5% or more, 20.0% or more, 22.5% or more, 25.0% or more, 27.5% or more, 30.0% or more, 32.5% or more, 35.0% or more, 37.5% or more, 40.0% or more, 42.5% or more, or 45.0% or more than ubiquitin gene.

Furthermore, the phrase "0.050 wt% or more relative to the amount of the total steviol glycosides contained in the leaf' means that the glycoside of the present invention exists at a percentage of 0.050 wt% or more with respect to the amount of the total steviol glycosides existing in the liquid extract derived from the dried leaf of the stevia plant of the present invention. Here, the total steviol glycosides does not contain any unknown steviol glycoside or any steviol glycoside existing in an amount less than the detection limit. Preferably, the total steviol glycosides consist of rebaudioside A, rebaudioside B, rebaudioside C, rebaudioside D, rebaudioside E, rebaudioside F, rebaudioside G, rebaudioside I, rebaudioside M, rebaudioside N, stevioside, dulcoside A, steviol bioside, rubusoside and novel steviol glycosides (Novel steviol glycoside A and/or Novel steviol glycoside B). The content of Novel steviol glycoside A or B in the leaf of the stevia plant of the present invention may be 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total steviol glycosides.

Alternatively, the content of Novel steviol glycoside A or B in the leaf of the stevia plant of the present invention may be 0.050 wt% or more, 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total content of rebaudioside A, rebaudioside B, rebaudioside C, rebaudioside D, rebaudioside F, rebaudioside N, rebaudioside M and stevioside contained in the leaf.

Here, the dried leaf of the plant of the present invention refer to those obtained by drying a fresh leaf of the plant of the present invention to reduce their water content to be 10 wt% or less, 7 wt% or less, 5 wt% or less, 4 wt% or less, 3 wt% or less, 2 wt% or less, or 1 wt% or less. Preferably, the water content of the dried leaf of the plant of the present invention is 3-4 wt%.

The genetic features of the present invention in the plant of the present invention may be generated by a genetic modification approach or may be generated by a non-genetic modification approach. Therefore, the plant of the present invention may be a plant obtained by a genetic modification approach or a progeny plant thereof (hereinafter, may be referred to as a "genetically modified plant"), or a plant obtained by a non-genetic modification approach or a progeny plant thereof (hereinafter, may be referred to as a "non-genetically modified plant"). In a preferred aspect, the plant of the present invention is a non-genetically modified plant. In a specific aspect, the plant of the present invention includes a stevia plant subjected to mutagenesis treatment and a progeny plant thereof. The mutagenesis treatment is specifically described in "7. Method of producing the plant of the present invention".

The plant of the present invention not only comprises the whole plant but may also comprise plant organs (for example, leaf, petal, stem, root, seed, etc.), plant tissues (for example, epidermis, phloem, parenchyma, xylem, vascular bundles, palisade tissue, spongy tissue, etc.), various forms of plant cells (for example, suspension cultured cells), a protoplast, a leaf piece, callus and the like.

In addition, the plant of the present invention may also comprise a tissue culture or a plant cell culture. This is because such a tissue culture or plant cell culture can be cultured to regenerate a plant. Examples of the tissue culture or the plant cell culture of the plant of the present invention include, but not limited to, an embryo, meristematic cells, pollen, a leaf, a root, a root apex, a petal, a protoplast, a leaf piece and callus.

An extract of the plant of the present invention can be obtained by allowing a fresh or dried leaf of the plant of the present invention to react with an appropriate solvent (an aqueous solvent such as water or an organic solvent such as alcohol, ether or acetone). For the extraction conditions, see the method described in WO2016/090460 or the method described in the example below.

Preferably, the extract of the plant of the present invention contains the glycoside of the present invention for 0.050 wt% or more relative to the total steviol glycosides. In other aspect of the present invention, the content of the glycoside of the present invention may be 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total steviol glycosides.

Alternatively, the content of Novel steviol glycoside A or B in the extract of the plant of the present invention may be 0.050 wt% or more, 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total content of rebaudioside A, rebaudioside B, rebaudioside C, rebaudioside D, rebaudioside F, rebaudioside N, rebaudioside M and stevioside.

In one aspect, the content of novel steviol glycoside A or novel steviol glycoside B comprised in a leaf or extract of the plant of the present invention may be 0.09 wt% or more, 0.10 wt% or more, 0.11 wt% or more, 0.12 wt% or more, 0.13 wt% or more, 0.14 wt% or more, 0.15 wt% or more, 0.16 wt% or more, 0.17 wt% or more, 0.18 wt% or more, 0.19 wt% or more, 0.20 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less based on the content of rebaudioside A.

In one aspect, the content of novel steviol glycoside A or novel steviol glycoside B comprised in a leaf or extract of the plant of the present invention may be 0.03 wt% or more, 0.04 wt% or more, 0.05 wt% or more, 0.06 wt% or more, 0.07 wt% or more, 0.08 wt% or more, 0.09 wt% or more, 0.10 wt% or more, 0.11 wt% or more, 0.12 wt% or more, 0.13 wt% or more, 0.14 wt% or more, 0.15 wt% or more, 0.16 wt% or more, 0.17 wt% or more, 0.18 wt% or more, 0.19 wt% or more, 0.20 wt% or more, 0.30 wt% or more, 0.40 wt% or more, 0.50 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less based on the content of stevioside.

In another aspect, the content of novel steviol glycoside B comprised in a leaf or extract of the plant of the present invention may be 5 wt% or more, 8 wt% or more, 10 wt% or more, 13 wt% or more, 15 wt% or more, 18 wt% or more, 20 wt% or more, 23 wt% or more, 25 wt% or more, 28 wt% or more, 30 wt% or more, 32 wt% or more, 35 wt% or more, 38 wt% or more, 40 wt% or more, 43 wt% or more, 45 wt% or more, 48 wt% or more, 50 wt% or more, and 100 wt% or less, 90 wt% or less, 85 wt% or less, 80 wt% or less, 75 wt% or less, 70 wt% or less, 65 wt% or less, 60 wt% or less based on the content of stevioside.

In one aspect, the content of novel steviol glycoside A or novel steviol glycoside B comprised in a leaf or extract of the plant of the present invention may be 0.03 wt% or more, 0.04 wt% or more, 0.05 wt% or more, 0.06 wt% or more, 0.07 wt% or more, 0.08 wt% or more, 0.09 wt% or more, 0.10 wt% or more, 0.11 wt% or more, 0.12 wt% or more, 0.13 wt% or more, 0.14 wt% or more, 0.15 wt% or more, 0.16 wt% or more, 0.17 wt% or more, 0.18 wt% or more, 0.19 wt% or more, 0.20 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less, 0.50 wt% or less based on the total content of rebaudioside A and stevioside.

In a specific aspect, the ratio of the content of novel steviol glycoside A or novel steviol glycoside B based on the total amount of steviol glycoside comprised in the leaf or extract of the plant of the present invention may be 0.055 to 10.00 wt%, 0.060 to 10.00 wt%, 0.065 to 10.00 wt%, 0.070 to 10.00 wt%, 0.075 to 10.00 wt%, 0.080 to 10.00 wt%, 0.085 to 10.00 wt%, 0.090 to 10.00 wt%, 0.095 to 10.00 wt%, 0.10 to 10.00 wt%, 0.15 to 10.00 wt%, 0.20 to 10.00 wt%, 0.30 to 10.00 wt%, 0.50 to 10.00 wt%, 0.60 to 10.00 wt%, 0.80 to 10.00 wt%, 1.00 to 10.00 wt%, 2.00 to 10.00 wt%, 4.00 to 10.00 wt%, 6.00 to 10.00 wt%, 8.00 to 10.00 wt%, 0.055 to 8.00 wt%, 0.060 to 8.00 wt%, 0.065 to 8.00 wt%, 0.070 to 8.00 wt%, 0.075 to 8.00 wt%, 0.080 to 8.00 wt%, 0.085 to 8.00 wt%, 0.090 to 8.00 wt%, 0.095 to 8.00 wt%, 0.10 to 8.00 wt%, 0.15 to 8.00 wt%, 0.20 to 8.00 wt%, 0.30 to 8.00 wt%, 0.50 to 8.00 wt%, 0.60 to 8.00 wt%, 0.80 to 8.00 wt%, 1.00 to 8.00 wt%, 2.00 to 8.00 wt%, 4.00 to 8.00 wt%, 6.00 to 8.00 wt%, 0.055 to 6.00 wt%, 0.060 to 6.00 wt%, 0.065 to 6.00 wt%, 0.070 to 6.00 wt%, 0.075 to 6.00 wt%, 0.080 to 6.00 wt%, 0.085 to 6.00 wt%, 0.090 to 6.00 wt%, 0.095 to 6.00 wt%, 0.10 to 6.00 wt%, 0.15 to 6.00 wt%, 0.20 to 6.00 wt%, 0.30 to 6.00 wt%, 0.50 to 6.00 wt%, 0.60 to 6.00 wt%, 0.80 to 6.00 wt%, 1.00 to 6.00 wt%, 2.00 to 6.00 wt%, 4.00 to 6.00 wt%, 0.055 to 4.00 wt%, 0.060 to 4.00 wt%, 0.065 to 4.00 wt%, 0.070 to 4.00 wt%, 0.075 to 4.00 wt%, 0.080 to 4.00 wt%, 0.085 to 4.00 wt%, 0.090 to 4.00 wt%, 0.095 to 4.00 wt%, 0.10 to 4.00 wt%, 0.15 to 4.00 wt%, 0.20 to 4.00 wt%, 0.30 to 4.00 wt%, 0.50 to 4.00 wt%, 0.60 to 4.00 wt%, 0.80 to 4.00 wt%, 1.00 to 4.00 wt%, 2.00 to 4.00 wt%, 0.055 to 2.00 wt%, 0.060 to 2.00 wt%, 0.065 to 2.00 wt%, 0.070 to 2.00 wt%, 0.075 to 2.00 wt%, 0.080 to 2.00 wt%, 0.085 to 2.00 wt%, 0.090 to 2.00 wt%, 0.095 to 2.00 wt%, 0.10 to 2.00 wt%, 0.15 to 2.00 wt%, 0.20 to 2.00 wt%, 0.30 to 2.00 wt%, 0.50 to 2.00 wt%, 0.60 to 2.00 wt%, 0.80 to 2.00 wt%, 1.00 to 2.00 wt%, 0.055 to 1.00 wt%, 0.060 to 1.00 wt%, 0.065 to 1.00 wt%, 0.070 to 1.00 wt%, 0.075 to 1.00 wt%, 0.080 to 1.00 wt%, 0.085 to 1.00 wt%, 0.090 to 1.00 wt%, 0.095 to 1.00 wt%, 0.10 to 1.00 wt%, 0.15 to 1.00 wt%, 0.20 to 1.00 wt%, 0.30 to 1.00 wt%, 0.50 to 1.00 wt%, 0.60 to 1.00 wt%, 0.80 to 1.00 wt%, 0.055 to 0.80 wt%, 0.060 to 0.80 wt%, 0.065 to 0.80 wt%, 0.070 to 0.80 wt%, 0.075 to 0.80 wt%, 0.080 to 0.80 wt%, 0.085 to 0.80 wt%, 0.090 to 0.80 wt%, 0.095 to 0.80 wt%, 0.10 to 0.80 wt%, 0.15 to 0.80 wt%, 0.20 to 0.80 wt%, 0.30 to 0.80 wt%, 0.50 to 0.80 wt%, 0.60 to 0.80 wt%, 0.055 to 0.60 wt%, 0.060 to 0.60 wt%, 0.065 to 0.60 wt%, 0.070 to 0.60 wt%, 0.075 to 0.60 wt%, 0.080 to 0.60 wt%, 0.085 to 0.60 wt%, 0.090 to 0.60 wt%, 0.095 to 0.60 wt%, 0.10 to 0.60 wt%, 0.15 to 0.60 wt%, 0.20 to 0.60 wt%, 0.30 to 0.60 wt%, 0.50 to 0.60 wt%, 0.055 to 0.50 wt%, 0.060 to 0.50 wt%, 0.065 to 0.50 wt%, 0.070 to 0.50 wt%, 0.075 to 0.50 wt%, 0.080 to 0.50 wt%, 0.085 to 0.50 wt%, 0.090 to 0.50 wt%, 0.095 to 0.50 wt%, 0.10 to 0.50 wt%, 0.15 to 0.50 wt%, 0.20 to 0.50 wt%, 0.30 to 0.50 wt%, 0.50 to 0.30 wt%, 0.055 to 0.30 wt%, 0.060 to 0.30 wt%, 0.065 to 0.30 wt%, 0.070 to 0.30 wt%, 0.075 to 0.30 wt%, 0.080 to 0.30 wt%, 0.085 to 0.30 wt%, 0.090 to 0.30 wt%, 0.095 to 0.30 wt%, 0.10 to 0.30 wt%, 0.15 to 0.30 wt%, or 0.20 to 0.30 wt%.

In another specific aspect, the ratio of the content of novel steviol glycoside A or novel steviol glycoside B based on the total amount of rebaudioside A, rebaudioside B, rebaudioside C, rebaudioside D, rebaudioside F, rebaudioside N, rebaudioside M and stevioside comprised in the leaf or extract of the plant of the present invention may be 0.050 to 10.00 wt%, 0.055 to 10.00 wt%, 0.060 to 10.00 wt%, 0.065 to 10.00 wt%, 0.070 to 10.00 wt%, 0.075 to 10.00 wt%, 0.080 to 10.00 wt%, 0.085 to 10.00 wt%, 0.090 to 10.00 wt%, 0.095 to 10.00 wt%, 0.10 to 10.00 wt%, 0.15 to 10.00 wt%, 0.20 to 10.00 wt%, 0.30 to 10.00 wt%, 0.50 to 10.00 wt%, 0.60 to 10.00 wt%, 0.80 to 10.00 wt%, 1.00 to 10.00 wt%, 2.00 to 10.00 wt%, 4.00 to 10.00 wt%, 6.00 to 10.00 wt%, 8.00 to 10.00 wt%, 0.050 to 8.00 wt%, 0.055 to 8.00 wt%, 0.060 to 8.00 wt%, 0.065 to 8.00 wt%, 0.070 to 8.00 wt%, 0.075 to 8.00 wt%, 0.080 to 8.00 wt%, 0.085 to 8.00 wt%, 0.090 to 8.00 wt%, 0.095 to 8.00 wt%, 0.10 to 8.00 wt%, 0.15 to 8.00 wt%, 0.20 to 8.00 wt%, 0.30 to 8.00 wt%, 0.50 to 8.00 wt%, 0.60 to 8.00 wt%, 0.80 to 8.00 wt%, 1.00 to 8.00 wt%, 2.00 to 8.00 wt%, 4.00 to 8.00 wt%, 6.00 to 8.00 wt%, 0.050 to 6.00 wt%, 0.055 to 6.00 wt%, 0.060 to 6.00 wt%, 0.065 to 6.00 wt%, 0.070 to 6.00 wt%, 0.075 to 6.00 wt%, 0.080 to 6.00 wt%, 0.085 to 6.00 wt%, 0.090 to 6.00 wt%, 0.095 to 6.00 wt%, 0.10 to 6.00 wt%, 0.15 to 6.00 wt%, 0.20 to 6.00 wt%, 0.30 to 6.00 wt%, 0.50 to 6.00 wt%, 0.60 to 6.00 wt%, 0.80 to 6.00 wt%, 1.00 to 6.00 wt%, 2.00 to 6.00 wt%, 4.00 to 6.00 wt%, 0.050 to 4.00 wt%, 0.055 to 4.00 wt%, 0.060 to 4.00 wt%, 0.065 to 4.00 wt%, 0.070 to 4.00 wt%, 0.075 to 4.00 wt%, 0.080 to 4.00 wt%, 0.085 to 4.00 wt%, 0.090 to 4.00 wt%, 0.095 to 4.00 wt%, 0.10 to 4.00 wt%, 0.15 to 4.00 wt%, 0.20 to 4.00 wt%, 0.30 to 4.00 wt%, 0.50 to 4.00 wt%, 0.60 to 4.00 wt%, 0.80 to 4.00 wt%, 1.00 to 4.00 wt%, 2.00 to 4.00 wt%, 0.050 to 2.00 wt%, 0.055 to 2.00 wt%, 0.060 to 2.00 wt%, 0.065 to 2.00 wt%, 0.070 to 2.00 wt%, 0.075 to 2.00 wt%, 0.080 to 2.00 wt%, 0.085 to 2.00 wt%, 0.090 to 2.00 wt%, 0.095 to 2.00 wt%, 0.10 to 2.00 wt%, 0.15 to 2.00 wt%, 0.20 to 2.00 wt%, 0.30 to 2.00 wt%, 0.50 to 2.00 wt%, 0.60 to 2.00 wt%, 0.80 to 2.00 wt%, 1.00 to 2.00 wt%, 0.050 to 1.00 wt%, 0.055 to 1.00 wt%, 0.060 to 1.00 wt%, 0.065 to 1.00 wt%, 0.070 to 1.00 wt%, 0.075 to 1.00 wt%, 0.080 to 1.00 wt%, 0.085 to 1.00 wt%, 0.090 to 1.00 wt%, 0.095 to 1.00 wt%, 0.10 to 1.00 wt%, 0.15 to 1.00 wt%, 0.20 to 1.00 wt%, 0.30 to 1.00 wt%, 0.50 to 1.00 wt%, 0.60 to 1.00 wt%, 0.80 to 1.00 wt%, 0.050 to 0.80 wt%, 0.055 to 0.80 wt%, 0.060 to 0.80 wt%, 0.065 to 0.80 wt%, 0.070 to 0.80 wt%, 0.075 to 0.80 wt%, 0.080 to 0.80 wt%, 0.085 to 0.80 wt%, 0.090 to 0.80 wt%, 0.095 to 0.80 wt%, 0.10 to 0.80 wt%, 0.15 to 0.80 wt%, 0.20 to 0.80 wt%, 0.30 to 0.80 wt%, 0.50 to 0.80 wt%, 0.60 to 0.80 wt%, 0.050 to 0.60 wt%, 0.055 to 0.60 wt%, 0.060 to 0.60 wt%, 0.065 to 0.60 wt%, 0.070 to 0.60 wt%, 0.075 to 0.60 wt%, 0.080 to 0.60 wt%, 0.085 to 0.60 wt%, 0.090 to 0.60 wt%, 0.095 to 0.60 wt%, 0.10 to 0.60 wt%, 0.15 to 0.60 wt%, 0.20 to 0.60 wt%, 0.30 to 0.60 wt%, 0.50 to 0.60 wt%, 0.050 to 0.50 wt%, 0.055 to 0.50 wt%, 0.060 to 0.50 wt%, 0.065 to 0.50 wt%, 0.070 to 0.50 wt%, 0.075 to 0.50 wt%, 0.080 to 0.50 wt%, 0.085 to 0.50 wt%, 0.090 to 0.50 wt%, 0.095 to 0.50 wt%, 0.10 to 0.50 wt%, 0.15 to 0.50 wt%, 0.20 to 0.50 wt%, 0.30 to 0.50 wt%, 0.50 to 0.30 wt%, 0.050 to 0.30 wt%, 0.055 to 0.30 wt%, 0.060 to 0.30 wt%, 0.065 to 0.30 wt%, 0.070 to 0.30 wt%, 0.075 to 0.30 wt%, 0.080 to 0.30 wt%, 0.085 to 0.30 wt%, 0.090 to 0.30 wt%, 0.095 to 0.30 wt%, 0.10 to 0.30 wt%, 0.15 to 0.30 wt%, or 0.20 to 0.30 wt%.

In another specific aspect, the ratio of the content of novel steviol glycoside A or novel steviol glycoside B based on the amount of rebaudioside A comprised in the leaf or extract of the plant of the present invention may be 0.09 to 10.00 wt%, 0.10 to 10.00 wt%, 0.11 to 10.00 wt%, 0.12 to 10.00 wt%, 0.13 to 10.00 wt%, 0.14 to 10.00 wt%, 0.15 to 10.00 wt%, 0.16 to 10.00 wt%, 0.17 to 10.00 wt%, 0.18 to 10.00 wt%, 0.19 to 10.00 wt%, 0.20 to 10.00 wt%, 0.09 to 8.00 wt%, 0.10 to 8.00 wt%, 0.11 to 8.00 wt%, 0.12 to 8.00 wt%, 0.13 to 8.00 wt%, 0.14 to 8.00 wt%, 0.15 to 8.00 wt%, 0.16 to 8.00 wt%, 0.17 to 8.00 wt%, 0.18 to 8.00 wt%, 0.19 to 8.00 wt%, 0.20 to 8.00 wt%, 0.09 to 6.00 wt%, 0.10 to 6.00 wt%, 0.11 to 6.00 wt%, 0.12 to 6.00 wt%, 0.13 to 6.00 wt%, 0.14 to 6.00 wt%, 0.15 to 6.00 wt%, 0.16 to 6.00 wt%, 0.17 to 6.00 wt%, 0.18 to 6.00 wt%, 0.19 to 6.00 wt%, 0.20 to 6.00 wt%, 0.09 to 4.00 wt%, 0.10 to 4.00 wt%, 0.11 to 4.00 wt%, 0.12 to 4.00 wt%, 0.13 to 4.00 wt%, 0.14 to 4.00 wt%, 0.15 to 4.00 wt%, 0.16 to 4.00 wt%, 0.17 to 4.00 wt%, 0.18 to 4.00 wt%, 0.19 to 4.00 wt%, 0.20 to 4.00 wt%, 0.09 to 2.00 wt%, 0.10 to 2.00 wt%, 0.11 to 2.00 wt%, 0.12 to 2.00 wt%, 0.13 to 2.00 wt%, 0.14 to 2.00 wt%, 0.15 to 2.00 wt%, 0.16 to 2.00 wt%, 0.17 to 2.00 wt%, 0.18 to 2.00 wt%, 0.19 to 2.00 wt%, 0.20 to 2.00 wt%, 0.09 to 1.00 wt%, 0.10 to 1.00 wt%, 0.11 to 1.00 wt%, 0.12 to 1.00 wt%, 0.13 to 1.00 wt%, 0.14 to 1.00 wt%, 0.15 to 1.00 wt%, 0.16 to 1.00 wt%, 0.17 to 1.00 wt%, 0.18 to 1.00 wt%, 0.19 to 1.00 wt%, 0.20 to 1.00 wt%, 0.09 to 0.80 wt%, 0.10 to 0.80 wt%, 0.11 to 0.80 wt%, 0.12 to 0.80 wt%, 0.13 to 0.80 wt%, 0.14 to 0.80 wt%, 0.15 to 0.80 wt%, 0.16 to 0.80 wt%, 0.17 to 0.80 wt%, 0.18 to 0.80 wt%, 0.19 to 0.80 wt%, 0.20 to 0.80 wt%, 0.09 to 0.60 wt%, 0.10 to 0.60 wt%, 0.11 to 0.60 wt%, 0.12 to 0.60 wt%, 0.13 to 0.60 wt%, 0.14 to 0.60 wt%, 0.15 to 0.60 wt%, 0.16 to 0.60 wt%, 0.17 to 0.60 wt%, 0.18 to 0.60 wt%, 0.19 to 0.60 wt%, or 0.20 to 0.60 wt%.

In another specific aspect, the ratio of the content of novel steviol glycoside A or novel steviol glycoside B based on the amount of stevioside comprised in the leaf or extract of the plant of the present invention may be 0.03 to 10.00 wt%, 0.04 to 10.00 wt%, 0.05 to 10.00 wt%, 0.06 to 10.00 wt%, 0.07 to 10.00 wt%, 0.08 to 10.00 wt%, 0.09 to 10.00 wt%, 0.10 to 10.00 wt%, 0.11 to 10.00 wt%, 0.12 to 10.00 wt%, 0.13 to 10.00 wt%, 0.14 to 10.00 wt%, 0.15 to 10.00 wt%, 0.16 to 10.00 wt%, 0.17 to 10.00 wt%, 0.18 to 10.00 wt%, 0.19 to 10.00 wt%, 0.20 to 10.00 wt%, 0.30 to 10.00 wt%, 0.40 to 10.00 wt%, 0.50 to 10.00 wt%, 0.03 to 8.00 wt%, 0.04 to 8.00 wt%, 0.05 to 8.00 wt%, 0.06 to 8.00 wt%, 0.07 to 8.00 wt%, 0.08 to 8.00 wt%, 0.09 to 8.00 wt%, 0.10 to 8.00 wt%, 0.11 to 8.00 wt%, 0.12 to 8.00 wt%, 0.13 to 8.00 wt%, 0.14 to 8.00 wt%, 0.15 to 8.00 wt%, 0.16 to 8.00 wt%, 0.17 to 8.00 wt%, 0.18 to 8.00 wt%, 0.19 to 8.00 wt%, 0.20 to 8.00 wt%, 0.30 to 8.00 wt%, 0.40 to 8.00 wt%, 0.50 to 8.00 wt%, 0.03 to 6.00 wt%, 0.04 to 6.00 wt%, 0.05 to 6.00 wt%, 0.06 to 6.00 wt%, 0.07 to 6.00 wt%, 0.08 to 6.00 wt%, 0.09 to 6.00 wt%, 0.10 to 6.00 wt%, 0.11 to 6.00 wt%, 0.12 to 6.00 wt%, 0.13 to 6.00 wt%, 0.14 to 6.00 wt%, 0.15 to 6.00 wt%, 0.16 to 6.00 wt%, 0.17 to 6.00 wt%, 0.18 to 6.00 wt%, 0.19 to 6.00 wt%, 0.20 to 6.00 wt%, 0.30 to 6.00 wt%, 0.40 to 6.00 wt%, 0.50 to 6.00 wt%, 0.03 to 4.00 wt%, 0.04 to 4.00 wt%, 0.05 to 4.00 wt%, 0.06 to 4.00 wt%, 0.07 to 4.00 wt%, 0.08 to 4.00 wt%, 0.09 to 4.00 wt%, 0.10 to 4.00 wt%, 0.11 to 4.00 wt%, 0.12 to 4.00 wt%, 0.13 to 4.00 wt%, 0.14 to 4.00 wt%, 0.15 to 4.00 wt%, 0.16 to 4.00 wt%, 0.17 to 4.00 wt%, 0.18 to 4.00 wt%, 0.19 to 4.00 wt%, 0.20 to 4.00 wt%, 0.30 to 4.00 wt%, 0.40 to 4.00 wt%, 0.50 to 4.00 wt%, 0.03 to 2.00 wt%, 0.04 to 2.00 wt%, 0.05 to 2.00 wt%, 0.06 to 2.00 wt%, 0.07 to 2.00 wt%, 0.08 to 2.00 wt%, 0.09 to 2.00 wt%, 0.10 to 2.00 wt%, 0.11 to 2.00 wt%, 0.12 to 2.00 wt%, 0.13 to 2.00 wt%, 0.14 to 2.00 wt%, 0.15 to 2.00 wt%, 0.16 to 2.00 wt%, 0.17 to 2.00 wt%, 0.18 to 2.00 wt%, 0.19 to 2.00 wt%, 0.20 to 2.00 wt%, 0.30 to 2.00 wt%, 0.40 to 2.00 wt%, 0.50 to 2.00 wt%, 0.03 to 1.00 wt%, 0.04 to 1.00 wt%, 0.05 to 1.00 wt%, 0.06 to 1.00 wt%, 0.07 to 1.00 wt%, 0.08 to 1.00 wt%, 0.09 to 1.00 wt%, 0.10 to 1.00 wt%, 0.11 to 1.00 wt%, 0.12 to 1.00 wt%, 0.13 to 1.00 wt%, 0.14 to 1.00 wt%, 0.15 to 1.00 wt%, 0.16 to 1.00 wt%, 0.17 to 1.00 wt%, 0.18 to 1.00 wt%, 0.19 to 1.00 wt%, 0.20 to 1.00 wt%, 0.30 to 1.00 wt%, 0.40 to 1.00 wt%, 0.50 to 1.00 wt%, 0.03 to 0.80 wt%, 0.04 to 0.80 wt%, 0.05 to 0.80 wt%, 0.06 to 0.80 wt%, 0.07 to 0.80 wt%, 0.08 to 0.80 wt%, 0.09 to 0.80 wt%, 0.10 to 0.80 wt%, 0.11 to 0.80 wt%, 0.12 to 0.80 wt%, 0.13 to 0.80 wt%, 0.14 to 0.80 wt%, 0.15 to 0.80 wt%, 0.16 to 0.80 wt%, 0.17 to 0.80 wt%, 0.18 to 0.80 wt%, 0.19 to 0.80 wt%, 0.20 to 0.80 wt%, 0.30 to 0.80 wt%, 0.40 to 0.80 wt%, 0.50 to 0.80 wt%, 0.03 to 0.60 wt%, 0.04 to 0.60 wt%, 0.05 to 0.60 wt%, 0.06 to 0.60 wt%, 0.07 to 0.60 wt%, 0.08 to 0.60 wt%, 0.09 to 0.60 wt%, 0.10 to 0.60 wt%, 0.11 to 0.60 wt%, 0.12 to 0.60 wt%, 0.13 to 0.60 wt%, 0.14 to 0.60 wt%, 0.15 to 0.60 wt%, 0.16 to 0.60 wt%, 0.17 to 0.60 wt%, 0.18 to 0.60 wt%, 0.19 to 0.60 wt%, 0.20 to 0.60 wt%, 0.30 to 0.60 wt%, 0.40 to 0.60 wt%, or 0.50 to 0.60 wt%.

In another specific aspect, the ratio of the content of novel steviol glycoside A or novel steviol glycoside B based on the amount of stevioside comprised in the leaf or extract of the plant of the present invention may be 5 to 100 wt%, 8 to 100 wt%, 10 to 100 wt%, 13 to 100 wt%, 15 to 100 wt%, 18 to 100 wt%, 20 to 100 wt%, 23 to 100 wt%, 25 to 100 wt%, 28 to 100 wt%, 30 to 100 wt%, 33 to 100 wt%, 35 to 100 wt%, 38 to 100 wt%, 40 to 100 wt%, 43 to 100 wt%, 45 to 100 wt%, 48 to 100 wt%, 50 to 100 wt%, 5 to 90 wt%, 8 to 90 wt%, 10 to 90 wt%, 13 to 90 wt%, 15 to 90 wt%, 18 to 90 wt%, 20 to 90 wt%, 23 to 90 wt%, 25 to 90 wt%, 28 to 90 wt%, 30 to 90 wt%, 33 to 90 wt%, 35 to 90 wt%, 38 to 90 wt%, 40 to 90 wt%, 43 to 90 wt%, 45 to 90 wt%, 48 to 90 wt%, 50 to 90 wt%, 5 to 85 wt%, 8 to 85 wt%, 10 to 85 wt%, 13 to 85 wt%, 15 to 85 wt%, 18 to 85 wt%, 20 to 85 wt%, 23 to 85 wt%, 25 to 85 wt%, 28 to 85 wt%, 30 to 85 wt%, 33 to 85 wt%, 35 to 85 wt%, 38 to 85 wt%, 40 to 85 wt%, 43 to 85 wt%, 45 to 85 wt%, 48 to 85 wt%, 50 to 85 wt%, 5 to 80 wt%, 8 to 80 wt%, 10 to 80 wt%, 13 to 80 wt%, 15 to 80 wt%, 18 to 80 wt%, 20 to 80 wt%, 23 to 80 wt%, 25 to 80 wt%, 28 to 80 wt%, 30 to 80 wt%, 33 to 80 wt%, 35 to 80 wt%, 38 to 80 wt%, 40 to 80 wt%, 43 to 80 wt%, 45 to 80 wt%, 48 to 80 wt%, 50 to 80 wt%, 5 to 75 wt%, 8 to 75 wt%, 10 to 75 wt%, 13 to 75 wt%, 15 to 75 wt%, 18 to 75 wt%, 20 to 75 wt%, 23 to 75 wt%, 25 to 75 wt%, 28 to 75 wt%, 30 to 75 wt%, 33 to 75 wt%, 35 to 75 wt%, 38 to 75 wt%, 40 to 75 wt%, 43 to 75 wt%, 45 to 75 wt%, 48 to 75 wt%, 50 to 75 wt%, 5 to 70 wt%, 8 to 70 wt%, 10 to 70 wt%, 13 to 70 wt%, 15 to 70 wt%, 18 to 70 wt%, 20 to 70 wt%, 23 to 70 wt%, 25 to 70 wt%, 28 to 70 wt%, 30 to 70 wt%, 33 to 70 wt%, 35 to 70 wt%, 38 to 70 wt%, 40 to 70 wt%, 43 to 70 wt%, 45 to 70 wt%, 48 to 70 wt%, 50 to 70 wt%, 5 to 65 wt%, 8 to 65 wt%, 10 to 65 wt%, 13 to 65 wt%, 15 to 65 wt%, 18 to 65 wt%, 20 to 65 wt%, 23 to 65 wt%, 25 to 65 wt%, 28 to 65 wt%, 30 to 65 wt%, 33 to 65 wt%, 35 to 65 wt%, 38 to 65 wt%, 40 to 65 wt%, 43 to 65 wt%, 45 to 65 wt%, 48 to 65 wt%, 50 to 65 wt%, 5 to 60 wt%, 8 to 60 wt%, 10 to 60 wt%, 13 to 60 wt%, 15 to 60 wt%, 18 to 60 wt%, 20 to 60 wt%, 23 to 60 wt%, 25 to 60 wt%, 28 to 60 wt%, 30 to 60 wt%, 33 to 60 wt%, 35 to 60 wt%, 38 to 60 wt%, 40 to 60 wt%, 43 to 60 wt%, 45 to 60 wt%, 48 to 60 wt%, or 50 to 60 wt%.

In another specific aspect, the ratio of the content of novel steviol glycoside A or novel steviol glycoside B based on the total amount of rebaudioside A and stevioside comprised in the leaf or extract of the plant of the present invention may be 0.03 to 10.00 wt%, 0.04 to 10.00 wt%, 0.05 to 10.00 wt%, 0.06 to 10.00 wt%, 0.07 to 10.00 wt%, 0.08 to 10.00 wt%, 0.09 to 10.00 wt%, 0.10 to 10.00 wt%, 0.11 to 10.00 wt%, 0.12 to 10.00 wt%, 0.13 to 10.00 wt%, 0.14 to 10.00 wt%, 0.15 to 10.00 wt%, 0.16 to 10.00 wt%, 0.17 to 10.00 wt%, 0.18 to 10.00 wt%, 0.19 to 10.00 wt%, 0.20 to 10.00 wt%, 0.03 to 8.00 wt%, 0.04 to 8.00 wt%, 0.05 to 8.00 wt%, 0.06 to 8.00 wt%, 0.07 to 8.00 wt%, 0.08 to 8.00 wt%, 0.09 to 8.00 wt%, 0.10 to 8.00 wt%, 0.11 to 8.00 wt%, 0.12 to 8.00 wt%, 0.13 to 8.00 wt%, 0.14 to 8.00 wt%, 0.15 to 8.00 wt%, 0.16 to 8.00 wt%, 0.17 to 8.00 wt%, 0.18 to 8.00 wt%, 0.19 to 8.00 wt%, 0.20 to 8.00 wt%, 0.03 to 6.00 wt%, 0.04 to 6.00 wt%, 0.05 to 6.00 wt%, 0.06 to 6.00 wt%, 0.07 to 6.00 wt%, 0.08 to 6.00 wt%, 0.09 to 6.00 wt%, 0.10 to 6.00 wt%, 0.11 to 6.00 wt%, 0.12 to 6.00 wt%, 0.13 to 6.00 wt%, 0.14 to 6.00 wt%, 0.15 to 6.00 wt%, 0.16 to 6.00 wt%, 0.17 to 6.00 wt%, 0.18 to 6.00 wt%, 0.19 to 6.00 wt%, 0.20 to 6.00 wt%, 0.03 to 4.00 wt%, 0.04 to 4.00 wt%, 0.05 to 4.00 wt%, 0.06 to 4.00 wt%, 0.07 to 4.00 wt%, 0.08 to 4.00 wt%, 0.09 to 4.00 wt%, 0.10 to 4.00 wt%, 0.11 to 4.00 wt%, 0.12 to 4.00 wt%, 0.13 to 4.00 wt%, 0.14 to 4.00 wt%, 0.15 to 4.00 wt%, 0.16 to 4.00 wt%, 0.17 to 4.00 wt%, 0.18 to 4.00 wt%, 0.19 to 4.00 wt%, 0.20 to 4.00 wt%, 0.03 to 2.00 wt%, 0.04 to 2.00 wt%, 0.05 to 2.00 wt%, 0.06 to 2.00 wt%, 0.07 to 2.00 wt%, 0.08 to 2.00 wt%, 0.09 to 2.00 wt%, 0.10 to 2.00 wt%, 0.11 to 2.00 wt%, 0.12 to 2.00 wt%, 0.13 to 2.00 wt%, 0.14 to 2.00 wt%, 0.15 to 2.00 wt%, 0.16 to 2.00 wt%, 0.17 to 2.00 wt%, 0.18 to 2.00 wt%, 0.19 to 2.00 wt%, 0.20 to 2.00 wt%, 0.03 to 1.00 wt%, 0.04 to 1.00 wt%, 0.05 to 1.00 wt%, 0.06 to 1.00 wt%, 0.07 to 1.00 wt%, 0.08 to 1.00 wt%, 0.09 to 1.00 wt%, 0.10 to 1.00 wt%, 0.11 to 1.00 wt%, 0.12 to 1.00 wt%, 0.13 to 1.00 wt%, 0.14 to 1.00 wt%, 0.15 to 1.00 wt%, 0.16 to 1.00 wt%, 0.17 to 1.00 wt%, 0.18 to 1.00 wt%, 0.19 to 1.00 wt%, 0.20 to 1.00 wt%, 0.03 to 0.80 wt%, 0.04 to 0.80 wt%, 0.05 to 0.80 wt%, 0.06 to 0.80 wt%, 0.07 to 0.80 wt%, 0.08 to 0.80 wt%, 0.09 to 0.80 wt%, 0.10 to 0.80 wt%, 0.11 to 0.80 wt%, 0.12 to 0.80 wt%, 0.13 to 0.80 wt%, 0.14 to 0.80 wt%, 0.15 to 0.80 wt%, 0.16 to 0.80 wt%, 0.17 to 0.80 wt%, 0.18 to 0.80 wt%, 0.19 to 0.80 wt%, 0.20 to 0.80 wt%, 0.03 to 0.60 wt%, 0.04 to 0.60 wt%, 0.05 to 0.60 wt%, 0.06 to 0.60 wt%, 0.07 to 0.60 wt%, 0.08 to 0.60 wt%, 0.09 to 0.60 wt%, 0.10 to 0.60 wt%, 0.11 to 0.60 wt%, 0.12 to 0.60 wt%, 0.13 to 0.60 wt%, 0.14 to 0.60 wt%, 0.15 to 0.60 wt%, 0.16 to 0.60 wt%, 0.17 to 0.60 wt%, 0.18 to 0.60 wt%, 0.19 to 0.60 wt%, 0.20 to 0.60 wt%, 0.03 to 0.50 wt%, 0.04 to 0.50 wt%, 0.05 to 0.50 wt%, 0.06 to 0.50 wt%, 0.07 to 0.50 wt%, 0.08 to 0.50 wt%, 0.09 to 0.50 wt%, 0.10 to 0.50 wt%, 0.11 to 0.50 wt%, 0.12 to 0.50 wt%, 0.13 to 0.50 wt%, 0.14 to 0.50 wt%, 0.15 to 0.50 wt%, 0.16 to 0.50 wt%, 0.17 to 0.50 wt%, 0.18 to 0.50 wt%, 0.19 to 0.50 wt%, or 0.20 to 0.50 wt%.

In one aspect of the present invention, a food or beverage comprising the extract of the plant of the present invention is provided. In another aspect of the present invention, the food or beverage is a beverage. Examples of the kinds of the food or beverage include those recited in "3. Food or beverage, flavoring agent and pharmaceutical product comprising novel steviol glycoside".

### 5. Flavor controlling agent comprising novel steviol glycoside

In one aspect of the present invention, a flavor controlling agent comprising the above-described compound represented by Formula (1), or a derivative, a salt or a hydrate thereof is provided. In one aspect of the present invention, a composition composed as described in "2. Sweetener composition comprising novel steviol glycoside" may also be used as a flavor controlling agent.

Herein, a "flavor controlling agent" refers to a substance that can be added to a food or beverage to control the flavor of the food or beverage. Preferably, the flavor controlling agent of the present invention can be added to a food or beverage so as to control the flavor of the food or beverage itself without the consumers recognizing the taste of the flavor controlling agent itself. For example, since the steviol glycoside of the present invention has weaker bitterness as compared to conventional steviol glycosides, it can be used as a flavor controlling agent for controlling the bitterness of the food or beverage.

In addition to the above-described compound represented by Formula (1) or a derivative, a salt or a hydrate thereof, the flavor controlling agent of the present invention preferably comprises one or more types of other sweeteners. Examples of such sweetener include: one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside; natural sweeteners such as fructose, sucrose, fructose-glucose syrup, glucose, maltose, high-fructose syrup, sugar alcohol, oligosaccharide, honey, pressed sugarcane juice (brown sugar syrup), starch syrup, Lo Han Kuo (*Siraitia grosvenorii)* powder, a Lo Han Kuo (*Siraitia grosvenorii)* extract, licorice powder, a licorice extract, *Thaumatococcus daniellii* seed powder and a *Thaumatococcus daniellii* seed extract; and artificial sweeteners such as acesulfame potassium, sucralose, neotame, aspartame and saccharin.

### 6. Method for producing novel steviol glycoside

A plant comprising the novel steviol glycoside is obtained, for example, by obtaining a plant having the aforementioned genetic features by the screening method described below. Thereafter, the novel steviol glycoside can be isolated/purified from the plant. A fresh or dried leaf of the plant of the present invention is allowed to react with an appropriate solvent (an aqueous solvent such as water or an organic solvent such as alcohol, ether or acetone) to extract the novel steviol glycoside in a liquid extract state. For extraction conditions and else, see the method described in WO2016/090460 or the method described in the example below.

Furthermore, the resulting liquid extract may be subjected to a known method such as a gradient of ethyl acetate or other organic solvent: water, HPLC or UPLC to isolate/purify the novel steviol glycoside.

The content of the novel steviol glycoside in the plant can be determined by the method described in WO2016/090460 or the method described in the example below. Specifically, the content can be determined by sampling a fresh leaf from the plant of the present invention and subjecting the leaf to LC-MS/MS.

### 7. Method of producing the plant of the present invention

In another aspect, the present invention provides a method of producing a stevia plant comprising the glycoside of the present invention, comprising a step of crossing the stevia plant of the present invention with a second stevia plant (herein, sometimes referred to as "the production method of the present invention").

The "stevia plant comprising the glycoside of the present invention" to be produced by the method has the same phenotype and genetic properties as those of the plant of the present invention.

The range of the content of a glycoside of the present invention, etc. in the plant obtained by the production method of the present invention are as described above about the plant of the present invention.

In one aspect, the plant obtained by the production method of the present invention has at least one of genetic features (1) to (8) of the present invention. In one aspect, the plant obtained by the production method of the present invention expresses a gene encoding a protein comprising the amino acid sequence of SEQ ID NO: 97. In a preferable aspect, the plant obtained by the production method of the present invention expresses a gene encoding a protein comprising the amino acid sequence of SEQ ID NO: 97 at a higher level than the plant of wild type line. In another preferable aspect, the plant obtained by the production method of the present invention expresses a gene encoding a protein comprising the amino acid sequence of SEQ ID NO: 97 at a higher level than ubiquitin gene. The expression level is as described in "4. Stevia plant comprising novel steviol glycoside and an extract thereof".

In the production method of the present invention, "hybridizing" means that the plant of the present invention (first generation (S1)) is crossed with a second plant (S1) to obtain a progeny plant thereof (plant produced by the production method of the present invention (second generation (S2)). The hybridizing method is preferably backcross. The "backcross" is an approach of further crossing a progeny plant (S2) generated between the plant of the present invention and the second plant, with the plant of the present invention (i.e., a plant having the genetic feature(s) of the present invention) (S1) to produce a plant having the genetic feature(s) of the present invention. When the second plant (S1) for use in the production method of the present invention has the same phenotype and genetic properties as those of the plant of the present invention, the crossing is substantially backcross. The genetic polymorphism of the present invention is inheritable according to the Mendel's law. In association with this, the phenotype correlating with the genetic polymorphism, i.e., comprising the glycoside of the present invention, is also inheritable according to the Mendel's law.

Alternatively, the plant of the present invention can also be produced by selfing. The selfing can be performed by the self-pollination of the stamen pollen of the plant of the present invention with the pistil of the plant of the present invention.

Since the plant produced by the production method of the present invention has the same phenotype and genetic properties as those of the plant of the present invention, the plant produced by the production method of the present invention can be further crossed with a third stevia plant to produce a stevia plant having a phenotype equivalent to that of the plant of the present invention.

In an alternative aspect, the plant of the present invention may be produced by regenerating a plant by the culture of the tissue culture or the cultured plant cell mentioned above. The culture conditions are the same as those for culturing a tissue culture or a cultured plant cell of the wild type stevia plant and are known in the art (Protocols for In Vitro cultures and secondary metabolite analysis of aromatic and medicinal plants, Method in molecular biology, vol. 1391, pp. 113-123).

In a further alternative aspect, the plant of the present invention may be produced by introducing the variation of the present invention to the genome of a stevia plant. In a specific aspect, the production method of the plant of the present invention comprises at least one of the following steps (1) to (8):
(1) a step of introducing a variation from C to T to a position corresponding to position 298 of SEQ ID NO: 1;
(2) a step of introducing a variation from A to C to a position corresponding to position 328 of SEQ ID NO: 1;
(3) a step of introducing a variation from C to T to a position corresponding to position 360 of SEQ ID NO: 1;
(4) a step of introducing a variation from C to T to a position corresponding to position 386 of SEQ ID NO: 1;
(5) a step of introducing a variation from C to T to a position corresponding to position 393 of SEQ ID NO: 1;
(6) a step of introducing a variation from C to T to a position corresponding to position 411 of SEQ ID NO: 1;
(7) a step of introducing a variation from A to C to a position corresponding to position 427 of SEQ ID NO: 1; and
(8) a step of introducing a variation from C to T to a position corresponding to position 453 of SEQ ID NO: 1.

The introduction of the variation may be performed by a genetic modification approach or may be performed by a non-genetic modification approach. Examples of the "non-genetic modification approach" include a method of inducing a variation in the gene of a host cell (or a host plant) without transfection with a foreign gene (mutagenesis treatment). Examples of such a method include a method of allowing a mutagen to act on a plant cell. Examples of such a mutagen include ethyl methanesulfonate (EMS) and sodium azide. For example, EMS can be used at a concentration such as 0.1%, 0.2%, 0.3%, 0.4%, 0.5%, 0.6%, 0.7%, 0.8%, 0.9% or 1.0% to treat a plant cell. The treatment time is 1 to 48 hours, 2 to 36 hours, 3 to 30 hours, 4 to 28 hours, 5 to 26 hours, or 6 to 24 hours. The procedures themselves of the treatment are known in the art and can be performed by dipping a water-absorbed seed obtained through a water absorption process in a treatment solution comprising the mutagen at the concentration described above for the treatment time described above.

An alternative example of the non-genetic modification approach can be a method of irradiating a plant cell with radiation or light beam such as X ray, γ ray, or ultraviolet ray. In this case, a cell irradiated using an appropriate dose (ultraviolet lamp intensity, distance, and time) of ultraviolet ray is cultured in a selective medium or the like, and then, a cell, a callus, or a plant having the trait of interest can be selected. In this operation, the irradiation intensity is 0.01 to 100 Gr, 0.03 to 75 Gr, 0.05 to 50 Gr, 0.07 to 25 Gr, 0.09 to 20 Gr, 0.1 to 15 Gr, 0.1 to 10 Gr, 0.5 to 10 Gr, or 1 to 10 Gr. The irradiation distance is 1 cm to 200 m, 5 cm to 100 m, 7 cm to 75 m, 9 cm to 50 m, 10 cm to 30 m, 10 cm to 20 m, 10 cm to 10 m. The irradiation time is 1 minute to 2 years, 2 minutes to 1 year, 3 minutes to 0.5 years, 4 minutes to 1 month, 5 minutes to 2 weeks, or 10 minutes to 1 week. The irradiation intensity, distance and time differ depending on the type of radiation or the state of the subject to be irradiated (cell, callus, or plant) and can be appropriately adjusted by those skilled in the art.

Approaches such as cell fusion, another culture (haploid induction), and remote crossing (haploid induction) are also known in the art.

In general, plant cells may involve a mutation during culture. Therefore, it is preferred to regenerate a plant individual, for more stably maintaining the trait.

The scope of the present invention does not exclude a plant obtained by the ex-post facto genetic recombination (e.g., genome editing) with the plant of the present invention as a host (e.g., a plant further provided with another trait by genetic recombination with the plant of the present invention as a host).

### 8. Screening method and kit for plant comprising novel steviol glycoside

The plant of the present invention and a plant having the same phenotype and genetic properties as those of the plant of the present invention can be screened for by detecting genetic features of the present invention and/or expression of the gene of the present invention from a tissue of the plant. In this context, "screening" means that the plant of the present invention is discriminated from the other plants to select the plant of the present invention. Thus, the present invention, in another aspect, provides a method of screening for a plant, comprising a step of detecting the presence and/or the absence of at least one of the genetic features (1) to (8) of the present invention from the genome of a test plant and/or a step of detecting expression of the gene of the present invention (herein, also referred to as "the screening method of the present invention"). The screening method of the present invention may comprise a step of selecting a plant in which at least one of the genetic features (1) to (8) of the present invention has been detected, and/or a plant in which expression of the gene of the present invention has been detected. In a specific aspect, the screening method of the present invention comprises a step of selecting a plant in which the expression level of the gene of the present invention is larger than the expression level in a control plant (for example, wild type plant or a plant not having (a) genetic features of the present invention) and/or a plant in which the expression level of the gene of the present invention is larger than the expression level of ubiquitin.

In one aspect, the genetic feature to be detected is the genetic feature (1) of the present invention. In another aspect, the genetic feature to be detected is the genetic feature (2) of the present invention. In another aspect, the genetic feature to be detected is the genetic feature (3) of the present invention. In another aspect, the genetic feature to be detected is the genetic feature (4) of the present invention. In another aspect, the genetic feature to be detected is the genetic feature (5) of the present invention. In another aspect, the genetic feature to be detected is the genetic feature (6) of the present invention. In another aspect, the genetic feature to be detected is the genetic feature (7) of the present invention. In another aspect, the genetic feature to be detected is the genetic feature (8) of the present invention.

The genetic features (1) to (8) of the present invention can be detected by any known method. In one aspect of the present invention, the genetic feature (1) of the present invention can be detected by dCAPS method using, for example, the following primer sets and restriction enzymes.

**Table 1 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | GATGGAAAACAAA ATTTAGATAAATAA AGCCTTGAACGT (SEQ ID NO: 51) | ACCAAAACAAGTTG ACTGTTACACA (SEQ ID NO: 53) | AclI | 377 | 38, 339, 377 |
| 2 | ATCAACCTGAGCA | ATTAAAGTTGGTGA | HpyCH4V | 339 | 40, 299, 339 |
| | ACAAAGTTACTG (SEQ ID NO: 52) | AAAAAAATAAAAAT AAATTTGGCATGC (SEQ ID NO: 54) | | | |
| 3 | ATCAACCTGAGCA ACAAAGTTACTG (SEQ ID NO: 52) | ATTAAAGTTGGTGA AAAAAAATAAAAT AAATTTGGCTTAT (SEQ ID NO: 55) | PsiI | 339 | 40, 299, 339 |

No. 1 combination of a primer set and a restriction enzyme in Table 1 above will be described as an example. If a candidate plant has the genetic feature (1), bands of approximately 341 bp long (e.g., SEQ ID NO: 94) and approximately 36 bp long (e.g., SEQ ID NO: 95) are obtained by performing PCR amplification using a forward primer having the nucleotide sequence shown in SEQ ID NO: 51 and a reverse primer having the nucleotide sequence shown in SEQ ID NO: 53 on the genomic DNA of the candidate plant; and treating the obtained PCR product (approximately 377 bp long, e.g., SEQ ID NO: 93) with a restriction enzyme AclI. On the other hand, when only a band of approximately 377 bp long (e.g., SEQ ID NO: 96) is obtained even by treating the PCR product of approximately 377 bp long obtained by PCR amplification with a restriction enzyme AclI, the candidate plant does not have the genetic feature (1). Similarly, the genetic feature (1) of the present invention can be detected by using No. 2 or No. 3 combination of a primer set and a restriction enzyme in Table 1 above.

In one aspect of the present invention, the genetic feature (2) of the present invention can be detected by dCAPS method using, for example, the following primer sets and restriction enzymes. The detailed detection method is the same as described in the genetic feature (1).

**Table 2 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | CCTTGGACTTCTTA TGCCAAATTTATTT TTATTTTGTATC (SEQ ID NO: 56) | ACCAAAACAAGTT GACTGTTACACA (SEQ ID NO: 53) | BciVI | 348 | 47, 301, 348 |
| 2 | CCTTGGACTTCTTA TGCCAAATTTATTT TTATTTTTTGGC (SEQ ID NO: 57) | ACCAAAACAAGTT GACTGTTACACA (SEQ ID NO: 53) | HaeIII | 348 | 39 309, 348 |
| 3 | CCTTGGACTTCTTA TGCCAAATTTATTT TTATTTTGGTTC | ACCAAAACAAGTT GACTGTTACACA (SEQ ID NO: 53) | NlaIV | 348 | 38, 310, 348 |
| | (SEQ ID NO: 58) | | | | |
| 4 | CCTTGGACTTCTTA TGCCAAATTTATTT TTATTTTTTGTA (SEQ ID NO: 59) | ACCAAAACAAGTT GACTGTTACACA (SEQ ID NO: 53) | RsaI | 348 | 39, 309, 348 |
| 5 | ATCAACCTGAGCA ACAAAGTTACTG (SEQ ID NO: 52) | ACAAACAAGAATG TTTATGGTCAATAT TAATTAAACTCGG (SEQ ID NO: 60) | Aval | 368 | 36, 332, 368 |
| 6 | ATCAACCTGAGCA ACAAAGTTACTG (SEQ ID NO: 52) | ACAAACAAGAATG TTTATGGTCAATAT TAATTAAAGTTCC (SEQ ID NO: 61) | BsaWI/ BspEI | 368 | 38, 330, 348 |
| 7 | ATCAACCTGAGCA ACAAAGTTACTG (SEQ ID NO: 52) | ACAAACAAGAATG TTTATGGTCAATAT TAATTAAAGCCGG (SEQ ID NO: 62) | NciI/ ScrFI | 368 | 38, 330, 348 |
| 8 | ATCAACCTGAGCA ACAAAGTTACTG (SEQ ID NO: 52) | ACAAACAAGAATG TTTATGGTCAATAT TAATTAAAGTCGC (SEQ ID NO: 63) | BstUI | 368 | 39, 329, 348 |
| 9 | ATCAACCTGAGCA ACAAAGTTACTG (SEQ ID NO: 52) | ACAAACAAGAATG TTTATGGTCAATAT TAATTAAAGTCCG (SEQ ID NO: 64) | HpaII | 368 | 38, 330, 348 |
| 10 | ATCAACCTGAGCA ACAAAGTTACTG (SEQ ID NO: 52) | ACAAACAAGAATG TTTATGGTCAATAT TAATTAAACTTGA (SEQ ID NO: 65) | SmlI | 368 | 36, 332, 348 |

In one aspect of the present invention, the genetic feature (3) of the present invention can be detected by dCAPS method using, for example, the following primer sets and restriction enzymes. The detailed detection method is the same as described in the genetic feature (1).

**Table 3 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | TTTTTTTCACCAAC TTTAATTAATATTG ACCATAAACATG (SEQ ID NO: 66) | ACCAAAACAAGTTG ACTGTTACACA (SEQ ID NO: 53) | AflIII/ PciI | 316 | 36, 280, 316 |
| 2 | TTTTTTTCACCAAC TTTAATTAATATTG ACCATAAAGATC (SEQ ID NO: 67) | ACCAAAACAAGTTG ACTGTTACACA (SEQ ID NO: 53) | BglII | 316 | 36, 280, 316 |
| 3 | TTTTTTTCACCAAC TTTAATTAATATTG ACCATAAACACG (SEQ ID NO: 68) | ACCAAAACAAGTTG ACTGTTACACA (SEQ ID NO: 53) | HpyCH4I V | 316 | 38, 278, 316 |
| 4 | ATCAACCTGAGCA ACAAAGTTACTG | AATGAAAGAAAAAA GAATTAAAGTTGAG | Hpy188I | 400 | 39, 361, 400 |
| | (SEQ ID NO: 52) | TCATACAATCAG (SEQ ID NO: 69) | | | |

In one aspect of the present invention, the genetic feature (4) of the present invention can be detected by dCAPS method using, for example, the following primer sets and restriction enzymes. The detailed detection method is the same as described in the genetic feature (1).

**Table 4 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | TGACCATAAACATT CTTGTTTGTATGAC TCAACTTTAACG (SEQ ID NO: 70) | ACCAAAACAAGTT GACTGTTACACA (SEQ ID NO: 53) | AclI/ HpyCH4IV | 290 | 36, 254, 290 |

In one aspect of the present invention, the genetic feature (5) of the present invention can be detected by dCAPS method using, for example, the following primer sets and restriction enzymes. The detailed detection method is the same as described in the genetic feature (1).

**Table 5 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | ATTGAATATCCTCG CAATCCTCACC (SEQ ID NO: 71) | AAATAGTGAGTCC TGTTTAAAAGATGT TATCAAAATGATC (SEQ ID NO: 72) | BclI | 367 | 36, 331, 367 |
| 2 | ATTGAATATCCTCG CAATCCTCACC (SEQ ID NO: 71) | AAATAGTGAGTCC TGTTTAAAAGATGT TATCAAAATGTAC (SEQ ID NO: 73) | BsrGI | 367 | 36, 331, 367 |
| 3 | ATTGAATATCCTCG CAATCCTCACC (SEQ ID NO: 71) | AAATAGTGAGTCC TGTTTAAAAGATGT TATCAAAATTATA (SEQ ID NO: 74) | PsiI | 367 | 37, 330, 367 |

In one aspect of the present invention, the genetic feature (6) of the present invention can be detected by dCAPS method using, for example, the following primer sets and restriction enzymes. The detailed detection method is the same as described in the genetic feature (1).

**Table 6 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | GACTCAACTTTAAT TCTTTTTTCTTTCA TTTTGATAACGT (SEQ ID NO: 75) | ACCAAAACAAGTT GACTGTTACACA (SEQ ID NO: 53) | AclI | 265 | 37, 228, 265 |
| 2 | GACTCAACTTTAAT TCTTTTTTCTTTCA TTTTGATAACAG (SEQ ID NO: 76) | ACCAAAACAAGTT GACTGTTACACA (SEQ ID NO: 53) | HpyCH4III | 265 | 39, 226, 265 |
| 3 | ATTGAATATCCTCG CAATCCTCACC (SEQ ID NO: 71) | AAATGGAACATAA TGTTCAAATAGTG AGTCCTGTTTTATA (SEQ ID NO: 77) | PsiI | 385 | 38, 347, 385 |

In one aspect of the present invention, the genetic feature (7) of the present invention can be detected by dCAPS method using, for example, the following primer sets and restriction enzymes. The detailed detection method is the same as described in the genetic feature (1).

**Table 7 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | TTTTTTCTTTCATT TTGATAACATCTTT TAAACAGGACGT (SEQ ID NO: 78) | ACCAAAACAAGTT GACTGTTACACA (SEQ ID NO: 53) | AatII/ NarI | 249 | 40, 209, 249 |
| 2 | TTTTTTCTTTCATT TTGATAACATCTTT TAAACAGGGATC (SEQ ID NO: 79) | ACCAAAACAAGTT GACTGTTACACA (SEQ ID NO: 53) | BamHI/ BstYI | 249 | 36, 215, 249 |
| 3 | TTTTTTCTTTCATT TTGATAACATCTTT TAAACAGGTATC (SEQ ID NO: 80) | ACCAAAACAAGTT GACTGTTACACA (SEQ ID NO: 53) | BciVI | 249 | 47, 202, 249 |
| 4 | TTTTTTCTTTCATT TTGATAACATCTTT TAAACAGGAATG (SEQ ID NO: 81) | ACCAAAACAAGTT GACTGTTACACA (SEQ ID NO: 53) | B sm I | 249 | 41, 208, 249 |
| 5 | TTTTTTCTTTCATT TTGATAACATCTTT TAAACAGGAATT (SEQ ID NO: 82) | ACCAAAACAAGTT GACTGTTACACA (SEQ ID NO: 53) | EcoRI | 249 | 36, 215, 249 |
| 6 | TTTTTTCTTTCATT TTGATAACATCTTT TAAACAGGGCGC (SEQ ID NO: 83) | ACCAAAACAAGTT GACTGTTACACA (SEQ ID NO: 53) | HaeII | 249 | 41, 208, 249 |
| 7 | TTTTTTCTTTCATT TTGATAACATCTTT | ACCAAAACAAGTT GACTGTTACACA | BanII | 249 | 40, 209, 249 |
| | TAAACAGGAGCC (SEQ ID NO: 84) | (SEQ ID NO: 53) | | | |
| 8 | TTTTTTCTTTCATT TTGATAACATCTTT TAAACAGGGGTC (SEQ ID NO: 85) | ACCAAAACAAGTT GACTGTTACACA (SEQ ID NO: 53) | NlaIV | 249 | 39, 210, 249 |
| 9 | CTACTCTCTACGTC CCCAAAATACC (SEQ ID NO: 86) | ATTTTTTTTTAAAA GAAAATGGAACAT AATGTTCAACTCG (SEQ ID NO: 87) | AvaI | 281 | 37, 244, 281 |
| 10 | CTACTCTCTACGTC CCCAAAATACC (SEQ ID NO: 86) | ATTTTTTTTTAAAA GAAAATGGAACAT AATGTTCAAATCC (SEQ ID NO: 88) | BspEI | 281 | 38, 243, 281 |
| 11 | CTACTCTCTACGTC CCCAAAATACC (SEQ ID NO: 86) | ATTTTTTTTTAAAA GAAAATGGAACAT AATGTTCAACCAG (SEQ ID NO: 89) | PspGI | 281 | 36, 245, 281 |
| 12 | CTACTCTCTACGTC CCCAAAATACC (SEQ ID NO: 86) | ATTTTTTTTTAAAA GAAAATGGAACAT AATGTTCACATAT (SEQ ID NO: 90) | NdeI | 281 | 37, 244, 281 |
| 13 | TTTTTTCTTTCATT TTGATAACATCTTT TAAACAGGACGT (SEQ ID NO: 78) | ACCAAAACAAGTT GACTGTTACACA (SEQ ID NO: 53) | AatII/ NarI | 249 | 40, 209, 249 |
| 14 | TTTTTTCTTTCATT TTGATAACATCTTT TAAACAGGGATC (SEQ ID NO: 79) | ACCAAAACAAGTT GACTGTTACACA (SEQ ID NO: 53) | BamHI/ BstYI | 249 | 36,215, 249 |

In one aspect of the present invention, the genetic feature (8) of the present invention can be detected by dCAPS method using, for example, the following primer sets and restriction enzymes. The detailed detection method is the same as described in the genetic feature (1).

**Table 8 Combinations of primer set and restriction enzyme**

| No. | Sequence of forward primer | Sequence of reverse primer | Restriction enzyme | Band size (bp) after restriction enzyme treatment | |
|---|---|---|---|---|---|
| | | | | Genetic feature is absent | Genetic feature is present |
| 1 | CTACTCTCTACGTC CCCAAAATACC (SEQ ID NO: 86) | TGTTAATCAGTTAT TATTTCCACATTAT TTTTTTTTCAAG (SEQ ID NO: 91) | Hpy188III | 307 | 37, 270, 307 |
| 2 | CTACTCTCTACGTC CCCAAAATACC (SEQ ID NO: 86) | TGTTAATCAGTTAT TATTTCCACATTAT TTTTTTTTTATA (SEQ ID NO: 92) | PsiI | 307 | 38, 269, 307 |

Expression of a gene encoding a protein having the amino acid sequence of SEQ ID NO: 97 in a test stevia plant can be detected by any known genetic expression detection method. Examples of the method include, but are not limited to, various hybridization methods using a nucleic acid specifically hybridizing to a nucleic acid encoding the gene or a unique fragment thereof or a transcript of the nucleic acid (for example, mRNA) or a spliced product, northern blotting, southern blotting, various PCR methods, immunoprecipitation using an antibody to a protein having the amino acid sequence of SEQ ID NO: 97, EIA, ELISA, IRA, IRMA, western blotting method, immunohistochemistry, immunocytochemical technique, and flow cytometry. Of these detection methods, a method which can determine an expression level is preferable.

In a specific aspect, expression of the gene can be detected and/or quantified by, e.g., PCR method using primers comprising the following nucleotide sequences. Note that, non-limiting examples of the detection method using these primers are described in Examples.
Forward: CCTGTTCATTACAAATTCAACCCG (SEQ ID NO: 99)
Reverse: AACCCTAACATGTTCAATGTCCCTA (SEQ ID NO: 100)

In a preferable aspect, the screening method of the present invention comprises measuring the expression level of the gene of the present invention in a test stevia plant. The expression level obtained may be compared to the expression level of the gene of the present invention in a control stevia plant or the expression level of ubiquitin in the same test stevia plant. Examples of the control stevia plant include an individual of a wild type line and an individual having no genetic features of the present invention. A larger expression level of the gene of the present invention in a test stevia plant than the expression level in a control stevia plant, and/or the larger expression level of the gene of the present invention in a test stevia plant than the expression level of ubiquitin in the same individual are used as indexes to show that the test stevia plant is the plant of the present invention.

The screening method of the present invention may further comprise a step of evaluating the content of glycoside of the present invention in a test stevia plant in which the genetic feature(s) of the present invention and/or the gene of the present invention has (have) been detected. The content of glycoside of the present invention is evaluated as described in the section relating to the plant of the present invention. In the aspect, the screening method of the present invention may be applied to a daughter plant obtained by selecting individuals having a high glycoside content of the present invention from test stevia plants in which the genetic feature(s) of the present invention has/have been detected, and crossing the selected individuals with another stevia plant. Thus, the screening method of the present invention may comprise one or more of the following steps.
(i) detecting the genetic feature(s) of the present invention (e.g., at least one of the genetic features (1) to (8) of the present invention) and/or expression of the gene of the present invention from test stevia plants,
(ii) evaluating the content of the glycoside of the present invention in test stevia plants in which genetic feature(s) of the present invention and/or expression of the gene of the present invention has been detected,
(iii) selecting an individual having a high content of glycoside of the present invention from test stevia plants in which genetic feature(s) of the present invention and/or expression of the gene of the present invention are detected,
(iv) crossing the individual having a high content of glycoside of the present invention selected with another stevia plant,
(v) detecting genetic feature(s) of the present invention (for example, at least one of the genetic features (1) to (8) of the present invention) and/or expression of the gene of the present invention from the genome of daughter plants obtained by crossing,
(vi) evaluating the content of glycoside of the present invention in the daughter plants in which genetic feature(s) of the present invention and/or expression of the gene of the present invention has been detected,
(vii) selecting an individual having a high content of glycoside of the present invention from the daughter plants in which genetic feature(s) of the present invention has/have been detected.

In the screening method of the present invention, the test stevia plant may be a non-genetically modified plant. Non-genetically modified plants are as described in the section relating to the plant of the present invention. In the screening method of the present invention, the test stevia plant may include a stevia plant subjected to a mutagenesis treatment and a progeny plant thereof. The mutagenesis treatment is as described in the section relating to the plant of the present invention, and includes treatment with a mutagen, treatment with radiation or irradiation with light, and the like.

The present invention also provides the above-mentioned primer set, e.g., the primer set described in Tables 1 to 8 above. The present invention further provides a primer set that can amplify the region having a nucleotide sequence selected from the group consisting of SEQ ID NOs: 1 and 98 by PCR, for example, a primer set of a forward primer comprising the nucleotide sequence of SEQ ID NO: 52 and a reverse primer comprising the nucleotide sequence of SEQ ID NO: 53, and a primer set of a forward primer comprising the nucleotide sequence of SEQ ID NO: 99 and a reverse primer comprising the nucleotide sequence of SEQ ID NO: 100.

In addition, the present invention provides a probe capable of detecting the presence and/or absence of the genetic features of the present invention, which may be referred to as the "probe of the present invention" hereinafter. The probe of the present invention may have a structure suitable for various detection methods for the presence and/or absence of the genetic feature(s) of the present invention. For example, the probe of the present invention may comprise a nucleotide sequence complementary to a portion of a genome comprising a variation site of the present invention. Non-limiting examples of the probe include probes specifically hybridizing to the nucleotide sequence selected from SEQ ID NOs: 3 to 50. Of these sequences, SEQ ID NOs: 4, 6, 8, 10, 12, 14, 16, 18, 20, 22, 24, 26, 28, 30, 32, 34, 36, 38, 40, 42, 46, 48 and 50 are specific for alleles comprising the variation of the present invention, and SEQ ID NOs: 3, 5, 7, 9, 11, 13, 15, 17, 19, 21, 23, 25, 27, 29, 31, 33, 35, 37, 39, 41, 43, 45, 47 and 49 are specific for alleles not having the variation of the present invention. In a preferable aspect, a probe that can detect the presence of genetic feature(s) of the present invention has hybridization conditions: it hybridizes to a nucleotide sequence selected from SEQ ID NOs: 4, 6, 8, 10, 12, 14, 16, 18, 20, 22, 24, 26, 28, 30, 32, 34, 36, 38, 40, 42, 46, 48 and 50 but does not hybridize to a nucleotide sequence selected from SEQ ID NOs: 3, 5, 7, 9, 11, 13, 15, 17, 19, 21, 23, 25, 27, 29, 31, 33, 35, 37, 39, 41, 43, 45, 47 and 49. In a preferable aspect, a probe that can detect the absence of genetic feature(s) of the present invention has hybridization conditions: it hybridizes to a nucleotide sequence selected from SEQ ID NOs: 3, 5, 7, 9, 11, 13, 15, 17, 19, 21, 23, 25, 27, 29, 31, 33, 35, 37, 39, 41, 43, 45, 47 and 49 but does not hybridize to a nucleotide sequence selected from SEQ ID NOs: 4, 6, 8, 10, 12, 14, 16, 18, 20, 22, 24, 26, 28, 30, 32, 34, 36, 38, 40, 42, 46, 48 and 50.

The presence of the genetic feature(s) of the present invention may be detected by detection of an allele comprising the variation(s) of the present invention and/or by non-detection of an allele not comprising the variation(s) of the present invention, and the absence of the genetic feature(s) of the invention by non-detection of an allele comprising the variation(s) of the present invention and/or by detection of an allele not comprising the variation(s) of the present invention. The probes of the present invention preferably have a label. Non-limiting examples of such labels include fluorescent labels, luminescent labels, radioactive labels, dyes, enzymes, quenchers, binding moieties with detectable labels, and the like. In a specific aspect, the probe of the present invention has a nucleotide sequence which specifically hybridize to the nucleotide sequence selected from SEQ ID NOs: 3 to 50 and a label.

The present invention further provides a kit, for example, a kit for screening, comprising a primer set described in Tables 1 to 8, and a restriction enzyme appropriate therefor.

The present invention also provides a screening kit comprising a primer set capable of amplifying by PCR a region having a nucleotide sequence selected from the group consisting of SEQ ID NO: 1, and a probe of the present invention.

These primer sets, probes and kits can be used to detect the genetic feature(s) of the present invention, used in the screening methods of the present invention, and the like. These primer sets and kits may also comprise an instruction including an explanation on the detection of genetic feature(s) of the present invention and on the screening method of the present invention, e.g., a written instruction, and media, e.g., a flexible disk, a CD, a DVD, a Blu-ray disk, a memory card, a USB memory, etc., having recorded thereon information regarding the method of use.

### 9. Nucleotide sequence related to plant of present invention

In another aspect, the present invention provides a nucleotide sequence related to the stevia plant of the present invention.

The nucleotide sequence related to a stevia plant having the genetic feature (1) comprises or consists of a nucleotide sequence selected from SEQ ID NOs: 2, 4, 6 and 8. The nucleotide sequence related to a stevia plant having the genetic feature (2) comprises or consists of a nucleotide sequence selected from SEQ ID NOs: 2, 10, 12 and 14. The nucleotide sequence related to a stevia plant having the genetic feature (3) comprises or consists of a nucleotide sequence selected from SEQ ID NOs: 2, 16, 18 and 20. The nucleotide sequence related to a stevia plant having the genetic feature (4) comprises or consists of a nucleotide sequence selected from SEQ ID NOs: 2, 22, 24 and 26. The nucleotide sequence related to a stevia plant having the genetic feature (5) comprises or consists of a nucleotide sequence selected from SEQ ID NOs: 2, 28, 30 and 32. The nucleotide sequence related to a stevia plant having the genetic feature (6) comprises or consists of a nucleotide sequence selected from SEQ ID NOs: 2, 34, 36 and 38. The nucleotide sequence related to a stevia plant having the genetic feature (7) comprises or consists of a nucleotide sequence selected from SEQ ID NOs: 2, 40, 42 and 44. The nucleotide sequence related to a stevia plant having the genetic feature (8) comprises or consists of a nucleotide sequence selected from SEQ ID NOs: 2, 46, 48 and 50.

### 10. Protein of the present invention and polynucleotide encoding the protein

In another aspect, the present invention provides a protein comprising the amino acid sequence of SEQ ID NO: 97 and a polynucleotide encoding the protein (for example, a polynucleotide comprising the nucleotide sequence of SEQ ID NO: 98). The polynucleotide is highly expressed in the plant of the present invention; on the other hands, the polynucleotide is rarely expressed in a plant having a low content of glycoside of the present invention. From this, the protein is considered to be involved in synthesis of glycoside of the present invention. Thus, if the protein is highly expressed in a stevia plant, it is expected that the content of glycoside of the present invention in the stevia plant increases, and/or, synthesis of the glycoside of the present invention is promoted.

### The present invention also provides

(a) a protein comprising an amino acid sequence obtained by deletion, substitution, insertion, and/or addition of 1 to 30 amino acids in the amino acid sequence of SEQ ID NO: 97, and having an activity to promote synthesis of the glycoside of the present invention; and
(b) a protein comprising an amino acid sequence having a sequence identity of 90% or more to the amino acid sequence of SEQ ID NO: 97 and having an activity to promote synthesis of the glycoside of the present invention (herein, a protein comprising the amino acid sequence of SEQ ID NO: 97, the above proteins of (a) and (b) sometimes generically referred to as "the protein of the present invention").

The present invention further provides
(a) a polynucleotide encoding a protein comprising an amino acid sequence obtained by deletion, substitution, insertion, and/or addition of 1 to 30 amino acids in the amino acid sequence of SEQ ID NO: 97, and having an activity to promote synthesis of the glycoside of the present invention;
(b) a polynucleotide encoding a protein comprising an amino acid sequence having a sequence identity of 90% or more to the amino acid sequence of SEQ ID NO: 97 and having an activity to promote synthesis of the glycoside of the present invention; and
(c) a polynucleotide hybridizing to a polynucleotide consisting of a nucleotide sequence complementary to a nucleotide sequence of SEQ ID NO: 98 in highly stringent conditions and encoding a protein having an activity to promote synthesis of the glycoside of the present invention (herein, a polynucleotide encoding a protein comprising the amino acid sequence of SEQ ID NO: 97 and the above polynucleotides (a) to (c) are sometimes generically referred to as "the polynucleotide of the present invention").

Examples of the "amino acid sequence obtained by deletion, substitution, insertion, and/or addition of 1 to 30 amino acids in the amino acid sequence of SEQ ID NO: 97" include an amino acid sequence obtained by deletion, substitution, insertion, and/or addition of, for example, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9 (1 to several), 1 to 8, 1 to 7, 1 to 6, 1 to 5, 1 to 4, 1 to 3, 1 to 2, or 1 amino acid residue(s) in the amino acid sequence of SEQ ID NO: 97. The number of amino acid residues to be deleted, substituted, inserted and/or added is generally and preferably small.

Examples of the "amino acid sequence having a sequence identity of 90% or more to the amino acid sequence of SEQ ID NO: 97" include an amino acid sequence having a sequence identity of 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more to the amino acid sequence of SEQ ID NO: 97. The sequence identity value is generally and preferably large. In one aspect, the "amino acid sequence having a sequence identity of 90% or more to the amino acid sequence of SEQ ID NO: 97" is different in at least one amino acid from the amino acid sequence of SEQ ID NO: 97.

As used herein, the "polynucleotide that hybridizes under highly stringent conditions" refers to a polynucleotide obtained by performing colony hybridization, plaque hybridization, Southern hybridization, or the like, using for example, all or a part of a polynucleotide consisting of a nucleotide sequence complementary to the nucleotide sequence set forth in SEQ ID NO: 98 or a polynucleotide consisting of a nucleotide sequence encoding the amino acid sequence set forth in SEQ ID NO: 97 as a probe. Examples of available methods of hybridization include methods described in "Sambrook & Russell, Molecular Cloning A Laboratory Manual Vol. 3, 2001 Cold Spring Harbor, Laboratory Press", "Ausubel, Current Protocols in Molecular Biology, John Wiley & Sons 1987-1997", and the like.

Examples of the "highly stringent conditions" as used herein are, but not limited to, conditions of (1) 5 x SSC, 5 x den Hald solution, 0.5% SDS, 50% formamide, 50°C; (2) 0.2 x SSC, 0.1% SDS, 60°C, (3) 0.2 x SSC, 0.1% SDS, 62°C; (4) 0.2 x SSC, 0.1% SDS, 65°C; or (5) 0.1 x SSC, 0.1% SDS, 65°C. Under these conditions, it can be expected that DNA having a high sequence identity is obtained more efficiently at higher temperatures. Meanwhile, it is considered that there are plural factors that have effects on the stringency of hybridization, such as temperature, the probe concentration, the probe length, the ionic strength, time, and the salt concentration and a person skilled in the art can attain similar stringency by selecting these factors as appropriate.

When using a commercially available kit for hybridization, for example, Alkphos Direct Labelling and Detection System (GE Healthcare) may be used. In this case, hybridized DNA can be detected after incubating a membrane with a labelled probe overnight in accordance with the protocol attached to the kit and then washing the membrane with a primary washing buffer comprising 0.1% (w/v) SDS under conditions at 55 to 60°C. Alternatively, hybridization can be detected by using the DIG nucleic acid detection kit (Roche Diagnostics), when the probe is labeled with digoxigenin (DIG) by using a commercially available reagent (for example, PCR labeling mixture (Roche Diagnostics)), in the production of a probe based on a nucleotide sequence complementary to the nucleotide sequence set forth in SEQ ID NO: 1 or a sequence complementary to all or a part of a nucleotide sequence encoding the amino acid sequence set forth in SEQ ID NO: 2.

Examples of hybridizable polynucleotide other than those described above include DNA having a sequence identity of 80% or more, 81 % or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more to the DNA of SEQ ID NO: 98 or DNA encoding the amino acid sequence set forth in SEQ ID NO: 97 as calculated by homology search software such as FASTA or BLAST using default parameters.

The sequence identity of an amino acid sequence or a nucleotide sequence can be determined using FASTA (Science 227 (4693): 1435-1441 (1985)) or the algorithm by Karlin and Altschul BLAST (Basic Local Alignment Search Tool) (Proc. Natl. Acad. Sci. USA 872264-2268, 1990; Proc Natl Acad Sci USA 90: 5873, 1993). The programs called blastn, blastx, blastp, tblastn, and tblastx based on the algorithm of BLAST have been developed (Altschul SF, et al: J Mol Biol 215:403, 1990). When analyzing a nucleotide sequence using blastn, parameters are set at, for example, score = 100, wordlength = 12. Moreover, when analyzing an amino acid sequence using blastp, parameters are, for example, score = 50, wordlength = 3. When using BLAST and Gapped BLAST programs, the default parameters of each program are used.

Whether or not a protein has an activity to promote synthesis of the glycoside of the present invention can be evaluated by expressing the polynucleotide of, e.g., (a) to (c) mentioned above in cells, preferably in stevia plant cells, and measuring the content of glycoside of the present invention. In expression of a polynucleotide, for example, expression thereof in plant cells, various known methods, such as agrobacterium method, a gene gun method, a PEG method, an electroporation method and a particle gun method, can be used without limit.

The present invention also provides a nucleic acid construct, vector and host cell comprising the polynucleotide of the present invention (herein, sometimes referred to as "the nucleic acid construct of the present invention", "the vector of the present invention" and "the host cell of the present invention"). The nucleic acid construct and vector of the present invention may comprise a heterogeneous nucleotide sequence, for example, an expression control sequence such as a heterogeneous promoter, enhancer and terminator. As the nucleic acid construct and vector of the present invention, those suitable for a host cell in which the polynucleotide of the present invention is to be expressed can be selected. Examples of the host cell include, but are not limited to, microorganism cells and plant cells. Non-limiting examples of the microorganism include *Escherichia coli* and yeast. The host cell may be a heterogeneous cell (cells derived from other plants except stevia) or a homogeneous cell (cells derived from stevia). Also, the host cell of the present invention may comprise the nucleic acid construct or vector of the present invention or may be transformed with the vector of the present invention.

The present invention further provides a method of producing the protein of the present invention, comprising expressing the polynucleotide of the present invention in the host cell of the present invention. The production method may further comprise purifying the protein of the present invention.

As to other general molecular biological processes, see "Sambrook and Russell, Molecular Cloning: A Laboratory Manual Vol. 3, Cold Spring Harbor Laboratory Press 2001", "Methods in Yeast Genetics, A laboratory manual (Cold Spring Harbor Laboratory Press, Cold Spring Harbor, NY)" and the like.

### Examples

Hereinafter, an example of the present invention will be described in detail, although the content of the present invention should not be limited thereto.

### [Isolation of novel steviol glycoside]

Two lines of novel stevia plants ((Cultivar A (EM3-4) and Cultivar B (EM2-27-15)) developed at Suntory Global Innovation Center (SIC) were prepared. These cultivars were obtained as follows. First, commercially available stevia seeds were sown and raised to subject the resulting seeds to genetic modification by a treatment with 0.2% (Cultivar B) or 0.3% (Cultivar A) ethyl methanesulfonate (EMS). The EMS-treated seeds were sown in the greenhouse at the Suntory World Research Center to obtain the original EMS-treated seedling (M0 generation). No difference in the germination rate was observed between the treatment concentrations. Cultivar A was derived from an individual of this M0 generation. Furthermore, the first treatment generation (M1 generation) seeds obtained by self-fertilization of all individuals of the M0 generation were collected and sown in the greenhouse at the Suntory World Research Center to obtain M1 generation seedlings. Cultivar B was derived from an individual of this M1 generation.

Extracts obtained from the leaf of Cultivars A and B were subj ected to high performance liquid chromatography (HPLC) separation-mass spectrometry (MS) to perform the screening analysis of the steviol glycosides contained in the stevia plants based on the molecular weights of steviol glycosides formed of sugar chains including D-glucopyranosyl (Glc) and/or xylopyranosyl (Xyl). In this regard, Cultivars A and B were plants having the genetic features 1-8.

A process for preparing a test liquid was as follows: 10.0 mg each of lyophilized dried stevia leaves was weighed into a glass vial, to which 1.0 mL of water/methanol (1/1 vol/vol) was added as an extracting solvent, and then the resultant was subjected to ultrasonic irradiation in an ultrasonic cleaner (AS ONE, AS52GTU) at a set temperature of 25°C for 20 minutes, thereby obtaining liquid extracts of steviol glycosides from the stevia leaves. The resultant was further 10-fold diluted with water/methanol and filtrated through a filter with a pore size of 0.45 µm (Nacalai tesque, Cosmonice filter S (solvent)) so as to be subjected to HPLC-MS.

For the HPLC part of HPLC-MS, Nexera LC-30AD (Shimadzu Corporation) was used as a liquid delivery unit LC pump, and SM-C18 (4.6 x 250 mm) (from Imtakt) was used as a separation column. Liquid delivery of the LC mobile phase was carried out by using 0.2% acetic acid-containing Milli-Q water as mobile phase A and methanol as mobile phase B, where the binary gradient as follows: the concentration of the mobile phase B was constantly maintained at 10% for 0-5 minutes, allowed to shift from 10% to 70% in the next 15 minutes, then allowed to shift from 70% to 100% in the following 5 minutes, and maintained at 100% for 5 minutes at the end. The flow rate of the mobile phase was 0.4 mL/min, and 5 µL of the stevia leaf liquid extract that had been diluted and filtrated with a filter was injected.

For the MS part, triple quadrupole mass spectrometer LCMS-8030 (Shimadzu Corporation) equipped with an electrospray ionization (ESI) ion source was used. The mass spectrometry measurement was carried out in a selected ion monitoring (SIM) mode by selecting the negative ion measurement mode and the m/z values. The m/z values were selected by a calculation based on the molecular weights of steviol glycosides formed of sugar chains including D-glucopyranosyl (Glc) and/or xylopyranosyl (Xyl). Accordingly, m/z = 965.3 (Glc (4)), 1097.4 (Glc (4), Xyl (1)), 1127.4 (Glc (5)), 1259.5 (Glc (5), Xyl (1)), and 1289.5 (Glc (6)) were selected. Furthermore, a high purity reagent and rebaudiosides A, D and M that were available were also measured under the same conditions so as to confirm the negative ion m/z values and the retention time in HPLC. The peak areas (arbitrary unit) of the mainly detected steviol glycosides are shown in Table 9.

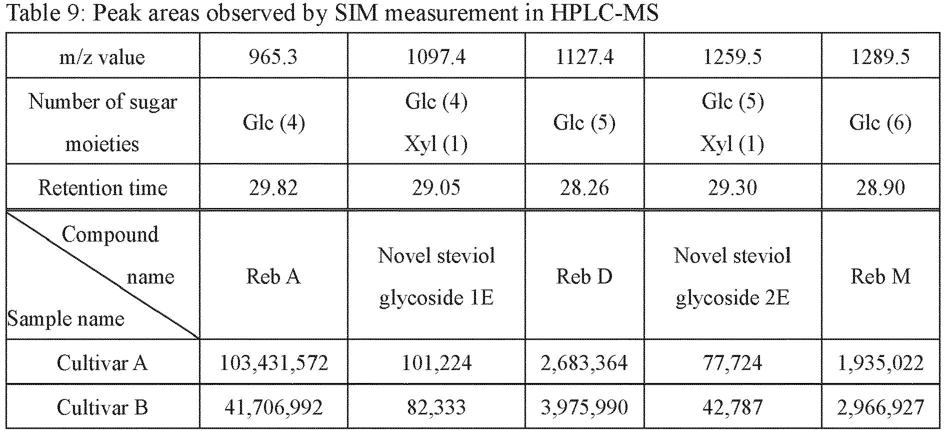

Figure 2 shows a selected ion chromatogram of Cultivar A at m/z of 1097.4. A peak of a molecular weight that had never been reported was observed in the selected ion chromatogram of a steviol glycoside (m/z 1097.4) in which the modified sugar chain contained four glucose moieties (Glc) and one xylose moiety (Xyl). Specifically, the peak at Rt 29.05 minutes shown in Figure 2 was an unknown substance. This substance was tentatively called "Novel steviol glycoside IE". A similar peak was also detected for Cultivar B.

Figure 3 shows a selected ion chromatogram of Cultivar A at m/z of 1259.5. A peak of a molecular weight that had never been reported was observed in the selected ion chromatogram of a steviol glycoside (m/z 1259.5) in which the modified sugar chain contained five glucose moieties (Glc) and one xylose moiety (Xyl). Specifically, the peak at Rt 29.30 minutes shown in Figure 3 was an unknown substance. This substance was tentatively called "Novel steviol glycoside 2E". A similar peak was also detected for Cultivar B.

### [Structural analysis of novel steviol glycosides]

According to the present invention, structural analyses of Novel steviol glycosides 1E and 2E detected from the cultivars were performed in the following procedure.
(i) Structural deduction by a fragmentation analysis through high-performance liquid chromatography (HPLC)-high resolution mass spectrometry (MS), MS/MS, and three-stage ion fragmentation (MS³-fragmentation).
(ii) Chemical synthesis of the deduced steviol glycoside standard products via chemical reaction.
(iii) Structural determination by matching with the retention time and the fragmented pattern of the chemically synthesized standard product in HPLC-high resolution MS and MS³-fragmentation.

Hereinafter, each of Steps (i)-(iii) above will be described in detail.

### (i) Structural deduction by a fragmentation analysis through high performance liquid chromatography (HPLC)-high resolution mass spectrometry (MS), MS/MS, and three-stage ion fragmentation (MS³-fragmentation)

Test liquids were prepared as follows: 10.0 mg each of lyophilized dried stevia leaves was weighed into a glass vial, to which 1.0 mL of water/methanol (1/1 vol/vol) was added as an extracting solvent, and then the resultant was subjected to ultrasonic irradiation in an ultrasonic cleaner (AS ONE, AS52GTU) at a set temperature of 25°C for 20 minutes, thereby obtaining liquid extracts of steviol glycosides from the stevia leaves. The resultant was further 10-fold diluted with water/methanol and filtrated through a filter with a pore size of 0.45 µm (Nacalai tesque, Cosmonice filter S (solvent system)) so as to be subjected to HPLC-MS.

In an equipment configuration for high performance liquid chromatography-electrospray ionization-high resolution mass spectrometry (HPLC-ESI-HRMS), the equipment for HPLC was configured by using Prominence LC-20AD (Shimadzu Corporation) as a liquid delivery unit LC pump and SM-C18 (4.6 x 250 mm) (from Imtakt) as a separation column. The LC mobile phase was delivered using 0.2% acetic acid-containing Milli-Q water as mobile phase A and methanol as mobile phase B, where the binary gradient was as follows: the concentration of the mobile phase B was constantly maintained at 10% for 0-5 minutes, then allowed to shift from 10% to 70% in the next 15 minutes, and further allowed to shift from 70% to 100% in the following 5 minutes. At the end, the concentration of the mobile phase B was maintained at 100% for 5 minutes. The flow rate of the mobile phase was 0.4 mL/min, and 20 µL of the stevia leaf liquid extract that had been diluted and subsequently filtrated with a filter was injected. For the mass spectrometry part, Orbitrap Elite MS (from Thermo Fisher Scientific) equipped with an ESI ion source was used. The mass spectrometry measurement was carried out in a negative ion measurement mode at m/z in a range of 150-2000 with resolution set to 60,000. The MS/MS measurement was carried out by selecting the targeted m/z of 1095.4 or 1257.5 and in a CID mode where fragmentation was induced by collision with an inert gas. The ion with the highest intensity in the MS/MS spectrum was targeted for MS³. Irradiation of energy required for fragmentation was performed at the standard collision energy unique to the apparatus, i.e., 35.

In order to study the fragmented patterns of Novel steviol glycosides IE and 2E, standard samples, i.e., rebaudiosides A, D and M, having known structures were subjected to MS/MS and MS³-fragmentation pattern analyses. As a result, MS/MS of the novel steviol glycosides gave data showing that the highest ion intensity appeared at the peak where all sugar chains attached to C-19 via an ester bond were released. This result represents the total molecular weight of the sugar chains attached to the carbon of C-19 via an ester bond.

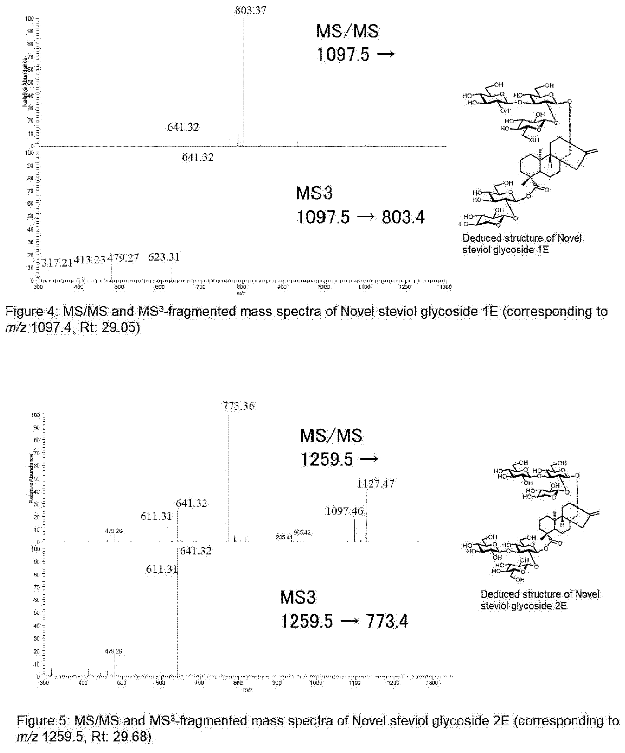

The MS/MS and MS³-fragmented mass spectra of Novel steviol glycoside IE (corresponding to m/z 1097.4, Rt: 29.05) are shown in Figure 4. In the MS/MS spectrum of the novel steviol glycoside, the main peak was detected at m/z of 803.37 corresponding to the release of one Glc moiety and one Xyl moiety. From this result, the number of sugar chains attached to the carbon of C-19 via an ester bond was found to be one Glc moiety and one Xyl moiety. In order to acquire further structural information, a MS³ spectrum was acquired by fragmenting the main peak at m/z of 803.4 obtained by MS/MS. As a result, a spectrum having the same peak pattern as the MS³ spectrum of rebaudioside A (965.4→803.4→) was acquired. Accordingly, the sugar chains attached to C-13 were presumed to be the same as rebaudioside A. The deduced structure is shown in Figure 4.

The MS/MS and MS³-fragmented mass spectra of Novel steviol glycoside 2E (corresponding to m/z 1259.5, Rt: 29.30) are shown in Figure 5. In the MS/MS spectrum of the novel steviol glycoside, the main peak was detected at m/z of 773.36 corresponding to the release of three Glc moieties. From this result, the number of sugar chains attached to the carbon of C-19 via an ester bond was found to be three Glc moieties. In order to acquire further structural information, a MS³ spectrum was acquired by fragmenting the main peak at m/z of 773.4 obtained by MS/MS. As a result, a spectrum having the same peak pattern as the MS³ spectrum of rebaudioside F (935.4→773.4→) was acquired. Accordingly, the sugar chains attached to C-13 were presumed to be the same as rebaudioside F. The deduced structure is shown in Figure 5.

### (ii) Chemical synthesis of the deduced steviol glycoside standard products (Novel steviol glycosides 1S and 2S) via chemical reaction

### [Synthesis of Novel steviol glycoside 1S]

### (1) Outline of synthetic pathways

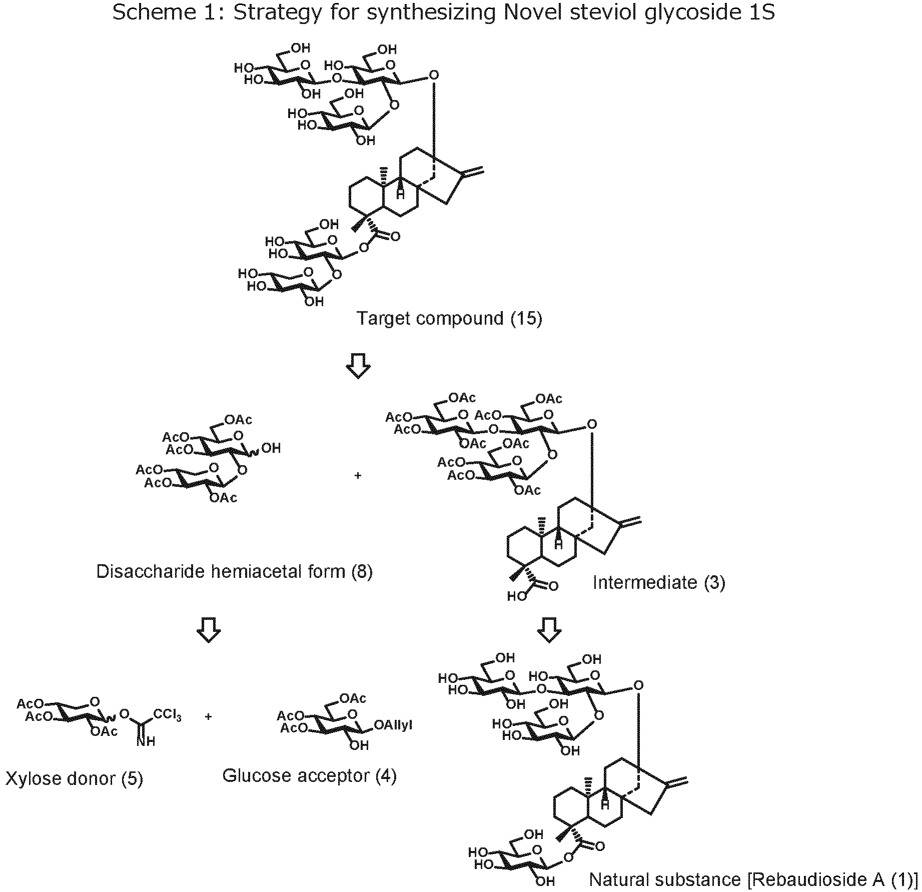

As can be appreciated from Scheme 1, for the synthesis of Novel steviol glycoside 1S (Target compound 15), the intermediate (3) and the disaccharide hemiacetal form (8) were condensed via a Mitsunobu reaction to obtain the backbone of Novel steviol glycoside 1S (Target compound 15). For the synthesis of the intermediate (3), the ester bond at C-19 of steviol of the known natural substance, i.e., rebaudioside A (1), was subjected to alkaline hydrolysis and then the hydroxy groups of the sugar chain were protected with acetyl (Ac) groups to obtain the intermediate (3). For the synthesis of the disaccharide hemiacetal form (8), a disaccharide backbone was produced through condensation reaction between an appropriately protected glucose acceptor (4) and a xylose donor (5), and the protecting group at the anomeric carbon of the reducing end was deprotected to give the disaccharide hemiacetal form (8). The resulting intermediate (3) and disaccharide hemiacetal form (8) were subjected to condensation via a Mitsunobu reaction, where the reaction proceeded in yield as high as 79% (only β) with complete β-selectivity. The protecting groups of the resulting compound were deprotected, thereby obtaining Novel steviol glycoside 1S (Target compound 15).

Next, each of the synthesis steps will be described.

### (2) Synthesis of intermediate (3)

The intermediate (3) was synthesized according to the method as described in WO2018/181515.

### (3) Synthesis of disaccharide hemiacetal form

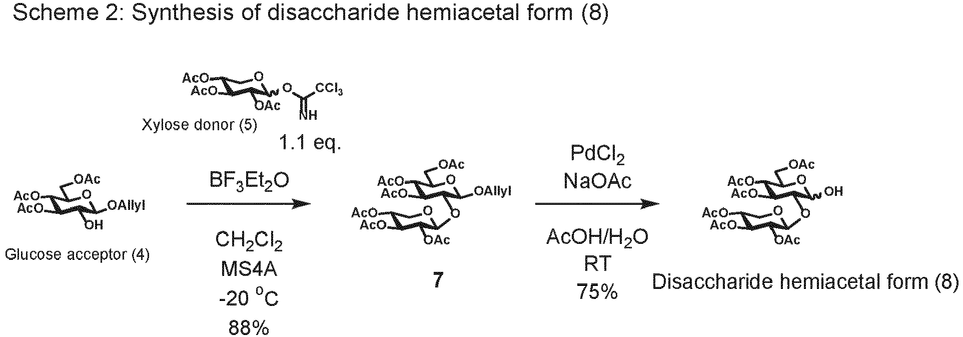

As can be appreciated from Scheme 2, for the synthesis of the disaccharide hemiacetal form (8), a glucose acceptor (4) (40.0 g, 115 mmol), a xylose donor (5) (53.4 g, 127 mmol) and 4Å molecular sieves (60 g) were dissolved in dichloromethane (1.2 L), to which a boron trifluoride-diethyl ether complex (1.46 mL, 11.5 mmol) was added at -20°C, and the resultant was agitated at -20°C for an hour. After confirming the completion of the reaction by TLC (ethyl acetate/hexane = 1/1, Rf value = 0.2), the resultant was neutralized with triethylamine (2.0 mL) (pH 8), and the 4Å molecular sieves were removed by filtration. The resultant was concentrated under a reduced pressure to obtain syrup, which was subjected to silica gel column chromatography to give Compound 7 (61.2 g, 88%) in the eluate (ethyl acetate/hexane = 1/1).

NMR spectra were determined for ¹H-NMR and ¹³C-NMR using "AVANCE III HD 400 spectrometer" manufactured by Bruker. The solvent and the frequencies used for the determinations were as follows. The same apparatus was used for determining the NMR spectra for other compounds described below.

### [Compound 7]

¹H-NMR (CDCl₃, 400 MHz) δ 2.01-2.10 (complex, 18H, OAc), 3.35 (dd, 1H), 3.68 (m, 1H), 3.73 (t, 1H), 4.13-4.27 (complex, 4H), 4.38 (m, 1H), 4.48 (d, J = 8.0 Hz, 1H), 4.74 (d, J = 6.4 Hz, 1H), 4.86 (t, 1H), 4.90-4.99 (complex, 2H), 5.09 (t, 1H), 5.35 (d, 1H), 5.91 (m, 1H); ¹³C-NMR (CDCl₃, 100 MHz) δ 20.6×2, 20.7×2, 20.8×2, 61.9, 62.0, 68.7, 68.9, 70.5, 70.7, 71.2, 71.5, 74.5, 98.9, 100.3, 117.8, 133.4, 169.4, 169.7, 170.0, 170.7.

Compound 7 (2.3 g, 3.8 mmol) was dissolved in acetic acid (100 mL) and water (10 mL), to which palladium chloride (1.2 g, 6.8 mmol) was added at room temperature, and the resultant was agitated in an argon atmosphere at room temperature for 18 hours. After confirming the completion of the reaction by TLC (chloroform/ethyl acetate = 2/1, Rf value = 0.2), palladium chloride was removed by filtration. The resultant was concentrated under a reduced pressure to obtain syrup, which was subjected to silica gel column chromatography to give the disaccharide hemiacetal form (8) (1.6 g, 75%) in the eluate (chloroform/ethyl acetate = 2/1).

### [Disaccharide hemiacetal form (8)]

¹H-NMR (CDCl₃, 400 MHz) δ 2.00-2.15 (complex, 27H, OAc), 3.31-3.39 (complex, 2H), 3.53-3.79 (complex, 3H), 3.86 (d, J = 2.8 Hz, 1H), 4.06-4.33 (complex, 6H), 4.58 (d, J = 7.2 Hz, 1H), 4.83-5.06 (complex, 5H), 5.09-5.22 (complex, 2H), 5.35 (m, 1H), 5.45 (t, 1H); ¹³C-NMR (CDCl₃, 100 MHz) δ 20.4, 20.5, 20.6×2, 20.7×2, 61.9, 62.2, 67.2, 68.3×2, 68.5, 68.7, 70.7, 71.4, 71.5, 76.7, 92.0, 101.6, 169.5, 169.7, 169.8×2, 170.1, 170.7.

### (4) Synthesis of Compound 15

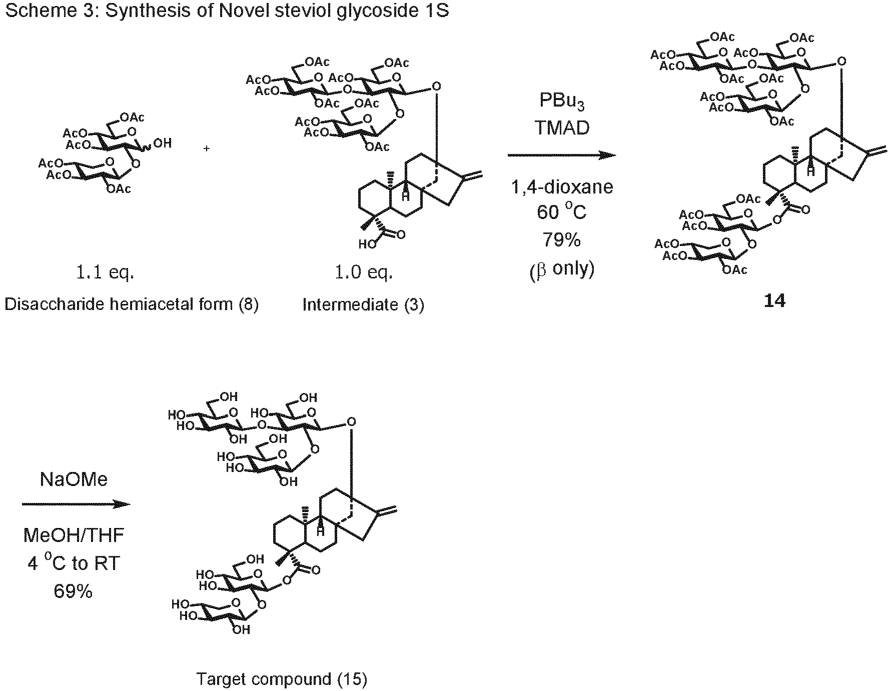

As can be appreciated from Scheme 3, for the synthesis of Compound 14, the disaccharide hemiacetal form (8) (9.4 g, 16.7 mmol) and the intermediate (3) (18.6 g, 15.1 mmol) were dissolved in 1,4-dioxane (150 mL), to which tributylphosphine (14.9 mL, 60.6 mmol) and 1,1'-azobis(N,N'-dimethylformamide) (TMAD) (10.4 g, 60.6 mmol) were added at room temperature and the resultant was agitated at 60°C for an hour. After confirming the completion of the reaction by TLC (toluene/ethyl acetate = 3/2, Rf value = 0.2), the resultant was diluted with ethyl acetate. The organic layer was washed with water, a saturated aqueous solution of sodium hydrogen carbonate and saturated saline, and dried with magnesium sulfate. Magnesium sulfate was removed by filtration. The resultant was concentrated under a reduced pressure to obtain syrup, which was subjected to silica gel column chromatography to give Compound 14 (21.1 g, 79%) in the eluate (toluene/ethyl acetate = 3/2).

### [Compound 14]

¹H-NMR (CDCl₃, 400 MHz) δ 0.75-1.32 (complex, 13H), 1.37-2.32 (complex, 77H), 3.36 (t, 1H), 3.50-3.72 (complex, 4H), 3.75-4.29 (complex, 14H), 4.41 (m, 1H), 4.52-4.65 (complex, 2H), 4.77-5.27 (complex, 20H), 5.62 (d, J = 7.6 Hz, 1H); ¹³C-NMR (CDCl₃, 100 MHz) δ 16.3, 19.3, 20.3, 20.4, 20.5×2, 20.6×3, 20.7×3, 20.9, 21.2, 21.3, 28.9, 36.5, 36.8, 39.3, 40.3, 41.1, 42.6, 43.3, 43.8, 45.1, 47.2, 53.5, 57.3, 60.3, 61.1, 61.7, 62.0, 62.6, 67.9, 68.0, 68.1, 68.3, 68.7, 70.6, 71.4, 71.6, 71.7, 71.8, 72.1, 72.9, 73.0, 74.7, 80.1, 85.8, 85.9, 91.2, 96.2, 98.9, 99.2, 100.9, 104.5, 125.2, 128.1, 128.7, 152.7, 168.9, 169.2, 169.3×2, 169.6, 169.7, 169.9, 170.0×2, 170.3×2, 170.5, 170.7, 174.5.

Compound (14) (20.0 g, 11.3 mmol) was dissolved in methanol (100 mL) and THF (100 mL), to which sodium methoxide (0.5M in methanol) (25 mL) was added at 4°C and the resultant was agitated at room temperature for an hour. After confirming the completion of the reaction by TLC (chloroform/methanol/water = 5/4/0.1, Rf value = 0.1), the resultant was neutralized by adding Amberlite 120B (H). The resultant was concentrated under a reduced pressure to obtain syrup, which was subjected to gel filtration column (GE Healthcare, Sephadex LH-20, ethanol) to give Compound 15 (10.2 g, 69%).

### [Compound 15]

¹H-NMR (pyridine-d5, 400 MHz) δ 0.70 (m, 1H), 0.88 (m, 1H), 0.91-1.40 (complex, 10H), 1.51-2.21 (complex, 14H), 2.43-2.55 (complex, 2H), 3.59-3.70 (complex, 2H), 3.77-4.51 (complex, 36H), 4.91 (s, 1H), 5.03 (d, J = 8.0 Hz, 1H), 5.20 (d, J = 7.6 Hz, 1H), 5.35 (d, J = 7.6 Hz, 1H), 5.52 (d, J = 8.0 Hz, 1H), 5.61 (m, 1H), 6.16 (d, J = 7.6 Hz, 1H); ¹³C-NMR (pyridine-d5, 100 MHz) δ 17.1, 20.4, 21.0, 22.9, 29.4, 38.1, 38.6, 40.2, 41.1, 42.3, 42.6, 44.4, 44.8, 45.9, 48.2, 54.5, 57.9, 62.6, 62.8×2, 63.8, 67.9, 70.4, 71.3, 71.6, 72.1, 72.8, 75.9, 76.3, 76.9, 77.9, 78.5, 78.7, 78.8, 79.0, 79.1, 79.5, 81.5, 82.3, 87.3, 88.6, 94.1, 98.2, 105.0, 105.1, 105.3, 107.1, 154.4, 176.4.

### [Synthesis of Novel steviol glycoside 2S]

### (1) Outline of synthetic pathways

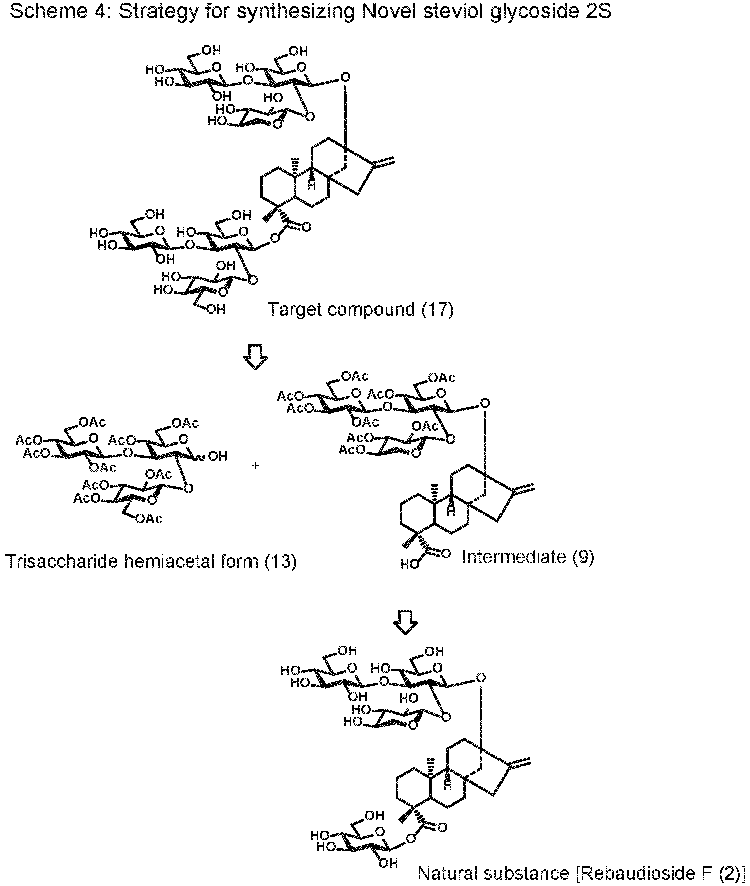

As can be appreciated from Scheme 4, for the synthesis of Novel steviol glycoside 2S (17), the intermediate (9) and the trisaccharide hemiacetal form (13) were condensed via a Mitsunobu reaction to obtain the backbone of Novel steviol glycoside 2S (17). For the synthesis of the intermediate (9), the ester bond at C-19 of steviol of the known natural substance, i.e., rebaudioside F (2), was subjected to alkaline hydrolysis and then the hydroxy groups of the sugar chain were protected with acetyl (Ac) groups to obtain the intermediate (9). The resulting intermediate (9) and the trisaccharide hemiacetal form (13) were subjected to condensation via a Mitsunobu reaction, where the reaction proceeded in yield as high as 53% (only β) with complete β-selectivity. The protecting groups of the resulting compound were deprotected, thereby obtaining Novel steviol glycoside 2S (17).

Next, each of the synthesis steps will be described.

### (2) Synthesis of intermediate (9)

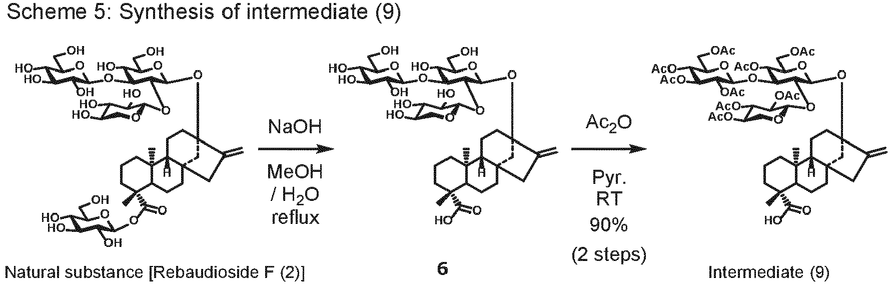

As can be appreciated from Scheme 5, for the synthesis of the intermediate (9), rebaudioside F (2) (1.8 g, 1.9 mmol) was dissolved in methanol (20 mL) and water (10 mL), to which 2 mol/L sodium hydroxide (10 mL) was added at room temperature, and the resultant was refluxed at 100°C for 3 hours. After confirming the completion of the reaction by TLC (chloroform/methanol = 4/1, Rf value = 0.2), the reaction solution was neutralized with Amberlite 120B (H) (pH 7). After the resin was removed by filtration, the resultant was concentrated under a reduced pressure to give Compound 6 (2.1 g)

Compound 6 (2.1 g) was dissolved in pyridine (20 mL), to which acetic anhydride (3.6 mL) was added at room temperature, and the resultant was agitated at room temperature for 24 hours. After confirming the completion of the reaction by TLC (chloroform/methanol = 50/1, Rf value = 0.2), a saturated aqueous solution of sodium hydrogen carbonate (40 mL) was added at 0°C and extraction was repeated for three times with ethyl acetate. The organic layer was concentrated under a reduced pressure to obtain syrup, which was subjected to silica gel column chromatography to give the intermediate 9 (2.0 g, 90% (2 steps)) in the eluate (chloroform/methanol = 50/1).

### [Intermediate 9]

¹H-NMR (CDCl₃, 400 MHz) δ 0.81 (m, 1H), 0.89-1.12 (complex, 8H), 1.22 (s, 3H), 1.41-2.22 (complex, 50H), 3.49 (dd, 1H), 3.58 (m, 1H), 3.65 (m, 1H), 3.85 (t, 1H), 3.96-4.15 (complex, 4H), 4.42 (m, 2H), 4.56 (d, J = 7.6 Hz, 1H), 4.81-4.94 (complex, 6H), 5.00 (t, 1H), 5.04-5.14 (complex, 3H), 5.25 (t, 1H); ¹³C-NMR (CDCl₃, 100 MHz) δ 16.0, 17.3, 19.1, 20.5, 20.6, 20.7, 20.8×2, 20.9, 21.8, 29.1, 29.8, 37.9, 38.0, 39.5, 40.7, 41.5, 42.2, 43.8, 44.0, 48.4, 53.8, 56.8, 61.7, 63.1, 66.8, 68.0, 68.7, 68.8, 69.7, 71.0, 71.6, 71.9, 72.4, 72.8, 81.3, 87.3, 96.6, 96.8, 99.2, 105.6, 151.9, 169.0, 169.5, 169.6, 170.1×2, 170.3, 170.6, 170.9, 171.0, 182.5.

### (3) Synthesis of trisaccharide hemiacetal form

The trisaccharide hemiacetal form was synthesized according to the method as described in WO2018/181515.

### (4) Synthesis of Compound 17

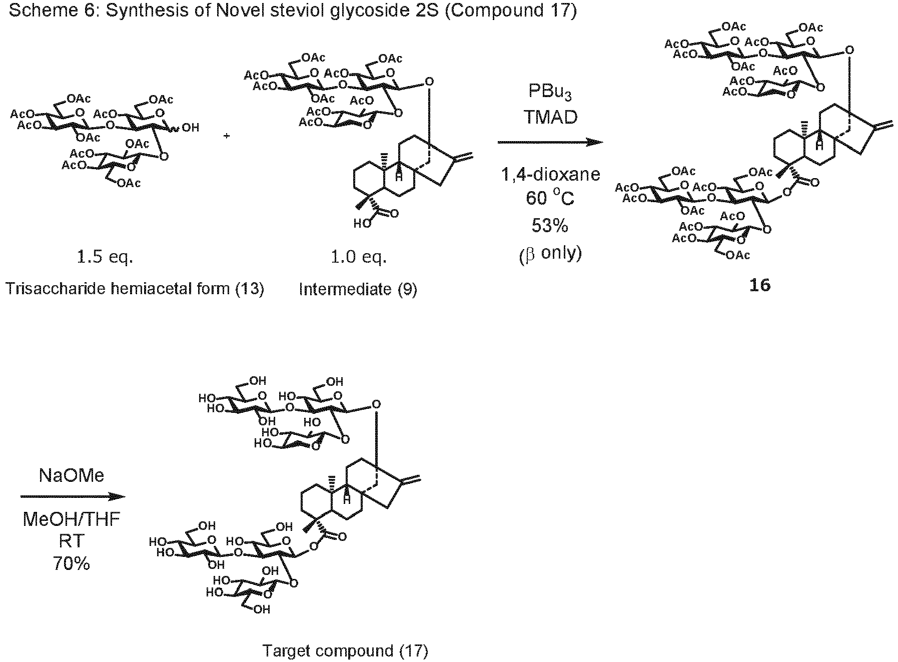

As can be appreciated from Scheme 6, for the synthesis of Compound 16, the trisaccharide hemiacetal form (13) (2.4 g, 2.6 mmol) and the intermediate (9) (2.0 g, 1.7 mmol) were dissolved in 1,4-dioxane (20 mL), to which tributylphosphine (4.3 mL, 17.3 mmol) and 1,1'-azobis(N,N'-dimethylformamide) (TMAD) (3.0 g, 17.3 mmol) were added at room temperature and the resultant was agitated at 60°C for 2 hours. After confirming the completion of the reaction by TLC (ethyl acetate/heptane = 2/1, Rf value = 0.1), the resultant was diluted with ethyl acetate. The organic layer was washed with water, a saturated aqueous solution of sodium hydrogen carbonate and saturated saline, and dried with magnesium sulfate. Magnesium sulfate was removed by filtration. The resultant was concentrated under a reduced pressure to obtain syrup, which was subjected to silica gel column chromatography to give Compound 16 (1.9 g, 53%, only β) in the eluate (ethyl acetate/heptane = 2/1).

### [Compound 16]

¹H-NMR (CDCl₃, 400 MHz) δ 0.78-1.05 (complex, 9H), 1.25 (t, 4H), 1.36-2.31 (complex, 86H), 3.51 (dd, 1H), 3.59 (m, 1H), 3.61-3.78 (complex, 4H), 3.81 (t, 1H), 3.91-4.21 (complex, 11H), 4.31 (dd, 1H), 4.40-4.59 (complex, 4H), 4.73-5.29 (complex, 21H), 5.60 (d, J = 7.2 Hz, 1H); ¹³C-NMR (CDCl₃, 100 MHz) δ 16.8, 17.5, 19.5, 20.5, 20.7×3, 20.8×3, 20.9×3, 21.0, 21.1×2, 21.5, 29.2, 37.4, 37.5, 39.6, 40.5, 41.6, 42.5, 43.8, 44.2, 48.1, 53.8, 57.4, 61.7, 62.0, 62.1, 62.3, 63.1, 66.7, 67.4, 68.0, 68.3, 68.4, 68.6, 68.8, 69.7, 71.1, 71.5, 71.8, 71.9, 72.0, 72.2, 72.3, 72.4, 72.9, 73.1, 75.0, 80.1, 81.5, 86.8, 91.3, 96.4, 97.0, 99.2, 99.3, 99.6, 104.9, 152.8, 169.0, 169.1, 169.3, 169.5×2, 169.6, 170.1×2, 170.2×3, 170.5, 170.6, 170.9, 174.8.

Compound (16) (2.2 g, 1.1 mmol) was dissolved in methanol (10 mL) and THF (10 mL), to which sodium methoxide (0.5M in MeOH) (2.5 mL) was added at room temperature, and the resultant was agitated at room temperature for 3 hours. After confirming the completion of the reaction by TLC (chloroform/methanol/water = 5/4/1, Rf value = 0.4), the resultant was neutralized with Amberlite 120B (H) (pH 7). After the resin was removed by filtration, the resultant was concentrated under a reduced pressure, which was dissolved in MeCN/H₂O = 1/2 and lyophilized to give Compound 17 (1.0 g, 70%).

### [Compound 17]

¹H-NMR (pyridine-d5, 400 MHz) δ 0.78 (m, 1H), 0.91 (m, 1H), 1.08 (m, 1H), 1.31-1.48 (complex, 9H), 1.62-1.80 (complex, 3H), 1.81-1.89 (complex, 3H), 2.00-2.06 (complex, 2H), 2.29 (m, 3H), 2.47 (m, 1H), 2.69 (m, 1H), 2.80 (m, 1H), 3.41 (t, 1H), 3.81 (m, 1H), 3.90-4.41 (complex, 33H), 4.53-4.61 (complex, 2H), 4.70 (m, 1H), 4.88-5.22 (complex, 14H), 5.30 (d, J = 8.0 Hz, 1H), 5.37 (d, J = 7.6 Hz, 1H), 5.51-5.57 (complex, 2H), 5.61 (s, 1H), 5.77 (s, 2H), 5.84 (d, J = 7.6 Hz, 1H), 6.46 (d, J = 8.0 Hz, 1H); ¹³C-NMR (pyridine-d5, 100 MHz) δ 18.3, 21.2, 21.7, 25.1, 29.8, 39.9, 41.3, 41.7, 42.5, 44.2, 44.7, 45.8, 47.9, 55.9, 58.9, 63.3, 63.5, 63.6, 64.2, 65.6, 68.5, 71.7, 72.2, 72.6, 72.9, 75.2, 76.9, 77.0, 77.2, 78.4, 79.2, 79.3, 79.4, 79.5, 79.6, 79.9, 80.0, 80.6, 83.7, 89.2, 89.5, 90.2, 96.5, 97.7, 105.4, 105.7, 105.9, 106.4, 107.3, 154.9, 178.5.

### (iii) Structural determination by matching with the retention time and the fragmented pattern of the chemically synthesized standard product in HPLC-high resolution MS and MS³-fragmentation

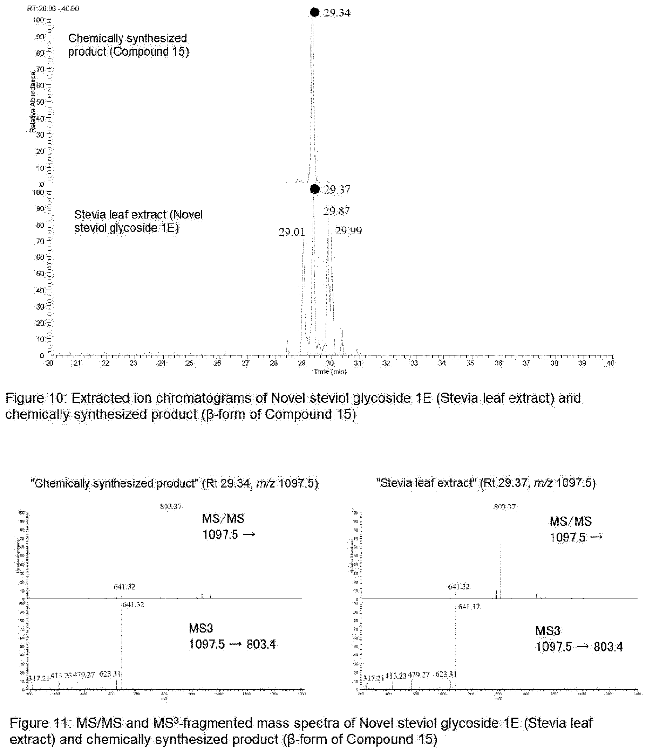

The chemically synthesized product of Novel steviol glycoside 1 (β-form of Compound 15) and the stevia leaf liquid extract were compared by HPLC-high resolution MS/MS and MS³-fragmentation by using Orbitrap Elite MS (from Thermo Fisher Scientific) equipped with a HPLC-ESI ion source under the conditions described in (i). As a result, the peaks of the chemically synthesized product and the stevia leaf liquid extract were detected at the retention time of 29.34 and 29.37 minutes, respectively (Figure 10). Here, the peaks at the retention time of 29.34 and 29.37 minutes in Figure 10 corresponded to the peak at the retention time of 29.05 minutes in Figure 2, which was confirmed by determining the retention time of the chemically synthesized product with the apparatus used in Figure 2 (LCMS-8030). Moreover, they also matched in the respective MS/MS and MS³-fragmented mass spectra (Figure 11). From this result, Novel steviol glycoside IE obtained from the liquid extract of the plant was confirmed to have the same structure as the β-form of Compound 15.

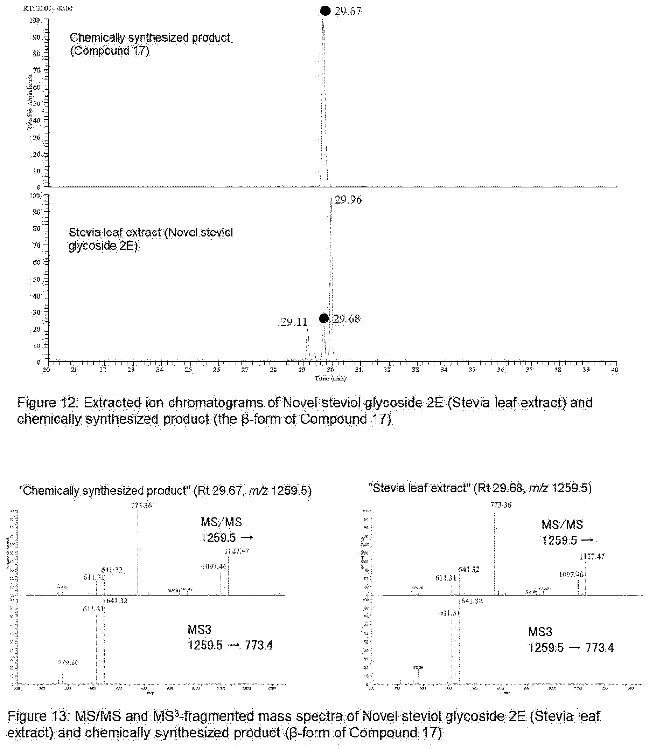

Furthermore, the chemically synthesized product of Novel steviol glycoside 2 (β-form of Compound 17) and the stevia leaf liquid extract were compared by HPLC-high resolution MS/MS and MS³-fragmentation by using Orbitrap Elite MS (from Thermo Fisher Scientific) equipped with a HPLC-ESI ion source under the conditions described in (i). As a result, the peaks of the chemically synthesized product and the stevia leaf liquid extract were detected at the retention time of 29.67 and 29.68 minutes, respectively (Figure 12). Here, the peaks at the retention time of 29.67 and 29.68 minutes in Figure 12 corresponded to the peak at the retention time of 29.30 minutes in Figure 3, which was confirmed by determining the retention time of the chemically synthesized product with the apparatus used in Figure 3 (LCMS-8030). Moreover, they also matched in the respective MS/MS and MS³-fragmented mass spectra (Figure 13). From this result, Novel steviol glycoside 2E obtained from the liquid extract of the plant was confirmed to have the same structure as the β-form of Compound 17.

### Evaluation of sweetness level of novel steviol glycosides

In order to evaluate the sweetness levels of Novel steviol glycosides A and B, samples were prepared by adding sugar to pure water to give Brix of 3.0 to 5.0 in 0.5 increments. Compounds 15 and 17 obtained by the chemical syntheses were used as Novel steviol glycosides A and B, respectively, where 48 mg of each sample was dissolved in 400 mL of pure water to be tested. In addition, samples were also prepared for comparison by dissolving 48 mg of each of Reb.A, Reb.D and Reb.M in 400 mL of pure water.

### (1) Evaluation of sweetness level of Novel steviol glycoside A

Evaluation was conducted by choosing a sugar-added sample that had an equivalent sweetness intensity to that of the sample added with the novel steviol glycoside, where sensory evaluation was conducted by panelists who had been trained about sensory attributes of sweeteners (7 members). As a result, Novel steviol glycoside A of the present invention was found to have a sweetness level that was about 354 times higher in average than that of sugar.

**Table 10: Evaluation of sweetness level of Novel steviol glycoside A**

| Sweetness level | Novel steviol glycoside A | Reb A | Reb D | Reb M |
|---|---|---|---|---|
| Lowest | 319.0 | 243.0 | 253.5 | 286.0 |
| Highest | 415.8 | 398.9 | 398.0 | 352.2 |
| Average | 354.1 | 312.0 | 317.9 | 341.1 |

### (2) Evaluation of sweetness level of Novel steviol glycoside B

Sweetness level was evaluated in the same manner as Novel steviol glycoside A. As a result, Novel steviol glycoside B of the present invention was found to have a sweetness level that was about 250 times higher in average than that of sugar. The results are shown in the table below.

**Table 11: Evaluation of sweetness level of Novel steviol glycoside B**

| Sweetness level | Novel steviol glycoside B | Reb A | Reb D | Reb M |
|---|---|---|---|---|
| Lowest | 223.6 | 239.5 | 241.1 | 236.5 |
| Highest | 299.2 | 329.2 | 393.6 | 373.4 |
| Average | 249.6 | 288.3 | 310.3 | 323.6 |

### Sensory evaluation of Novel steviol glycoside A (Compound 15)

In order to evaluate the taste quality of various steviol glycosides, Reb.A, Reb.D, Reb.M, sugar and Novel steviol glycoside A (Compound 15) were each added to pure water to prepare beverage samples. All of the beverage samples were adjusted to have final sweetness level (Brix) of 5 in terms of sugar (sucrose), and thus the sweetness levels of Reb.A, Reb.D, Reb.M and Novel steviol glycoside A (Compound 15) were 312, 317, 341 and 354, respectively.

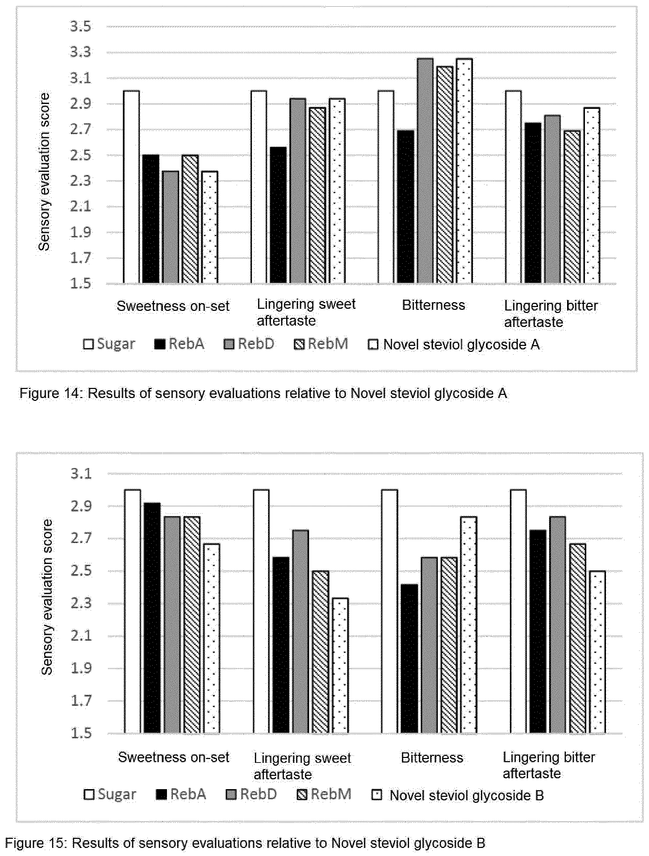

The resulting beverage samples were subjected to sensory evaluation for rating the attributes, which were sweetness on-set, lingering sweet aftertaste, bitterness and lingering bitter aftertaste. Panelists who had been trained about sensory attributes of sweeteners (7 members) evaluated based on the following evaluation criteria. For each evaluation item, the steviol glycosides were scored in 0.5 increments provided that the score of sugar was 3. A higher score represents faster sweetness on-set, shorter lingering sweet aftertaste, less bitterness and shorter lingering bitter aftertaste. The results are shown in Figure 14. The evaluation scores shown in the diagram are the average scores of the scores from the 8 panelists. As a result of the sensory evaluations, Novel steviol glycoside A was found to have less sweetness and shorter lingering bitter aftertaste as compared to other glycosides and less bitterness as compared to other components including sugar.

### Sensory evaluation of Novel steviol glycoside B (Compound 17)

In order to evaluate the taste quality of various steviol glycosides, Reb.A, Reb.D, Reb.M, sugar and Novel steviol glycoside B (Compound 17) were each added to pure water to prepare beverage samples. All of the beverage samples were adjusted to have final sweetness level (Brix) of 5 in terms of sugar (sucrose), and thus the sweetness levels of Reb.A, Reb.D, Reb.M and Novel steviol glycoside B (Compound 17) were 288, 310, 324 and 250, respectively.

The resulting beverage samples were subjected to sensory evaluation for rating the attributes, which were sweetness on-set, lingering sweet aftertaste, bitterness and lingering bitter aftertaste. Panelists who had been trained about sensory attributes of sweeteners (7 members) evaluated based on the following evaluation criteria. For each evaluation item, the steviol glycosides were scored in 0.5 increments provided that the score of sugar was 3. A higher score represents faster sweetness on-set, shorter lingering sweet aftertaste, less bitterness and shorter lingering aftertaste. The results are shown in Figure 15. The evaluation scores shown in the diagram are the average scores of the scores from the 6 panelists. As a result of the sensory evaluations, Novel steviol glycoside B was found to have less bitterness as compared to other glycosides.

### Identification of genetic features of plant containing Novel steviol glycosides A and B

Lines rich in Novel steviol glycosides A and B (Mutants: Cultivar lines A and B) and lines with small Novel glycosides A and B contents (Wild-types: Cultivar lines X and Y) were used to identify the genetic features specific to the lines rich in Novel steviol glycosides A and B. When the genomes of the respective lines were sequenced, the lines rich in Novel steviol glycosides A and B were found to have substitutions at the 298th, 328th, 360th, 386th, 393rd, 411th, 427th and 453rd bases of SEQ ID NO:1, insertion of 15 bases between the 90th and 91st bases of SEQ ID NO: 101, and substitutions at the 98th, 102nd, 111th, 113th, 116th, 119th and 122nd bases of SEQ ID NO: 103 with respect to the wild-types (nucleotide sequences of the mutant lines corresponding to SEQ ID NOS:1, 101 and 103 are shown as SEQ ID NOS:2, 102 and 104, respectively). In addition, the substitutions in SEQ ID NO:1, the insertion in SEQ ID NO:101 and the substitutions in SEQ ID NO: 103 were found to exist in the introns of the genes coding for the protein including the amino acid sequences represented by SEQ ID NOS:97, 105 and 107, respectively (herein, sometimes referred to as PI, P2 and P3). Subsequently, whether or not these mutations affect the expression level of each gene was examined. After 100 mg of the expanded leaves of Cultivars A, B, X (SR001) and Y (SS075-49) were cryogenically ground with liquid nitrogen, total RNA was extracted using RNAeasy Plant mini kit from QIAGEN according to the manufacturer's protocol. 500 ng of the extracted total RNA was used for reverse transcription. For the reverse transcription, cDNA was synthesized using SuperScript IV VILO manufactured by Thermo Fisher Scientific according to the manufacturer's protocol. Semi-quantitative PCR was conducted using 1 µL of a 10-fold dilution of the reverse transcription reaction solution. Semi-quantitative PCR was carried out with Ampdirect manufactured by Shimadzu Corporation according to the manufacturer's protocol. PCR reaction was performed by heat denaturation at 95°C for 10 minutes, followed by 32, 33 and 28 cycles of reactions at 94°C for 30 seconds, 55°C for 30 seconds and 72°C for 25 seconds, for "P3", "P1 and P2" and "actin and ubiquitin as controls", respectively. PCR reaction was followed by electrophoresis using LabChip GX Touch manufactured by PerkinElmer according to the manufacturer's protocol.

The following primers were used for semi-quantitative PCR.
P1
   Forward: CCTGTTCATTACAAATTCAACCCG (SEQ ID NO:99)
   Reverse: AACCCTAACATGTTCAATGTCCCTA (SEQ ID NO:100)
P2
   Forward: ATCAGATTTATCATCTTGCATGCCC (SEQ ID NO:109)
   Reverse: TGCCAATTACATTCGTCTTAATCGT (SEQ ID NO:110)
P3
   Forward: AAAAGTTGCTGGTTGAAGTTGATCA(SEQ ID NO:111)
   Reverse: CACACTAAATATGCTTGGTCTTGC (SEQ ID NO: 112)
Actin
   Forward: CGCCATCCTCCGTCTTGATCTTGC (SEQ ID NO: 113)
   Reverse: CCGTTCGGCGGTGGTGGTAA (SEQ ID NO:114)
Ubiquitin
   Forward: TCACTCTTGAAGTGGAGAGTTCCGA (SEQ ID NO:115)
   Reverse: GCCTCTGTTGGTCCGGTGGG (SEQ ID NO:116)

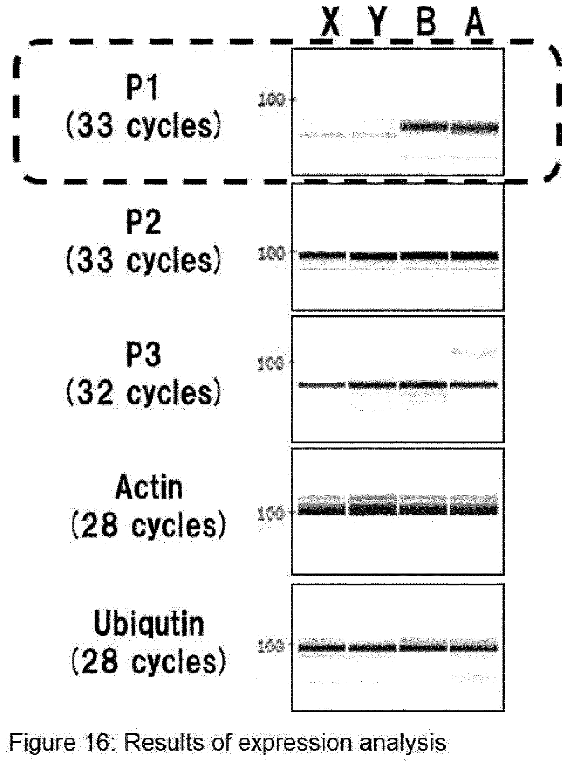

The results of the expression analysis are shown in Figure 16 and Table 12. The numerical values shown in Table 12 are the relative band intensities of the P1-P3 genes to the band intensity of the ubiquitin gene, i.e., 100, in the electrophoresis image shown in Figure 16.

**Table 12: Expression levels of P1-P3 genes in each cultivar**

| Sample name | P1 | P2 | P3 |
|---|---|---|---|
| Cultivar A | 146.27 | 126.23 | 99.01 |
| Cultivar B | 124.57 | 107.76 | 93.79 |
| Cultivar X | 0.20 | 72.14 | 40.23 |
| Cultivar Y | 0.13 | 94.66 | 77.95 |

From these results, the P1 gene was confirmed to be highly expressed in the two lines including Novel Glycosides A and B. The mutations found in the P1 gene exist in the introns and the existence of one or more of these mutations seemed to enhance the expression level of PI, by which the syntheses of Novel glycosides A and B were promoted in the plant.

### Identification of content of Novel Glycoside A in plant having genetic features 1-8

Suitable amounts of fresh leaves were sampled from individuals of Cultivars A, B, X and Y that were used in the above-described "Identification of genetic features of plant containing Novel steviol glycosides A and B" to quantify the concentrations of the sweetness components by LC/MS-MS (Shimadzu LCMS8050). Specifically, a prescribed amount of fresh leaves was freeze-dried, and the crushed dry pieces were fed into pure water. The resultant was subjected to an ultrasonic treatment for 20 minutes for extraction, centrifuged and filtrated to give 5 mL of a liquid extract. This liquid extract was subjected to LC/MS-MS analysis with LCMS8050 in ion mode (Shimadzu LCMS8050) to quantify the concentrations of Reb.A, Reb.B, Reb.C, Reb.D, Reb.E, Reb.F, Reb.G, Reb.I, Reb.M, Reb.N, stevioside, dulcoside A, steviol bioside, rubusoside and Novel glycoside A. The total thereof was considered to be the concentration of the sweetness components (amount of total steviol glycosides (TSG) of this example). All of the water content of the dried leaves was less than about 3%. The results are shown in Tables 13-17. The values of the following results are the average values of two measurements.

**Table 13: Amount of TSG in dried leaves of each cultivar**

| **Sample name** | **Weight of stevia leaves (g)** | **Concentration of TSG (mg/L)** | **Amount of TSG (mg)** | **Rate of TSG (%)** |
|---|---|---|---|---|
| **Cultivar A** | 0.0565 | 1617.35 | 8.087 | 14.3% |
| **Cultivar B** | 0.0501 | 905.42 | 4.527 | 9.0% |
| **Cultivar X** | 0.0445 | 1142.40 | 5.712 | 12.9% |
| **Cultivar Y** | 0.0576 | 1267.14 | 6.336 | 11.1% |

**Table 14: Rate of each steviol glycoside in TSG contained in dried leaves of each cultivar**

| | **Rate of each steviol glycoside in TSG** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside A** | **Total*** |
| **Cultivar A** | 40.4% | 0.2% | 36.6% | 0.7% | 11.0% | 7.9% | 0.2% | 0.2% | 0.061% | 100% |
| **Cultivar B** | 64.6% | 0.6% | 11.8% | 7.7% | 5.5% | 4.0% | 2.3% | 1.3% | 0.205% | 100% |
| **Cultivar X** | 24.1% | 0.1% | 6.1% | 0.5% | 62.4% | 1.2% | 0.0% | 0.1% | 0.017% | 100% |
| **Cultivar Y** | 38.9% | 0.2% | 6.5% | 1.9% | 47.9% | 1.7% | 0.1% | 0.4% | 0.045% | 100% |

| | | | | | | | | | | |
|---|---|---|---|---|---|---|---|---|---|---|
| ^{∗}: Total content includes Reb A, Reb B, Reb C, Reb D, Reb E, Reb F, Reb G, Reb I, Reb M, Reb N, stevioside, dulcoside A, steviol bioside, rubusoside and Novel steviol glycoside A. | | | | | | | | | | |

**Table 15: Rate of Novel glycoside A relative to eight types of major steviol glycosides^{∗1}**

| Sample name | Rate of steviol glycoside A |
|---|---|
| Cultivar A | 0.063% |
| Cultivar B | 0.210% |
| Cultivar X | 0.018% |
| Cultivar Y | 0.046% |

| | |
|---|---|
| ^{∗1}: Reb A, Reb B, Reb C, Reb D, Reb F, Reb M, Reb N and stevioside recited in Table 14 | |

**Table 16: Rate of each steviol glycoside contained in dried leaves of each cultivars**

| | **Content of each steviol glycoside in dried leaves** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside A** | **TSG** |
| **Cultivar A** | 5.8% | 0.0% | 5.2% | 0.1% | 1.6% | 1.1% | 0.0% | 0.0% | 0.009% | 14.3% |
| **Cultivar B** | 5.8% | 0.1% | 1.1% | 0.7% | 0.5% | 0.4% | 0.2% | 0.1% | 0.019% | 9.0% |
| **Cultivar X** | 3.1% | 0.0% | 0.8% | 0.1% | 8.0% | 0.1% | 0.0% | 0.0% | 0.002% | 12.9% |
| **Cultivar Y** | 4.3% | 0.0% | 0.7% | 0.2% | 5.3% | 0.2% | 0.0% | 0.0% | 0.005% | 11.1% |

**Table 17: Rate of Novel steviol glycoside A relative to each steviol glycoside contained in dried leaves of each cultivar**

| | **Rate of Novel steviol glycoside A relative to each steviol glycoside contained in dried leaves** | | | | | | | | |
|---|---|---|---|---|---|---|---|---|---|
| **Sample name** | **Reb A** | **Reb B** | **Reb C** | **Reb D** | **Stevioside** | **Reb F** | **Reb M** | **Reb N** | **Reb A+ Stevioside** |
| **Cultivar A** | 0.15% | 30.00% | 0.16% | 8.57% | 0.55% | 0.76% | 30.00% | 30.00% | 0.12% |
| **Cultivar B** | 0.33% | 35.00% | 1.78% | 2.73% | 3.82% | 5.25% | 9.13% | 16.15% | 0.30% |
| **Cultivar X** | 0.08% | 20.00% | 0.33% | 4.00% | 0.03% | 1.67% | - | 20.00% | 0.02% |
| **Cultivar Y** | 0.13% | 25.00% | 0.77% | 2.63% | 0.10% | 2.94% | 50.00% | 12.50% | 0.06% |

### Identification of content of Novel Glycoside B in plant having genetic features 1-8

Suitable amounts of fresh leaves were sampled from individuals of Cultivars B and X that were used in the above-described "Identification of genetic features of plant containing Novel steviol glycosides A and B" to quantify the concentrations of the sweetness components by LC/MS-MS (Shimadzu LCMS8050). Specifically, a prescribed amount of fresh leaves was freeze-dried, and the crushed dry pieces were fed into pure water. The resultant was subjected to an ultrasonic treatment for 20 minutes for extraction, centrifuged and filtrated to give 5 mL of a liquid extract. This liquid extract was subjected to LC/MS-MS analysis with LCMS8050 in ion mode (Shimadzu LCMS8050) to quantify the concentrations of Reb.A, Reb.B, Reb.C, Reb.D, Reb.E, Reb.F, Reb.G, Reb.I, Reb.M, Reb.N, stevioside, dulcoside A, steviol bioside, rubusoside and Novel steviol glycoside B. The total thereof was considered to be the concentration of the sweetness components (amount of total steviol glycosides (TSG) of this example). All of the water content of the dried leaves was less than about 3%. The results are shown in Tables 18-22.

**Table 18: Amount of TSG in dried leaves of each cultivar**

| **Sample name** | **Weight of stevia leaves (g)** | **Concentration of TSG (mg/L)** | **Amount of TSG (mg)** | **Rate of TSG (%)** |
|---|---|---|---|---|
| **Cultivar B** | 0.0524 | 777.42 | 3.887 | 7.4% |
| **Cultivar X** | 0.0553 | 1672.45 | 8.362 | 15.1% |

**Table 19: Rate of each steviol glycoside in TSG contained in dried leaves of each cultivar**

| | **Rate of each steviol glycoside in TSG** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside B** | **Total*** |
| **Cultivar B** | 66.6% | 0.7% | 7.2% | 10.0% | 4.1% | 2.2% | 4.9% | 1.9% | 1.328% | 100% |
| **Cultivar X** | 30.8% | 0.1% | 6.0% | 0.8% | 58.0% | 1.1% | 0.0% | 0.1% | 0.000% | 100% |

| | | | | | | | | | | |
|---|---|---|---|---|---|---|---|---|---|---|
| ^{∗}: Total content includes Reb A, Reb B, Reb C, Reb D, Reb E, Reb F, Reb G, Reb I, Reb M, Reb N, stevioside, dulcoside A, steviol bioside, rubusoside and Novel steviol glycoside B. | | | | | | | | | | |

**Table 20: Rate of Novel glycoside B relative to eight types of major steviol glycosides^{∗1}**

| Sample name | Rate of steviol glycoside B |
|---|---|
| Cultivar B | 1.360% |
| Cultivar X | 0.000% |

| | |
|---|---|
| ^{∗1}: Reb A, Reb B, Reb C, Reb D, Reb F, Reb M, Reb N and stevioside recited in Table 19 | |

**Table 21: Rate of each steviol glycoside contained in dried leaves of each cultivars**

| | **Content of each steviol glycoside in dried leaves** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside B** | **TSG** |
| **Cultivar B** | 4.9% | 0.1% | 0.5% | 0.7% | 0.3% | 0.2% | 0.4% | 0.1% | 0.099% | 7.4% |
| **Cultivar X** | 4.7% | 0.0% | 0.9% | 0.1% | 8.8% | 0.2% | 0.0% | 0.0% | 0.000% | 15.1% |

**Table 22: Rate of Novel steviol glycoside B relative to each steviol glycoside contained in dried leaves of each cultivar**

| | **Rate of Novel steviol glycoside B relative to each steviol glycoside contained in dried leaves** | | | | | | | | |
|---|---|---|---|---|---|---|---|---|---|
| **Sample name** | **Reb A** | **Reb B** | **Reb C** | **Reb D** | **Stevioside** | **Reb F** | **Reb M** | **Reb N** | **Reb A+ Stevioside** |
| **Cultivar B** | 1.99% | 178.85% | 18.32% | 13.29% | 32.22% | 59.12% | 27.25% | 71.02% | 1.88% |

## Claims

1. A stevia plant comprising a compound represented by Formula (1): 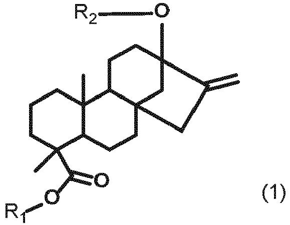 wherein, (i) R₁ represents Xyl(1-2)Glc1- while R₂ represents Glc(1-2)[Glc(1-3)]Glc1-; or (ii) R₁ represents Glc(1-2)[Glc(1-3)]Glc1- while R₂ represents Xyl(1-2)[Glc(1-3)]Glc1-, where Glc represents glucose and Xyl represents xylose).

2. The plant according to Claim 1, wherein the compound is represented by Formula (2) or (3) below:

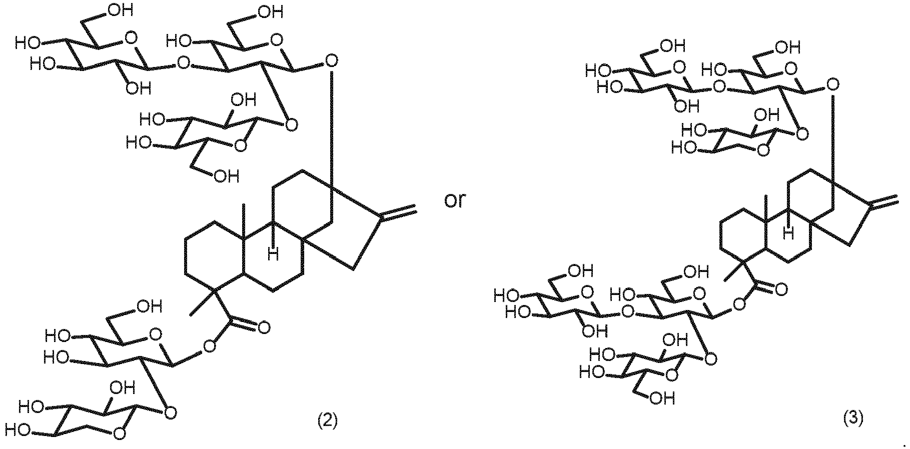

3. The plant according to Claim 1 or 2, wherein the compound is represented by Formula (4) or (5) below:

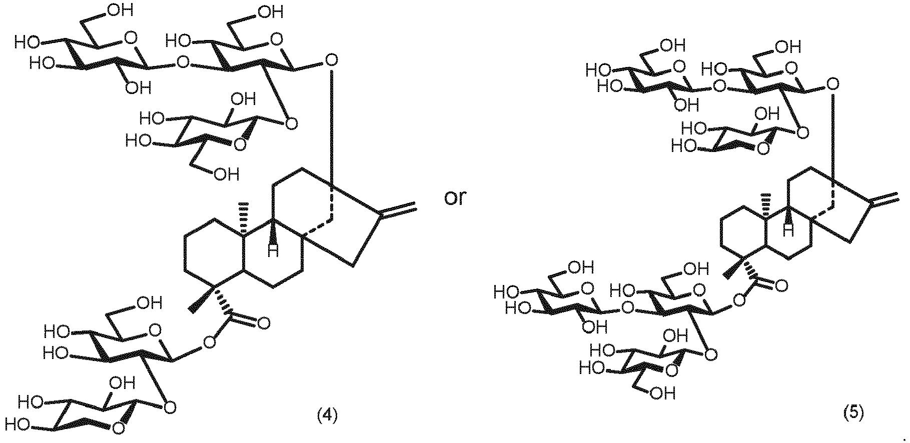

4. The plant according to any one of claims 1 to 3, wherein the content of the compound comprised in a leaf is 0.050 wt% or more relative to the amount of total steviol glycosides comprised in the leaf.

5. The plant according to any one of claims 1 to 4, having at least one genetic feature selected from the following (1) to (8):
(1) being homozygous or heterozygous for the allele wherein the base at the position corresponding to position 298 of SEQ ID NO: 1 is T;
(2) being homozygous or heterozygous for the allele wherein the base at the position corresponding to position 328 of SEQ ID NO: 1 is C;
(3) being homozygous or heterozygous for the allele wherein the base at the position corresponding to position 360 of SEQ ID NO: 1 is T;
(4) being homozygous or heterozygous for the allele wherein the base at the position corresponding to position 386 of SEQ ID NO: 1 is T;
(5) being homozygous or heterozygous for the allele wherein the base at the position corresponding to position 393 of SEQ ID NO: 1 is T;
(6) being homozygous or heterozygous for the allele wherein the base at the position corresponding to position 411 of SEQ ID NO: 1 is T;
(7) being homozygous or heterozygous for the allele wherein the base at the position corresponding to position 427 of SEQ ID NO: 1 is C; and
(8) being homozygous or heterozygous for the allele wherein the base at the position corresponding to position 453 of SEQ ID NO: 1 is T.

6. The plant according to any one of claims 1 to 5, wherein a gene encoding a protein having the amino acid sequence of SEQ ID NO: 97 is expressed at a higher level than a wild type line.

7. The plant according to any one of claims 1 to 6, wherein the plant is a non-genetically modified plant.

8. The plant according to any one of claims 1 to 7, wherein the plant comprises a stevia plant subjected to mutagenesis treatment and a progeny plant thereof.

9. A method of producing a stevia plant comprising a compound as defined in any one of claims 1 to 3, comprising a step of crossing the plant according to any one of claims 1 to 8 with a second stevia plant.

10. An extract of the stevia plant according to any one of claims 1 to 8, comprising the compound as defined in any one of claims 1 to 3.

11. The extract according to claim 10, wherein the content of the compound as defined in any one of claims 1 to 3 is 0.050 wt% or more relative to a content of total steviol glycoside.

12. A method of producing the extract according to claim 10 or 11, comprising a step of obtaining the extract from the plant according to any one of claims 1 to 8.

13. A method of producing a purified product of the compound as defined in any one of claims 1 to 3, comprising: a step of obtaining an extract from the plant according to any one of claims 1 to 8; and a step of purifying the compound from the obtained extract.

14. A food or beverage, a sweetener composition, a flavoring agent or a pharmaceutical product comprising the plant according to any one of claims 1 to 8 or the extract according to claim 10 or 11.

15. The food or beverage, sweetener composition, flavoring agent or pharmaceutical product according to claim 14, wherein the content of the compound as defined in any one of claims 1 to 3 is 1 mass ppm to 600 mass ppm.

16. The food or beverage according to claim 14 or 15, wherein the food or beverage is a beverage.

17. A method of producing a food or beverage, a sweetener composition, a flavoring agent or a pharmaceutical product, comprising:
a step of providing the extract according to claim 10 or 11 or a purified product thereof; and
a step of adding the extract or the purified product to a raw material for the food or beverage, sweetener composition, flavoring agent or pharmaceutical product.

18. A method of screening for a plant comprising the compound as defined in any one of claims 1 to 3, comprising:
(i) a step of detecting from the genome of a test stevia plant the presence and/or the absence of at least one of genetic features (1) to (8) as defined in claim 5; and/or
(ii) a step of detecting expression of a gene encoding a protein having the amino acid sequence of SEQ ID NO: 97 in the test stevia plant.

19. The method according to claim 18, further comprising a step of evaluating the content of the compound as defined in any one of claims 1 to 3 in the test stevia plant.

20. A screening kit for the stevia plant comprising the compound as defined in any one of claims 1 to 3, comprising a reagent for detecting the presence and/or absence of at least one of genetic features (1) to (8) as defined in claim 5 and/or a reagent for detecting expression of a gene encoding a protein having the amino acid sequence of SEQ ID NO: 97.

21. A method of producing a stevia plant comprising the compound as defined in any one of claims 1 to 3, comprising:
(1) a step of introducing a variation from C to T to a position corresponding to position 298 of SEQ ID NO: 1;
(2) a step of introducing a variation from A to C to a position corresponding to position 328 of SEQ ID NO: 1;
(3) a step of introducing a variation from C to T to a position corresponding to position 360 of SEQ ID NO: 1;
(4) a step of introducing a variation from C to T to a position corresponding to position 386 of SEQ ID NO: 1;
(5) a step of introducing a variation from C to T to a position corresponding to position 393 of SEQ ID NO: 1;
(6) a step of introducing a variation from C to T to a position corresponding to position 411 of SEQ ID NO: 1
(7) a step of introducing a variation from A to C to a position corresponding to position 427 of SEQ ID NO: 1; and/or
(8) a step of introducing a variation from C to T to a position corresponding to position 453 of SEQ ID NO: 1.


SEQUENCE LISTING

<110>  SUNTORY HOLDINGS LIMITED

<120>  Plant comprsing new steviol glycoside

<130>  G2421WO

<150>  JP 2019-141705
<151>  2019-07-31

<160>  116

<170>  PatentIn version 3.5

<210>  1
<211>  635
<212>  DNA
<213>  Stevia rebaudiana

<400>  1
atcaacctga gcaacaaagt tactgacaac acgaccatcg tcaatgcaca tcctaggacc      60

ataagtattg aatatcctcg caatcctcac ctgcaggttt tttttttttt tttttaatca     120

aaccaaatta atgattcttt ctgtagataa ccagagatta tatattatcc taaaatttaa     180

acaaaactac tctctacgtc cccaaaatac catgtgccat tgattacaat caaataaact     240

taatcttatt agtaaactga tggaaaacaa aatttagata aataaagcct tggacttctt     300

atgccaaatt tatttttatt ttttttcacc aactttaatt aatattgacc ataaacattc     360

ttgtttgtat gactcaactt taattctttt ttctttcatt ttgataacat cttttaaaca     420

ggactcacta tttgaacatt atgttccatt ttcttttaaa aaaaaataat gtggaaataa     480

taactgatta acataataat aattttgaga aagataatat atagaaacaa atttacttta     540

caataataag cttatcttga tttatggcat gcaatataat tttaattgcc ctttaacctt     600

taatatagtt tgtgtaacag tcaacttgtt ttggt                                635


<210>  2
<211>  635
<212>  DNA
<213>  Stevia rebaudiana

<400>  2
atcaacctga gcaacaaagt tactgacaac acgaccatcg tcaatgcaca tcctaggacc      60

ataagtattg aatatcctcg caatcctcac ctgcaggttt tttttttttt tttttaatca     120

aaccaaatta atgattcttt ctgtagataa ccagagatta tatattatcc taaaatttaa     180

acaaaactac tctctacgtc cccaaaatac catgtgccat tgattacaat caaataaact     240

taatcttatt agtaaactga tggaaaacaa aatttagata aataaagcct tggacttttt     300

atgccaaatt tatttttatt ttttttcccc aactttaatt aatattgacc ataaacattt     360

ttgtttgtat gactcaactt taattttttt ttttttcatt ttgataacat tttttaaaca     420

ggactcccta tttgaacatt atgttccatt tttttttaaa aaaaaataat gtggaaataa 480

taactgatta acataataat aattttgaga aagataatat atagaaacaa atttacttta 540

caataataag cttatcttga tttatggcat gcaatataat tttaattgcc ctttaacctt 600

taatatagtt tgtgtaacag tcaacttgtt ttggt 635

<210> 3
<211> 21
<212> DNA
<213> Stevia rebaudiana

<400> 3
ccttggactt cttatgccaa a 21

<210> 4
<211> 21
<212> DNA
<213> Stevia rebaudiana

<400> 4
ccttggactt tttatgccaa a 21

<210> 5
<211> 41
<212> DNA
<213> Stevia rebaudiana

<400> 5
ataaataaag ccttggactt cttatgccaa atttattttt a 41

<210> 6
<211> 41
<212> DNA
<213> Stevia rebaudiana

<400> 6
ataaataaag ccttggactt tttatgccaa atttattttt a 41

<210> 7
<211> 51
<212> DNA
<213> Stevia rebaudiana

<400> 7
tttagataaa taaagccttg gacttcttat gccaaattta tttttatttt t 51

<210> 8
<211> 51
<212> DNA
<213> Stevia rebaudiana

<400> 8
tttagataaa taaagccttg gactttttat gccaaattta tttttatttt t 51

<210>  9
<211>  21
<212>  DNA
<213>  Stevia rebaudiana

<400>  9
   attttttttc accaacttta a                                                21


<210>  10
<211>  21
<212>  DNA
<213>  Stevia rebaudiana

<400>  10
   attttttttc cccaacttta a                                                21


<210>  11
<211>  41
<212>  DNA
<213>  Stevia rebaudiana

<400>  11
atttattttt attttttttc accaacttta attaatattg a                          41


<210>  12
<211>  41
<212>  DNA
<213>  Stevia rebaudiana

<400>  12
atttattttt attttttttc cccaacttta attaatattg a                          41


<210>  13
<211>  51
<212>  DNA
<213>  Stevia rebaudiana

<400>  13
gccaaattta tttttatttt ttttcaccaa ctttaattaa tattgaccat a              51


<210>  14
<211>  51
<212>  DNA
<213>  Stevia rebaudiana

<400>  14
gccaaattta tttttatttt ttttccccaa ctttaattaa tattgaccat a              51


<210>  15
<211>  21
<212>  DNA
<213>  Stevia rebaudiana

<400>  15
cataaacatt cttgtttgta t                                               21

<210>  16
<211>  21
<212>  DNA
<213>  Stevia rebaudiana

<400>  16
cataaacatt tttgtttgta t                                                21


<210>  17
<211>  41
<212>  DNA
<213>  Stevia rebaudiana

<400>  17
taatattgac cataaacatt cttgtttgta tgactcaact t                          41


<210>  18
<211>  41
<212>  DNA
<213>  Stevia rebaudiana

<400>  18
taatattgac cataaacatt tttgtttgta tgactcaact t                          41


<210>  19
<211>  51
<212>  DNA
<213>  Stevia rebaudiana

<400>  19
ttaattaata ttgaccataa acattcttgt ttgtatgact caactttaat t               51


<210>  20
<211>  51
<212>  DNA
<213>  Stevia rebaudiana

<400>  20
ttaattaata ttgaccataa acatttttgt ttgtatgact caactttaat t               51


<210>  21
<211>  21
<212>  DNA
<213>  Stevia rebaudiana

<400>  21
aactttaatt ctttttctt t                                                 21


<210>  22
<211>  21
<212>  DNA
<213>  Stevia rebaudiana

<400>  22
aactttaatt tttttttttt t                                                21

<210>  23
<211>  41
<212>  DNA
<213>  Stevia rebaudiana

<400>  23
tgtatgactc aactttaatt cttttttctt tcattttgat a                          41


<210>  24
<211>  41
<212>  DNA
<213>  Stevia rebaudiana

<400>  24
tgtatgactc aactttaatt tttttttttt tcattttgat a                          41


<210>  25
<211>  50
<212>  DNA
<213>  Stevia rebaudiana

<400>  25
ttgtttgtat gactcaactt taattctttt ttctttcatt ttgataacat                50


<210>  26
<211>  50
<212>  DNA
<213>  Stevia rebaudiana

<400>  26
ttgtttgtat gactcaactt taattttttt ttttttcatt ttgataacat                50


<210>  27
<211>  21
<212>  DNA
<213>  Stevia rebaudiana

<400>  27
   attctttttt ctttcatttt g                                                21


<210>  28
<211>  21
<212>  DNA
<213>  Stevia rebaudiana

<400>  28
   attttttttt ttttcatttt g                                                21


<210>  29
<211>  35
<212>  DNA
<213>  Stevia rebaudiana

<400>  29
aactttaatt cttttttctt tcattttgat aacat                                 35

<210>  30
<211>  35
<212>  DNA
<213>  Stevia rebaudiana

<400>  30
aactttaatt tttttttttt tcattttgat aacat                                35


<210>  31
<211>  45
<212>  DNA
<213>  Stevia rebaudiana

<400>  31
gactcaactt taattctttt ttctttcatt ttgataacat ctttt                     45


<210>  32
<211>  45
<212>  DNA
<213>  Stevia rebaudiana

<400>  32
gactcaactt taattttttt ttttttcatt ttgataacat ttttt                     45


<210>  33
<211>  21
<212>  DNA
<213>  Stevia rebaudiana

<400>  33
ttgataacat cttttaaaca g                                               21


<210>  34
<211>  21
<212>  DNA
<213>  Stevia rebaudiana

<400>  34
ttgataacat tttttaaaca g                                               21


<210>  35
<211>  35
<212>  DNA
<213>  Stevia rebaudiana

<400>  35
tttcattttg ataacatctt ttaaacagga ctcac                                35


<210>  36
<211>  35
<212>  DNA
<213>  Stevia rebaudiana

<400>  36
tttcattttg ataacatttt ttaaacagga ctccc                                35

<210>  37
<211>  45
<212>  DNA
<213>  Stevia rebaudiana

<400>  37
ttttctttca ttttgataac atcttttaaa caggactcac tattt                        45


<210>  38
<211>  45
<212>  DNA
<213>  Stevia rebaudiana

<400>  38
   ttttttttca ttttgataac attttttaaa caggactccc tattt                        45


<210>  39
<211>  21
<212>  DNA
<213>  Stevia rebaudiana

<400>  39
aacaggactc actatttgaa c                                                  21


<210>  40
<211>  21
<212>  DNA
<213>  Stevia rebaudiana

<400>  40
aacaggactc cctatttgaa c                                                  21


<210>  41
<211>  35
<212>  DNA
<213>  Stevia rebaudiana

<400>  41
tcttttaaac aggactcact atttgaacat tatgt                                   35


<210>  42
<211>  35
<212>  DNA
<213>  Stevia rebaudiana

<400>  42
ttttttaaac aggactccct atttgaacat tatgt                                   35


<210>  43
<211>  45
<212>  DNA
<213>  Stevia rebaudiana

<400>  43
taacatcttt taaacaggac tcactatttg aacattatgt tccat                        45

<210>  44
<211>  45
<212>  DNA
<213>  Stevia rebaudiana

<400>  44
taacattttt taaacaggac tccctatttg aacattatgt tccat                    45


<210>  45
<211>  21
<212>  DNA
<213>  Stevia rebaudiana

<400>  45
gttccatttt cttttaaaaa a                                              21


<210>  46
<211>  21
<212>  DNA
<213>  Stevia rebaudiana

<400>  46
gttccatttt tttttaaaaa a                                              21


<210>  47
<211>  35
<212>  DNA
<213>  Stevia rebaudiana

<400>  47
acattatgtt ccattttctt ttaaaaaaaa ataat                               35


<210>  48
<211>  35
<212>  DNA
<213>  Stevia rebaudiana

<400>  48
acattatgtt ccattttttt ttaaaaaaaa ataat                               35


<210>  49
<211>  45
<212>  DNA
<213>  Stevia rebaudiana

<400>  49
tttgaacatt atgttccatt ttcttttaaa aaaaaataat gtgga                    45


<210>  50
<211>  45
<212>  DNA
<213>  Stevia rebaudiana

<400>  50
tttgaacatt atgttccatt tttttttaaa aaaaaataat gtgga                   45

<210>  51
<211>  39
<212>  DNA
<213>  Artificial Sequence

<220>
<223>  Synthetic DNA

<400>  51
gatggaaaac aaaatttaga taaataaagc cttgaacgt                              39


<210>  52
<211>  25
<212>  DNA
<213>  Artificial Sequence

<220>
<223>  Synthetic DNA

<400>  52
atcaacctga gcaacaaagt tactg                                             25


<210>  53
<211>  25
<212>  DNA
<213>  Artificial Sequence

<220>
<223>  Synthetic DNA

<400>  53
accaaaacaa gttgactgtt acaca                                             25


<210>  54
<211>  41
<212>  DNA
<213>  Artificial Sequence

<220>
<223>  Synthetic DNA

<400>  54
attaaagttg gtgaaaaaaa ataaaaataa atttggcatg c                           41


<210>  55
<211>  41
<212>  DNA
<213>  Artificial Sequence

<220>
<223>  Synthetic DNA

<400>  55
attaaagttg gtgaaaaaaa ataaaaataa atttggctta t                           41


<210>  56
<211>  40

<212>  DNA
<213>  Artificial Sequence

<220>
<223>  Synthetic DNA

<400>  56
ccttggactt cttatgccaa atttattttt attttgtatc                              40


<210>  57
<211>  40
<212>  DNA
<213>  Artificial Sequence

<220>
<223>  Synthetic DNA

<400>  57
   ccttggactt cttatgccaa atttattttt attttttggc                               40


<210>  58
<211>  40
<212>  DNA
<213>  Artificial Sequence

<220>
<223>  Synthetic DNA

<400>  58
ccttggactt cttatgccaa atttattttt attttggttc                              40


<210>  59
<211>  40
<212>  DNA
<213>  Artificial Sequence

<220>
<223>  Synthetic DNA

<400>  59
   ccttggactt cttatgccaa atttattttt attttttgta                               40


<210>  60
<211>  40
<212>  DNA
<213>  Artificial Sequence

<220>
<223>  Synthetic DNA

<400>  60
acaaacaaga atgtttatgg tcaatattaa ttaaactcgg                              40


<210>  61
<211>  40
<212>  DNA
<213>  Artificial Sequence

<220>
<223>  Synthetic DNA

<400>  61
acaaacaaga atgtttatgg tcaatattaa ttaaagttcc                              40


<210>  62
<211>  40
<212>  DNA
<213>  Artificial Sequence

<220>
<223>  Synthetic DNA

<400>  62
acaaacaaga atgtttatgg tcaatattaa ttaaagccgg                              40


<210>  63
<211>  40
<212>  DNA
<213>  Artificial Sequence

<220>
<223>  Synthetic DNA

<400>  63
acaaacaaga atgtttatgg tcaatattaa ttaaagtcgc                              40


<210>  64
<211>  40
<212>  DNA
<213>  Artificial Sequence

<220>
<223>  Synthetic DNA

<400>  64
acaaacaaga atgtttatgg tcaatattaa ttaaagtccg                              40


<210>  65
<211>  40
<212>  DNA
<213>  Artificial Sequence

<220>
<223>  Synthetic DNA

<400>  65
acaaacaaga atgtttatgg tcaatattaa ttaaacttga                              40


<210>  66
<211>  40
<212>  DNA
<213>  Artificial Sequence

<220>
<223>  Synthetic DNA

<400>  66
tttttttcac caactttaat taatattgac cataaacatg                                40

<210>  67
<211>  40
<212>  DNA
<213>  Artificial Sequence

<220>
<223>  Synthetic DNA

<400>  67
tttttttcac caactttaat taatattgac cataaagatc                                40

<210>  68
<211>  40
<212>  DNA
<213>  Artificial Sequence

<220>
<223>  Synthetic DNA

<400>  68
tttttttcac caactttaat taatattgac cataaacacg                                40

<210>  69
<211>  40
<212>  DNA
<213>  Artificial Sequence

<220>
<223>  Synthetic DNA

<400>  69
aatgaaagaa aaaagaatta aagttgagtc atacaatcag                                40

<210>  70
<211>  40
<212>  DNA
<213>  Artificial Sequence

<220>
<223>  Synthetic DNA

<400>  70
tgaccataaa cattcttgtt tgtatgactc aactttaacg                                40

<210>  71
<211>  25
<212>  DNA
<213>  Artificial Sequence

<220>
<223>  Synthetic DNA

<400>  71
attgaatatc ctcgcaatcc tcacc                                                25

<210>  72
<211>  40
<212>  DNA
<213>  Artificial Sequence

<220>
<223>  Synthetic DNA

<400>  72
aaatagtgag tcctgtttaa aagatgttat caaaatgatc                              40


<210>  73
<211>  40
<212>  DNA
<213>  Artificial Sequence

<220>
<223>  Synthetic DNA

<400>  73
aaatagtgag tcctgtttaa aagatgttat caaaatgtac                              40


<210>  74
<211>  40
<212>  DNA
<213>  Artificial Sequence

<220>
<223>  Synthetic DNA

<400>  74
aaatagtgag tcctgtttaa aagatgttat caaaattata                              40


<210>  75
<211>  40
<212>  DNA
<213>  Artificial Sequence

<220>
<223>  Synthetic DNA

<400>  75
gactcaactt taattctttt ttctttcatt ttgataacgt                              40


<210>  76
<211>  40
<212>  DNA
<213>  Artificial Sequence

<220>
<223>  Synthetic DNA

<400>  76
gactcaactt taattctttt ttctttcatt ttgataacag                              40


<210>  77
<211>  40

<212>  DNA
<213>  Artificial Sequence

<220>
<223>  Synthetic DNA

<400>  77
aaatggaaca taatgttcaa atagtgagtc ctgttttata                              40


<210>  78
<211>  40
<212>  DNA
<213>  Artificial Sequence

<220>
<223>  Synthetic DNA

<400>  78
ttttttcttt cattttgata acatctttta aacaggacgt                              40


<210>  79
<211>  40
<212>  DNA
<213>  Artificial Sequence

<220>
<223>  Synthetic DNA

<400>  79
ttttttcttt cattttgata acatctttta aacagggatc                              40


<210>  80
<211>  40
<212>  DNA
<213>  Artificial Sequence

<220>
<223>  Synthetic DNA

<400>  80
ttttttcttt cattttgata acatctttta aacaggtatc                              40


<210>  81
<211>  40
<212>  DNA
<213>  Artificial Sequence

<220>
<223>  Synthetic DNA

<400>  81
ttttttcttt cattttgata acatctttta aacaggaatg                              40


<210>  82
<211>  40
<212>  DNA
<213>  Artificial Sequence

<220>
<223>  Synthetic DNA

<400>  82
   tttttctttt cattttgata acatctttta aacaggaatt                              40


<210>  83
<211>  40
<212>  DNA
<213>  Artificial Sequence

<220>
<223>  Synthetic DNA

<400>  83
tttttctttt cattttgata acatctttta aacagggcgc                              40


<210>  84
<211>  40
<212>  DNA
<213>  Artificial Sequence

<220>
<223>  Synthetic DNA

<400>  84
tttttctttt cattttgata acatctttta aacaggagcc                              40


<210>  85
<211>  40
<212>  DNA
<213>  Artificial Sequence

<220>
<223>  Synthetic DNA

<400>  85
   tttttctttt cattttgata acatctttta aacaggggtc                              40


<210>  86
<211>  25
<212>  DNA
<213>  Artificial Sequence

<220>
<223>  Synthetic DNA

<400>  86
ctactctcta cgtccccaaa atacc                                             25


<210>  87
<211>  40
<212>  DNA
<213>  Artificial Sequence

<220>
<223>  Synthetic DNA

<400>  87
attttttttt aaaagaaaat ggaacataat gttcaactcg                              40


<210>  88
<211>  40
<212>  DNA
<213>  Artificial Sequence

<220>
<223>  Synthetic DNA

<400>  88
attttttttt aaaagaaaat ggaacataat gttcaaatcc                              40


<210>  89
<211>  40
<212>  DNA
<213>  Artificial Sequence

<220>
<223>  Synthetic DNA

<400>  89
attttttttt aaaagaaaat ggaacataat gttcaaccag                              40


<210>  90
<211>  40
<212>  DNA
<213>  Artificial Sequence

<220>
<223>  Synthetic DNA

<400>  90
attttttttt aaaagaaaat ggaacataat gttcacatat                              40


<210>  91
<211>  40
<212>  DNA
<213>  Artificial Sequence

<220>
<223>  Synthetic DNA

<400>  91
   tgttaatcag ttattatttc cacattattt ttttttcaag                               40


<210>  92
<211>  40
<212>  DNA
<213>  Artificial Sequence

<220>
<223>  Synthetic DNA

<400>  92
   tgttaatcag ttattatttc cacattattt tttttttata                               40

<210>  93
<211>  377
<212>  DNA
<213>  Artificial Sequence

<220>
<223>  Synthetic DNA

<400>  93
gatggaaaac aaaatttaga taaataaagc cttgaacgtt ttatgccaaa tttattttta      60

ttttttttcc ccaactttaa ttaatattga ccataaacat ttttgtttgt atgactcaac      120

tttaattttt ttttttttca ttttgataac attttttaaa caggactccc tatttgaaca      180

ttatgttcca ttttttttta aaaaaaaata atgtggaaat aataactgat taacataata      240

ataattttga gaaagataat atatagaaac aaatttactt tacaataata agcttatctt      300

gatttatggc atgcaatata attttaattg ccctttaacc tttaatatag tttgtgtaac      360

agtcaacttg ttttggt                                                    377


<210>  94
<211>  341
<212>  DNA
<213>  Artificial Sequence

<220>
<223>  Synthetic DNA

<400>  94
cgttttatgc caaatttatt tttatttttt ttccccaact ttaattaata ttgaccataa      60

acatttttgt ttgtatgact caactttaat tttttttttt ttcattttga taacattttt      120

taaacaggac tccctatttg aacattatgt tccatttttt tttaaaaaaa aataatgtgg      180

aaataataac tgattaacat aataataatt ttgagaaaga taatatatag aaacaaattt      240

actttacaat aataagctta tcttgattta tggcatgcaa tataatttta attgcccttt      300

aacctttaat atagtttgtg taacagtcaa cttgttttgg t                         341


<210>  95
<211>  36
<212>  DNA
<213>  Artificial Sequence

<220>
<223>  Synthetic DNA

<400>  95
gatggaaaac aaaatttaga taaataaagc cttgaa                               36


<210>  96
<211>  377
<212>  DNA
<213>  Artificial Sequence

<220>
<223>  Synthetic DNA

<400>  96
gatggaaaac aaaatttaga taaataaagc cttgaacgtc ttatgccaaa tttattttta      60

ttttttttca ccaactttaa ttaatattga ccataaacat tcttgtttgt atgactcaac      120

tttaattctt ttttctttca ttttgataac atcttttaaa caggactcac tatttgaaca      180

ttatgttcca ttttctttta aaaaaaaata atgtggaaat aataactgat taacataata      240

ataattttga gaaagataat atatagaaac aaatttactt tacaataata agcttatctt      300

gatttatggc atgcaatata attttaattg ccctttaacc tttaatatag tttgtgtaac      360

agtcaacttg ttttggt                                                    377


<210>  97
<211>  306
<212>  PRT
<213>  Stevia rebaudiana

<400>  97

Met Gly Ser Glu Leu Thr Tyr Arg His Gln Asp Thr Leu Pro Gly Ala
1               5                   10                  15

Ser Glu Ser Tyr Ser Pro Lys Pro Asn Lys Gln Ser Val Thr Arg Val
            20                  25                  30

Val Arg Tyr Ile Leu Arg Glu Gln Arg Leu Val Phe Val Leu Leu Gly
        35                  40                  45

Ile Ala Ile Ala Thr Ser Val Phe Thr Leu Leu Pro Ser Ser Thr Asn
    50                  55                  60

Thr Val Thr Asp Ala Tyr Ser Val Ser Glu Ser Val Gln Leu Met Asn
65                  70                  75                  80

Pro Gln Arg Ser Val Tyr Pro Ala Arg Phe Asn Met Gly Gly Lys Ile
                85                  90                  95

Pro Leu Gly Leu Lys Arg Lys Gly Leu Arg Ile Val Val Thr Gly Gly
            100                 105                 110

Ala Gly Phe Val Gly Ser His Leu Val Asp Arg Leu Ile Ala Arg Gly
        115                 120                 125

Asp Ser Val Ile Val Val Asp Asn Phe Phe Thr Gly Asn Lys Asp Asn
    130                 135                 140

Val Met His His Phe Gly Asn Pro Arg Phe Glu Leu Ile Arg His Asp
145                 150                 155                 160

Val Val Glu Pro Leu Leu Leu Glu Val Asp Gln Ile Tyr His Leu Ala
                165                 170                 175

Cys Pro Ala Ser Pro Val His Tyr Lys Phe Asn Pro Thr Asn Val Val
            180                 185                 190

Gly Thr Leu Asn Met Leu Gly Leu Ala Lys Arg Val Gly Ala Arg Phe
        195                 200                 205

Leu Leu Thr Ser Thr Ser Glu Val Tyr Gly Asp Pro Leu Gln His Pro
    210                 215                 220

Gln Val Glu Thr Tyr Trp Gly Asn Val Asn Pro Ile Ala Gln Val Val
225                 230                 235                 240

Gln Glu Thr Ile Asp Pro Asn Ala Ser Ile Glu Phe Lys Pro Asn Thr
                245                 250                 255

Glu Asp Asp Pro His Lys Arg Lys Pro Asp Ile Thr Lys Ala Lys Glu
            260                 265                 270

Leu Leu Gly Trp Lys Pro Lys Val Pro Leu Arg Lys Gly Leu Pro Met
        275                 280                 285

Met Val Ser Asp Phe Arg Arg Arg Ile Phe Gly Asp Gln Asn Asn Pro
    290                 295                 300

Ser Ala
305

<210>  98
<211>  921
<212>  DNA
<213>  Stevia rebaudiana

<400>  98
atgggatctg agttaacata ccggcaccaa gataccctac cgggggcgtc ggaatcttac      60

tcgccgaagc ctaacaagca gtccgtgact cgcgtcgttc gttacatcct ccgtgagcag     120

cgtctcgtat ttgtgctttt gggcattgca atcgctacct ccgtcttcac gctcctccca     180

tcatccacaa acaccgtcac cgatgcctat tccgtctctg aatcggtcca gttgatgaac     240

ccgcagcgat ccgtgtatcc tgcgagattc aatatggcgg ggaagattcc gctaggtttg     300

aaacgaaagg ggttaaggat tgtggtgacc ggtggtgcag gttttgtcgg aagtcatcta     360

gttgaccggt tgattgcgag gggagacagt gtgattgttg ttgataattt cttcactggt     420

aacaaggata acgtgatgca tcactttgga aaccctagat ttgagcttat tcgtcacgac 480

gttgttgagc ctttgctact cgaggtcgat cagatctatc acctggcttg tcctgcttcc 540

cctgttcatt acaaattcaa cccgacaaat gtggtaggga cattgaacat gttagggttg 600

gcgaagaggg ttggtgcgcg gttcttgcta acaagcacca gcgaggtata cggtgatcct 660

ttacagcatc ctcaagtcga aacctactgg ggcaacgtca atcctatcgc ccaggtggtc 720

caggaaacga tagacccaaa cgcaagcata gagttcaaac caaacacaga agacgaccca 780

cacaaacgga agccggatat cacaaaggct aaggaactcc tgggttggaa gcccaaggtc 840

cctcttcgca aaggtctccc catgatggtt tctgacttca ggcgccgcat ttttggtgac 900

caaaacaacc catccgctta a 921

<210> 99
<211> 24
<212> DNA
<213> Artificial Sequence

<220>
<223> Synthetic DNA

<400> 99
cctgttcatt acaaattcaa cccg 24

<210> 100
<211> 25
<212> DNA
<213> Artificial Sequence

<220>
<223> Synthetic DNA

<400> 100
aaccctaaca tgttcaatgt cccta 25

<210> 101
<211> 316
<212> DNA
<213> Stevia rebaudiana

<400> 101
cgggcgtgtt gtgagtaatt ttatagctca agcacttcgg ttagtcctcc attataatca 60

gaatcagtat ttttaatttt aaatatattt ggttaatctt aattcttgca gcgatgaacc 120

tttaaccgtt caagcccctg gaacccaaac ccgcagtttc tgttacgtct ctgatatggt 180

attttgaaaa ataatatgat tatacattta tgatataact ggtgacgcgt gtcaaacatt 240

gacttttttt taaaccgtat aggttgatgg gcttgttaaa ctaatggaag gtgaaaacac 300

tggaccaatc aacatc 316

<210> 102
<211> 331
<212> DNA
<213> Stevia rebaudiana

<400> 102
cgggcgtgtt gtgagtaatt ttatagctca agcacttcgg ttagtcctcc attataatca 60

gaatcagtat ttttaatttt aaatatattt tttttaaata tatttggtta atcttaattc 120

ttgcagcgat gaacctttaa ccgttcaagc ccctggaacc caaacccgca gtttctgtta 180

cgtctctgat atggtatttt gaaaaataat atgattatac atttatgata taactggtga 240

cgcgtgtcaa acattgactt ttttttaaac cgtataggtt gatgggcttg ttaaactaat 300

ggaaggtgaa aacactggac caatcaacat c 331

<210> 103
<211> 183
<212> DNA
<213> Stevia rebaudiana

<400> 103
aacccaattg gtaagcgtgc atctctgttt attttttgtt aattattgtg tatttgttga 60

attatcaatt atattggtga ttctttcttc tttaaaataa ataaaaaccg tgaaattatg 120

gtttttatta tttatgtatt ttgcaggagt taggagttgc tatgatgaag gaaaacgcgt 180

ggc 183

<210> 104
<211> 183
<212> DNA
<213> Stevia rebaudiana

<400> 104
aacccaattg gtaagcgtgc atctctgttt attttttgtt aattattgtg tatttgttga 60

attatcaatt atattggtga ttctttcttc tttaaaaaaa aaaaaaaccg gggaaataag 120

ggttttatta tttatgtatt ttgcaggagt taggagttgc tatgatgaag gaaaacgcgt 180

ggc 183

<210> 105
<211> 251
<212> PRT
<213> Stevia rebaudiana

<400> 105

Met Ala Asn Asn Ala Ser Asn Gly Glu His Lys Ser Ser Lys Pro Pro
1               5                   10                  15

Pro Thr Pro Ser Pro Leu Arg Asn Ser Lys Phe Phe Gln Ser Asn Met
            20                  25                  30

Arg Ile Leu Val Thr Gly Gly Ala Gly Phe Ile Gly Ser His Leu Val
        35                  40                  45

Asp Lys Leu Met Glu Asn Glu Lys Asn Glu Val Ile Val Ala Asp Asn
    50                  55                  60

Tyr Phe Thr Gly Ser Lys Asp Asn Leu Arg Lys Trp Ile Gly His Pro
65                  70                  75                  80

Arg Phe Glu Leu Ile Arg His Asp Val Thr Glu Pro Leu Leu Val Glu
                85                  90                  95

Val Asp Gln Ile Tyr His Leu Ala Cys Pro Ala Ser Pro Ile Phe Tyr
            100                 105                 110

Lys His Asn Pro Val Lys Thr Ile Ala Glu Thr Leu Met Phe Asp Tyr
        115                 120                 125

His Arg Gln His Gly Ile Glu Ile Arg Ile Ala Arg Ile Phe Asn Thr
    130                 135                 140

Tyr Gly Pro Arg Met Asn Ile Asp Asp Gly Arg Val Val Asp Gly Leu
145                 150                 155                 160

Val Lys Leu Met Glu Gly Glu Asn Thr Gly Pro Ile Asn Ile Gly Asn
                165                 170                 175

Pro Gly Glu Phe Thr Met Ile Glu Leu Ala Glu Met Val Lys Glu Leu
            180                 185                 190

Ile Asn Pro Lys Ile Glu Ile Lys Met Val Glu Asn Thr Pro Asp Asp
        195                 200                 205

Pro Arg Gln Arg Lys Pro Asp Ile Thr Asn Ala Lys Lys Met Leu Gly
    210                 215                 220

Trp Glu Pro Lys Ile Lys Leu Arg Asp Gly Leu Pro Leu Met Glu Ala
225                 230                 235                 240

Asp Phe Arg Leu Arg Leu Gly Val Ala Lys Lys
                245                 250

<210>  106
<211>  936
<212>  DNA
<213>  Stevia rebaudiana

<400>  106

atggcgaata atgcttcaaa cggagaacat aaatcttcaa aaccgcctcc aacgccttct 60

cctttaagga actccaaatt ctttcagtca aacatgagga tattggttac tggtggtgct 120

ggatttatcg gctctcactt ggtggataaa cttatggaaa atgaaaagaa tgaggtaatt 180

gttgctgata attactttac tggctcaaaa gataatctta gaaaatggat tggtcatcca 240

agatttgagc ttattcgtca tgatgtcact gaaccattgc tagttgaggt tgatcagatt 300

tatcatcttg catgccccgc ttcaccaatt ttttataaac acaatcctgt aaagacgatt 360

aagacgaatg taattggcac actaaatatg cttggtcttg cgaagcgagt cggggcaagg 420

attttgctta cctcaacatc tgaggtatac ggtgatcctc ttgtgcatcc gcaaccagag 480

agttactggg gtaatgtcaa cccgattgga gttcggagtt gctatgatga aggaaaacgt 540

gtggcagaga ctctgatgtt tgattatcac aggcaacatg gaatagaaat aagaattgcg 600

cgtattttca acacatatgg accccggatg aatattgatg acgggcgtgt tgttgatggg 660

cttgttaaac taatggaagg tgaaaacact ggaccaatca acatcggtaa tccaggtgaa 720

ttcacaatga tcgaactcgc tgagatggtt aaggagctta ttaatccgaa aatagagatt 780

aagatggtgg aaaacacacc cgatgatcca cgacaaagaa aacccgacat cacaaatgcg 840

aagaagatgc ttggatggga gccgaagatc aaactgcgcg atggacttcc gctcatggaa 900

gctgatttta ggctgaggct tggagttgcc aagaag 936

<210> 107
<211> 313
<212> PRT
<213> Stevia rebaudiana

<400> 107

Met Ala Asn Asn Ala Ser Asn Gly Glu His Lys Val Thr Lys Pro Pro
1               5                   10                  15

Pro Thr Pro Ser Pro Leu Arg Asn Ser Lys Phe Leu Gln Ser Asn Met
            20                  25                  30

Arg Ile Leu Val Thr Gly Gly Ala Gly Phe Ile Gly Ser His Leu Val
        35                  40                  45

Asp Lys Leu Met Glu Asn Glu Lys Asn Glu Val Ile Val Ala Asp Asn
    50                  55                  60

Tyr Phe Thr Gly Ser Lys Asp Asn Leu Arg Lys Trp Ile Gly His Pro
65                  70                  75                  80

Arg Phe Glu Leu Ile Arg His Asp Val Thr Glu Lys Leu Leu Val Glu
                85                  90                  95

Val Asp Gln Ile Tyr His Leu Ala Cys Pro Ala Ser Pro Ile Phe Tyr
            100                 105                 110

Lys Tyr Asn Pro Val Lys Thr Ile Lys Thr Asn Val Ile Gly Thr Leu
        115                 120                 125

Asn Met Leu Gly Leu Ala Lys Arg Val Gly Ala Arg Ile Leu Leu Thr
    130                 135                 140

Ser Thr Ser Glu Val Tyr Gly Asp Pro Leu Val His Pro Gln Pro Glu
145                 150                 155                 160

Thr Tyr Trp Gly Asn Val Asn Pro Ile Gly Val Arg Ser Cys Tyr Asp
                165                 170                 175

Glu Gly Lys Arg Val Ala Glu Thr Leu Met Phe Asp Tyr His Arg Gln
            180                 185                 190

His Gly Ile Glu Ile Arg Ile Ala Arg Ile Phe Asn Thr Tyr Gly Pro
        195                 200                 205

Arg Met Asn Ile Asp Asp Gly Arg Val Val Asp Gly Leu Ile Arg Leu
    210                 215                 220

Met Glu Gly Glu Asn Thr Gly Pro Ile Asn Ile Gly Asn Pro Gly Glu
225                 230                 235                 240

Phe Thr Met Ile Glu Leu Ala Glu Thr Val Lys Glu Leu Ile Asn Pro
                245                 250                 255

Lys Ile Glu Ile Lys Met Val Glu Asn Thr Pro Asp Asp Pro Arg Gln
            260                 265                 270

Arg Lys Pro Asp Ile Thr Asn Ala Lys Lys Met Leu Gly Trp Glu Pro
        275                 280                 285

Lys Ile Lys Leu Arg Asp Gly Leu Pro Leu Met Glu Ala Asp Phe Arg
    290                 295                 300

Leu Arg Leu Gly Val Ala Lys Lys Ile
305                 310

<210> 108
<211> 942
<212> DNA
<213> Stevia rebaudiana

<400> 108

atggcaaata acgcttcaaa tggtgaacat aaagttacaa aaccacctcc aaccccatct 60

cctctgcgta attctaaatt cttgcagtca aatatgagga tattggttac tggtggtgct 120

ggatttatcg gatctcacct agtggataaa ctgatggaaa atgaaaagaa tgaggtgatt 180

gttgctgata attattttac tggttcaaaa gacaacctta gaaagtggat tggtcatcca 240

agatttgagc ttattcgaca tgatgtcaca gaaaagttgc tggttgaagt tgatcagata 300

taccatcttg catgtcctgc ttcacccatt ttttataaat acaatcctgt taaaacgata 360

aagactaatg ttattggcac actaaatatg cttggtcttg ccaagcgagt tggagcaagg 420

attttgctta cctcgacctc ggaggtatat ggtgatcctc tcgtgcatcc acaacctgag 480

acctactggg gtaatgtcaa cccaattgga gttaggagtt gctatgatga aggaaaacgc 540

gtggcagaga ctttgatgtt tgattatcac agacaacatg gcatagaaat aagaattgct 600

cgtattttca acacatatgg accccggatg aacatcgatg acggacgtgt cgttgatggg 660

cttattcggc tgatggaagg tgagaacacc ggaccaatca acattggtaa tccgggtgaa 720

tttacaatga ttgaactcgc tgaaacagtc aaagagctta taaatccaaa aatagagatt 780

aagatggtgg aaaacacgcc tgatgatcca agacaaagaa aaccggatat tacaaatgct 840

aaaaagatgc ttggatggga gcctaagatt aaattgcgcg atggtcttcc gcttatggaa 900

gcagacttta ggttaaggct tggagttgca aagaagatat aa 942

<210> 109
<211> 25
<212> DNA
<213> Artificial Sequence

<220>
<223> Synthetic DNA

<400> 109
atcagattta tcatcttgca tgccc 25

<210> 110
<211> 25
<212> DNA
<213> Artificial Sequence

<220>
<223> Synthetic DNA

<400> 110
tgccaattac attcgtctta atcgt 25

<210> 111
<211> 25
<212> DNA
<213> Artificial Sequence

<220>

<223>  Synthetic DNA

<400>  111
aaaagttgct ggttgaagtt gatca                                          25


<210>  112
<211>  24
<212>  DNA
<213>  Artificial Sequence

<220>
<223>  Synthetic DNA

<400>  112
cacactaaat atgcttggtc ttgc                                           24


<210>  113
<211>  24
<212>  DNA
<213>  Artificial Sequence

<220>
<223>  Synthetic DNA

<400>  113
cgccatcctc cgtcttgatc ttgc                                           24


<210>  114
<211>  20
<212>  DNA
<213>  Artificial Sequence

<220>
<223>  Synthetic DNA

<400>  114
ccgttcggcg gtggtggtaa                                                20


<210>  115
<211>  25
<212>  DNA
<213>  Artificial Sequence

<220>
<223>  Synthetic DNA

<400>  115
tcactcttga agtggagagt tccga                                          25


<210>  116
<211>  20
<212>  DNA
<213>  Artificial Sequence

<220>
<223>  Synthetic DNA

<400>  116

gcctctgttg gtccggtggg 20